# EUROPEAN PATENT APPLICATION

(11) **EP 3 792 353 A1**
(43) Date of publication of application: **17.03.2021**
(21) Application number: 19382795.3
(22) Date of filing: 13.09.2019
(51) Int. Cl.: C12N 9/10, C12N 15/82

(54) **ARTIFICIAL CHROMOPLAST BIOGENESIS**

(71) Applicant: CRAG - Centre de Recerca en Agrigenomica CSIC-IRTA-UB-UAB, 08193 Cerdanyola del Vallés (Barcelona) (ES); Consejo Superior de Investigaciones Científicas (CSIC), 28006 Madrid (ES)
(72) Inventor: RODRIGUEZ-CONCEPCION, Manuel, 08193 Cerdanyola del Vallés (Barcelona) (ES); LLORENTE, Briardo, 08193 Cerdanyola del Vallés (Barcelona) (ES); TORRES MONTILLA, Salvador, 08193 Cerdanyola del Vallés (Barcelona) (ES); MORELLI, Luca, 08193 Cerdanyola del Vallés (Barcelona) (ES)
(74) Representative: Hoffmann Eitle

(57) **Abstract**

In the present invention, we show for the first time that the supply of phytoene to feed the carotenoid pathway functions as a primary determinant of chromoplast biogenesis in green tissues. We provide evidence that **exceeding a threshold level** of phytoene, the first committed intermediate of the carotenoid pathway, is sufficient to induce chloroplast-to-chromoplast differentiation in leaves. The present invention thus reveals a novel aspect of carotenoid biology and additionally presents a powerful tool for biotechnological applications of the modulation of plastid identity, that could be used in the development of new biofortified crops, food or as a source of extracts for a variety of industries.

## Description

### FIELD OF THE INVENTION

The present invention generally relates to the field of plant molecular biology and synthetic biology and concerns both a method for the induction of artificial chromoplasts from chloroplasts cells, and products derived therefrom.

### BACKGROUND OF THE INVENTION

Plastids comprise a group of morphologically and functionally diverse plant organelles capable of differentiating from one plastid type to another in response to developmental and environmental stimuli (Jarvis and Lopez-Juez 2013, Liebers, Grubler et al. 2017). Such plastidial conversions are essential to sustain many fundamental biological processes and largely contribute to cell specialization in the different plant tissues (Kleffmann, von Zychlinski et al. 2007, Barsan, Sanchez-Bel et al. 2010, Barsan, Zouine et al. 2012, Liebers, Grubler et al. 2017). Among the different plastid types, chromoplasts are plastids of great importance in nature and agriculture because of their capacity to accumulate high levels of carotenoids, plant pigments of isoprenoid nature that impart color in the yellow to red range (Rodriguez-Concepcion et al. 2018). Besides health-promoting carotenoids such as beta-carotene (pro-vitamin A), chromoplasts accumulate other isoprenoids of nutritional interest such as tocopherols (vitamin E). Both carotenoids and tocopherols are essential nutrients that animals cannot synthesize and therefore must be taken in the diet (Fraser and Bramley 2004, Brehelin, Kessler et al. 2007, Giuliano 2017, Gramegna, Rosado et al. 2018, Rodriguez-Concepcion, Avalos et al. 2018). They are also added-value compounds widely used in several industries, including cosmetic, food, and animal feed industries (Ogbonna 2009, Rodriguez-Concepcion, Avalos et al. 2018). Therefore the ability to promote the differentiation of chromoplasts from chloroplasts (in chlorophyll containing photosynthetically active cells) has several potential biotechnological applications including but not restricted to food, feed, agriculture, cosmetic or nutraceutical, either alone or in combination with other inventions.

Chromoplasts can differentiate from preexisting plastids such as proplastids (i.e., undifferentiated plastids) and amyloplasts (i.e., starch-storing plastids), but most often they develop from chloroplasts (i.e., photosynthetic, chlorophyll containing plastids). Transformation of chloroplasts into chromoplasts occurs during the development of many flower petals and fruits but only a few plant species differentiate true chromoplasts in chlorophyll containing leaves. The yellow to red colors that some leaves acquire as they senesce (e.g. in the autumn or when they are exposed to continuous darkness) are due to carotenoids becoming visible when the chlorophylls degrade. This senescence process, however, does not involve the transformation of chloroplasts into chromoplasts but into a completely different type of plastids named gerontoplasts (Koiwa, Ikeda et al. 1986, Hormaetxe, Hernandez et al. 2004, Egea, Barsan et al. 2010, Jarvis and Lopez-Juez 2013, Liebers, Grubler et al. 2017).

The most prominent changes during chloroplast to chromoplast differentiation are the remodeling of the internal plastid structures, together with a concurrent loss of photosynthetic competence and over-accumulation of carotenoid pigments (Egea, Barsan et al. 2010, Jarvis and Lopez-Juez 2013, Liebers, Grubler et al. 2017, Llorente, Martinez-Garcia et al. 2017, Gramegna, Rosado et al. 2018). The remodeling of the internal plastid structures generates increased metabolic sink capacity for carotenoid biosynthesis and storage, and it is, therefore, a critical developmental mechanism regulating carotenoid production in plants (Yuan, Zhang et al. 2015, Llorente, Martinez-Garcia et al. 2017). Due to its unique capacity to accumulate high amounts of carotenoids and other dietary nutrients such as tocopherols, the control of chromoplast differentiation has been proposed as a very promising strategy for improving the nutritional and health benefits of crops, and for other biotechnological endeavors (Yuan, Owsiany et al. 2015, Yuan, Zhang et al. 2015, Giuliano 2017, Llorente, Martinez-Garcia et al. 2017, Wurtzel 2019). However, the experimental manipulation of chromoplast differentiation for biotechnological applications has been hampered by our limited understanding of the mechanisms regulating this process. The only regulator of chromoplast development identified to date is ORANGE (OR), a chaperone found to localize in both plastids and nuclei. The specific molecular mechanism by which ORANGE triggers chromoplast differentiation remains unclear, as it independently promotes carotenoid biosynthesis, stability, and storage. As described below, the phytoene inductive threshold mechanism for artificial biogenesis was found to be independent of ORANGE, that is, does not require ORANGE dependent functions.

Here, we show for the first time that the supply of a sufficiently large amount of phytoene into chlorophyll containing plastids (chloroplasts) functions as a primary determinant of chromoplast artificial or synthetic biogenesis in green tissues (i.e., leaves or any other chloropyll containing, photosynthetic organs, tissues or cells). We provide evidence that **exceeding a threshold level** of phytoene, the first committed intermediate of the carotenoid pathway, is sufficient to induce chloroplast-to-chromoplast differentiation in green tissues. The present invention thus reveals a novel aspect of carotenoid biology and additionally presents a powerful tool for biotechnological applications of the artificial or synthetic differentiation of plastids into chromoplasts that could be used in new biofortified food and feed crops or nutraceutical and pharmaceutical compositions.

### BRIEF DESCRIPTION OF THE FIGURES

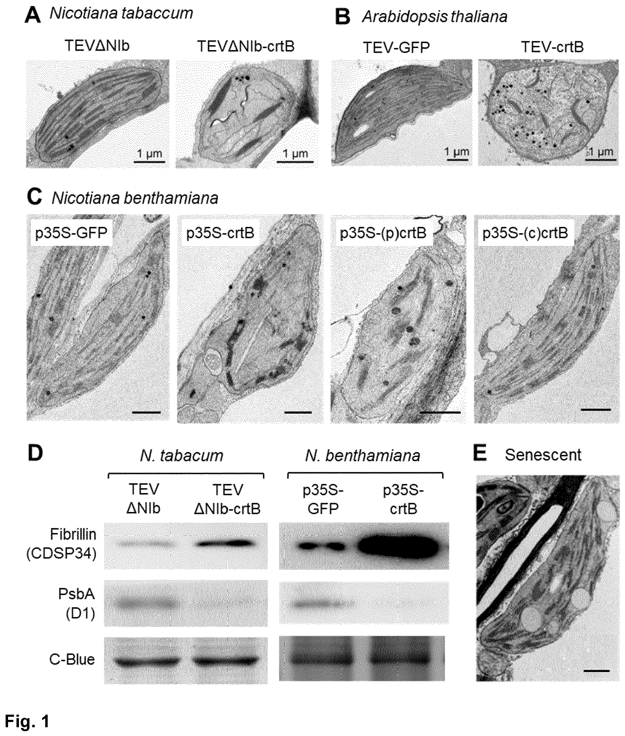

**Figure 1****. Chromoplast-like plastids develop from chloroplasts in leaves producing crtB.** A, B, C, E, TEM images of representative plastids from the indicated species and treatments. Bars, 1 µm. D, Immonoblot analysis of plastidial proteins.

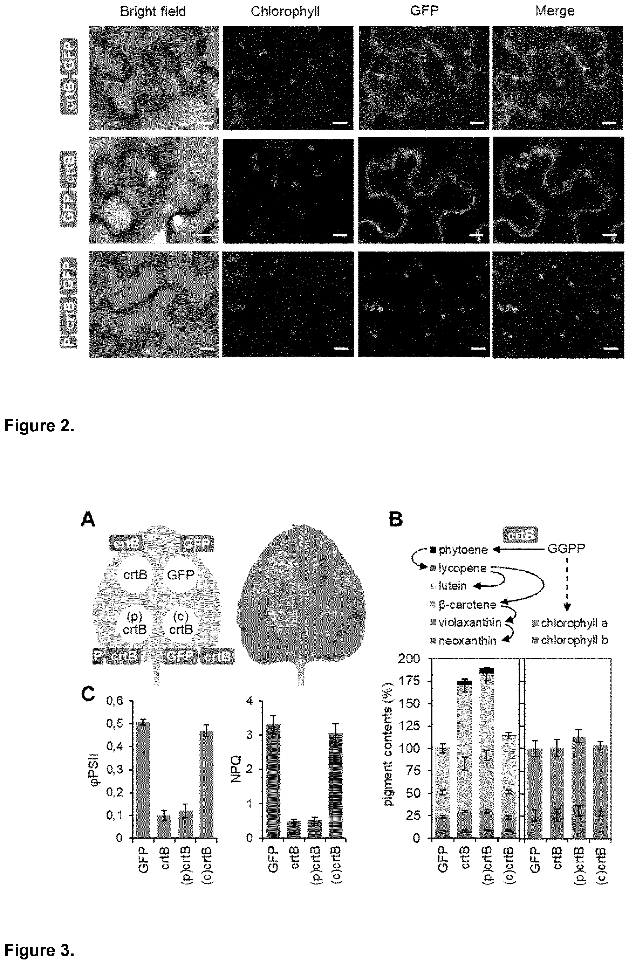

**Figure 2****. Plant-produced crtB stays in the cytosol but can also enter chloroplats.** Subcellular localization of the indicated constructs in *N. benthamiana* leaf cells. Bars, 20 µm.
**Figure 3****. Bacterial crtB needs to enter the chloroplast to induce chromoplast differentiation.** A, Phenotypes of N. benthamiana leaf sections expressing the indicated constructs. B, Photosynthetic pigment levels in the sections shown in B. C, Photosynthetic parameters of the sections shown in B.

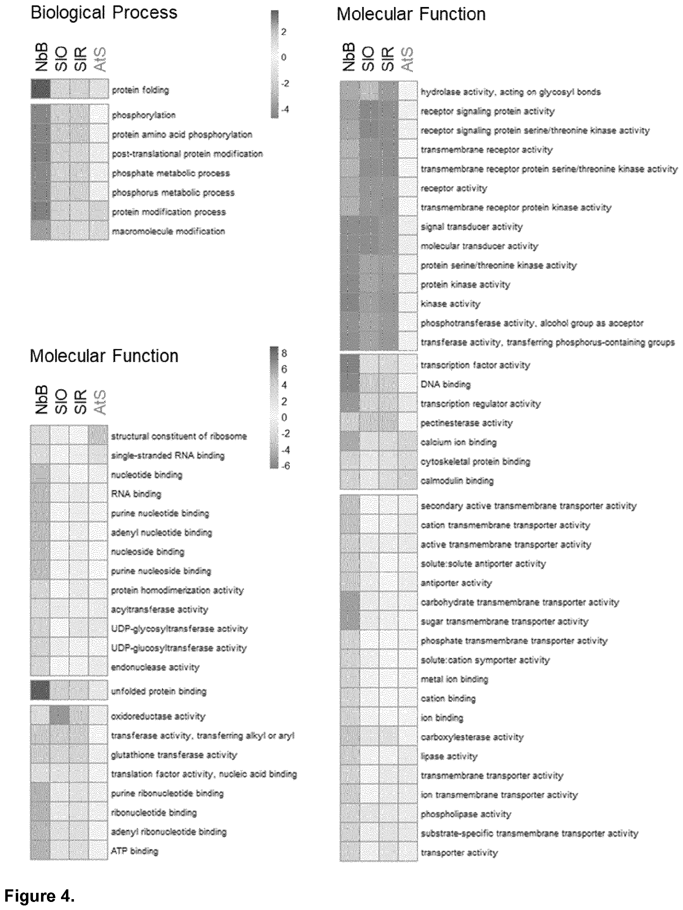

**Figure 4****. Chromoplast differentiation in *N. benthamiana* leaves ressembles that in tomato fruit.** Heatmap represents of GO term enrichment values (Z-scores) in calculated from log2FC values in *N. benthamiana* leaves (crtB vs. GFP, NbB), tomato fruit (light ripe vs. mature green, SLO, and red ripe vs. mature green, SIR) and Arabidopsis leaves (30D senescent vs. 16D controls, AtS).

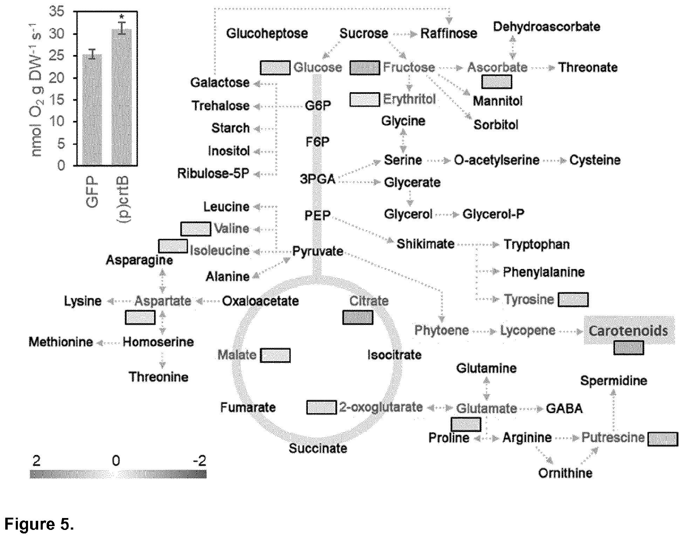

**Figure 5****. Chromoplast differentiation induces glycolytic and oxidative energy metabolism.** Heatmap represents statistically significant fold-change values of metabolite levels in *N. benthamiana* leaves (crtB vs. GFP). Inset represents respiration rates.

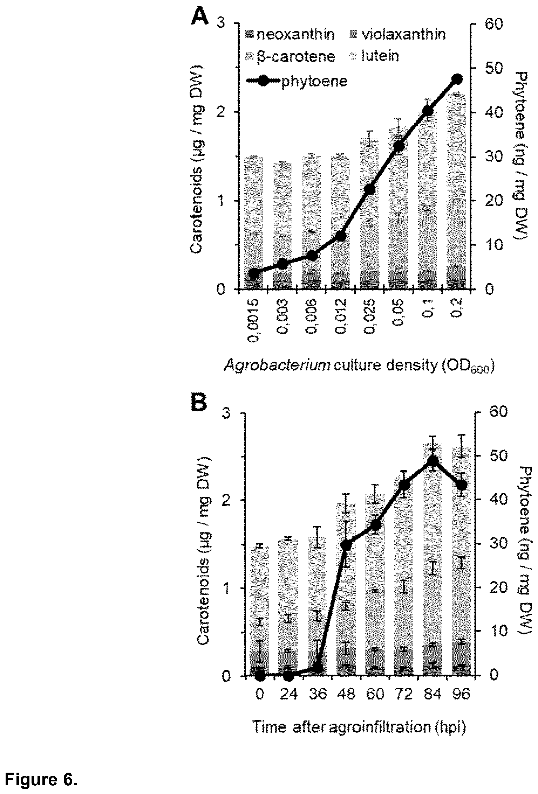

**Figure 6****. Phytoene levels need to reach an activation threshold to induce chloroplast-to-chromoplast differentiation.** A, Phytoene and downstream carotenoid contents in *N. benthamiana* leaves infiltrated with serial dilutions of *A. tumefaciens* cultures to express (p)crtB at different levels. B, Phytoene and downstream carotenoid contents at different time points after agroinfiltration of *N. benthamiana* leaves with constructs to express (p)crtB. In all cases, values correspond to the mean and SD values.

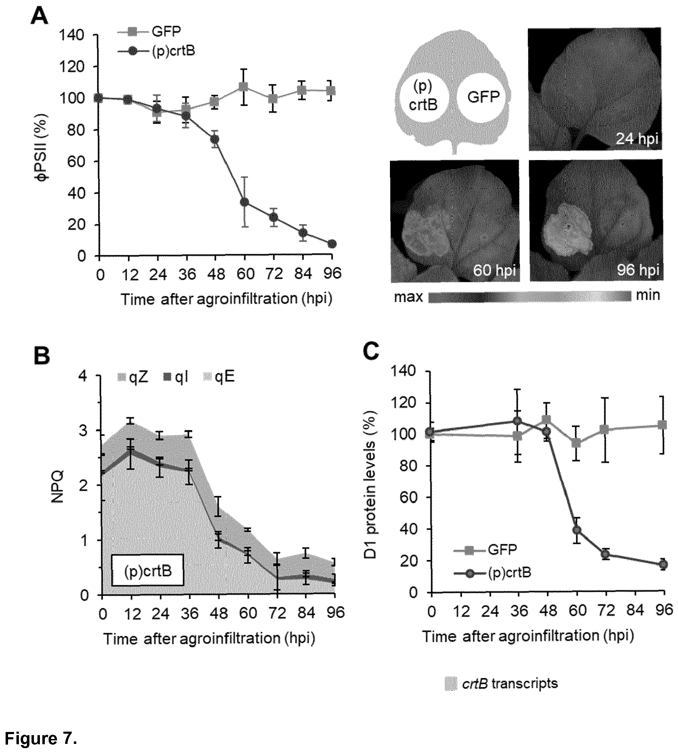

**Figure 7****. Time-course of chloroplast-to-chromoplast differentiation.** A, Effective quantum yield in leaf sections at different time points after agroinfiltration with the indicated constructs. Representative images are shown. B, Non-photochemical quenching in leaf sections at different time points after agroinfiltration with (p)crtB. C, D1 protein contents at different time points after agroinfiltration with the indicated constructs. Values correspond to the mean and SD values.

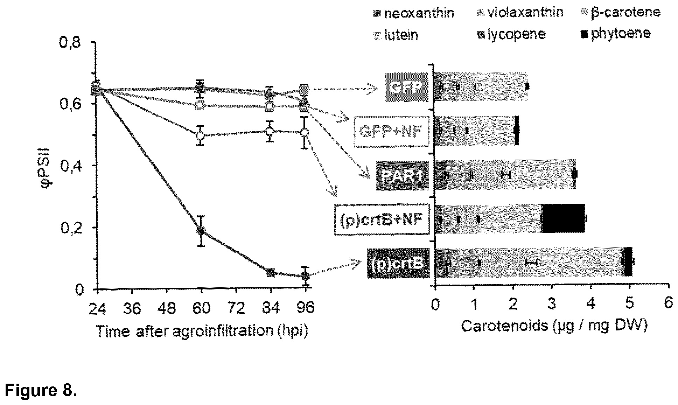

**Figure 8****. Carotenoid overaccumulation is necessary but not sufficient to transform leaf chloroplasts into chromoplasts.** Effective quantum yield in leaf sections at different time points after agroinfiltration with the indicated constructs. Samples treated with norflurazon (NF) are boxed in white. At 96 hpi, carotenoid contents were quantified. Values correspond to the mean and SD values.

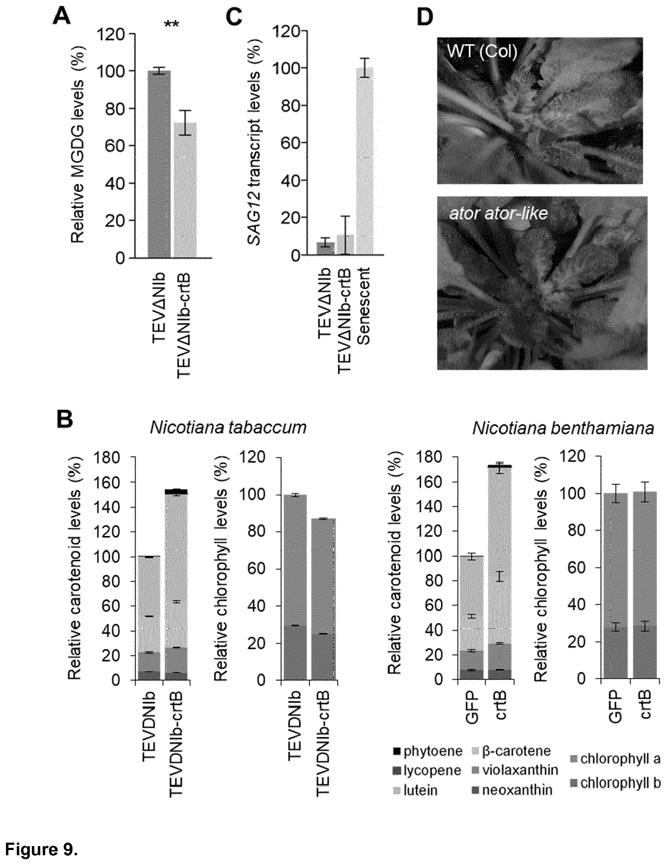

**Figure 9****. Comparison of crtB-triggered phenotypes in different plants.** A, MGDG levels in *N. tabacum.* B, Photosynthetic pigment levels in *N. tabacum* and *N. benthamiana.* C, *SAG12* gene expression in *N. tabacum.* D, Representative images of crtB-infected leaves from an Arabidopsis wild-type Columbia (WT Col) plant and an OR-defective double *ator ator-like* mutant.

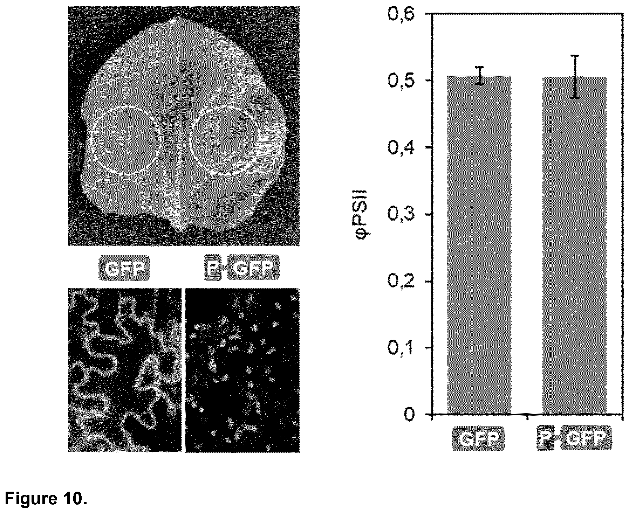

**Figure 10****. Plastid-targeted GFP does not affect chloroplast development.** *N. benthamiana* leaves were agroinfiltrated with constructs to express a plastid-targeted version of GFP (P-GFP) or the untargeted GFP protein (upper left picture). At 96 hpi, agroinfiltrated tissue was examined under a confocal laser scanning microscope to confirm the localization of P-GFP in chloroplasts and GFP in the cytosol (lower left pictures). Effective quantum yield of PSII was identical in both samples (plot in the right).

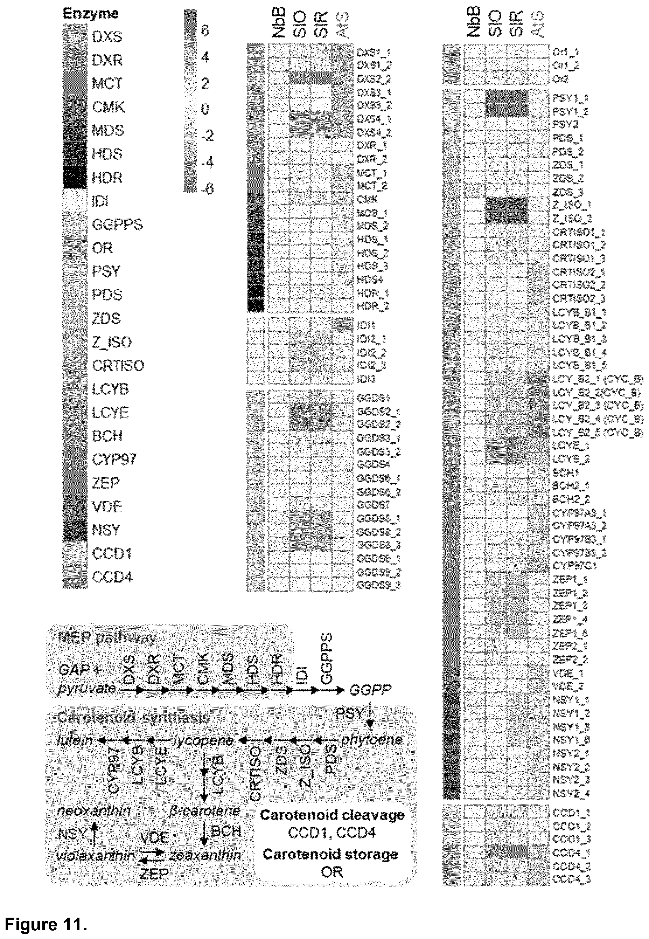

**Figure 11****. Chromoplast differentiation in *N. benthamiana* leaves does not require changes in the expression of carotenoid-related genes.** Heatmap represents log2FC values in *N. benthamiana* leaves (crtB vs. GFP, NbB), tomato fruit (light ripe vs. mature green, SLO, and red ripe vs. mature green, SIR) and Arabidopsis leaves (30D senescent vs. 16D controls, AtS).

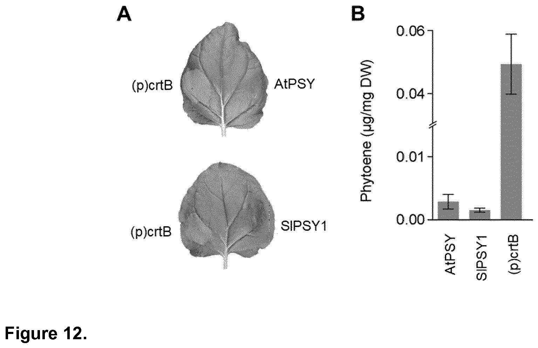

**Figure 12****. Plant PSY enzymes do not induce chromoplast differentiation in leaves.** A, Phenotypes of leaf tissue expressing AtPSY, SIPSY1, or crtB(p). B, Phytoene content in the leaf sections shown in A.

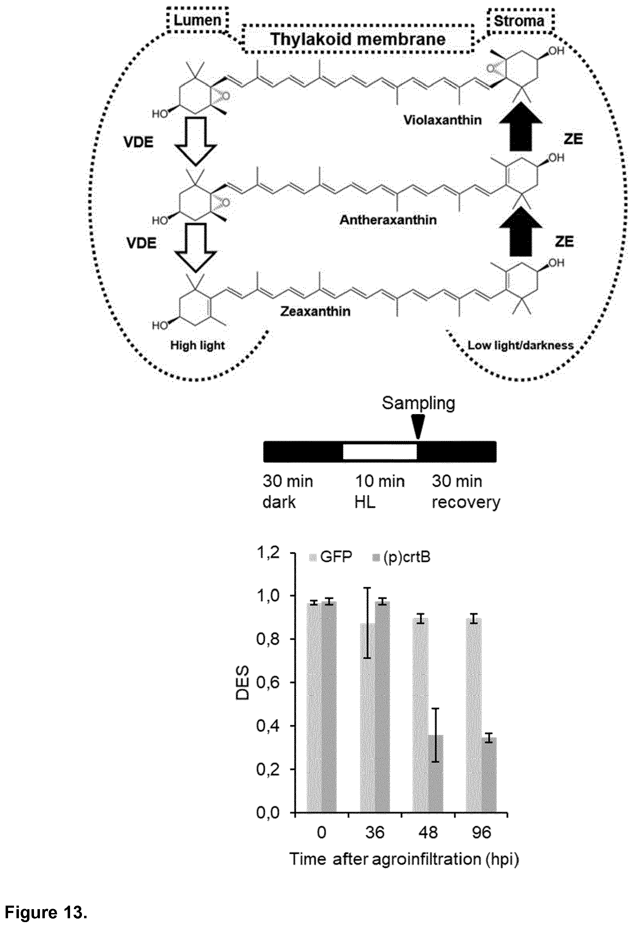

**Figure 13****. crtB-mediated chromoplast differentiation impairs the xanthophyll cycle.** DES, de-epoxidation state calculated as (Zx+0.5×Ax)/(Zx+Ax+Vx), where Zx, Ax and Vx are the concentrations of zeaxanthin, antheraxanthin and violaxanthin, respectively.

### DESCRIPTION OF THE INVENTION

### Definitions

A "recombinant nucleic acid" designates a nucleic acid which has been engineered and is not found as such in wild type organisms. In some particular embodiments, this term may refer to a gene operably linked to a promoter that is different from its naturally occurring promoter.

The term "gene" designates any nucleic acid encoding a protein. The term gene encompasses DNA, such as cDNA or gDNA, as well as RNA. The gene may be first prepared by e.g., recombinant, enzymatic and/or chemical techniques, and subsequently replicated in a host cell or an in vitro system. The gene typically comprises an open reading frame encoding a desired protein. The gene may contain additional sequences such as a transcription terminator or a signal peptide.

The term "operably linked" means a configuration in which a control sequence is placed at an appropriate position relative to a coding sequence, in such a way that the control sequence directs expression of the coding sequence.

The term "control sequences" means nucleic acid sequences necessary for expression of a gene. Control sequences may be native or heterologous. Well-known control sequences and currently used by the person skilled in the art will be preferred. Such control sequences include, but are not limited to, a leader, polyadenylation sequence, propeptide sequence, promoter, signal peptide sequence, and transcription terminator. Preferably, the control sequences include a promoter and a transcription terminator.

The term "expression cassette" denotes a nucleic acid construct comprising a coding region, i.e. a gene, and a regulatory region, i.e. comprising one or more control sequences, operably linked. Preferably, the control sequences are suitable for plant host cells.

As used herein, the term "signal peptide", may refer to sequences that when in translated into functional protein enhance the traffic of said protein into specific sub-cellular compartments, in the specific case of the present invention, into plastids, and more specifically into chloroplasts. Many signal peptides have been identified to date (from Rubisco i.e., Ribulose-1,5-bisphosphate carboxylase/oxygenase EC 4.1.1.39 , or in US20020178467A1, or in https://doi.org/10.1016/j.tplants.2013.04.003).

As used herein, the term "expression vector" means a DNA or RNA molecule that comprises an expression cassette. Preferably, the expression vector is a linear or circular double stranded DNA molecule.

As used herein, the term "native" or "endogenous", with respect to a specific organism (e.g. bacterium or plant), refers to a genetic element or a protein naturally present in said organism. The term "heterologous", with respect to a specific organism, refers to a genetic element or a protein that is not naturally present in said organism.

As used herein, the term "sequence identity" or "identity" refers to the number (%) of matches (identical amino acid residues) in positions from an alignment of two polypeptide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithms (e.g. Needleman and Wunsch algorithm; Needleman and Wunsch, 1970) which aligns the sequences optimally over the entire length, while sequences of substantially different lengths are preferably aligned using a local alignment algorithm (e.g. Smith and Waterman algorithm (Smith and Waterman, 1981) or Altschul algorithm (Altschul et al., 1997; Altschul et al., 2005)). Alignment for purposes of determining percent amino acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/). Those skilled in the art can determine appropriate parameters for measuring alignment, including any algorithms needed to achieve maximal alignment over the full length of the sequences being compared. For purposes herein, % amino acid sequence identity values refers to values generated using the pair wise sequence alignment program EMBOSS Needle that creates an optimal global alignment of two sequences using the Needleman-Wunsch algorithm, wherein all search parameters are set to default values, i.e. Scoring matrix = BLOSUM62, Gap open = 10, Gap extend = 0.5, End gap penalty = false, End gap open = 10 and End gap extend = 0.5

As used in this specification, the term "about" refers to a range of values ± 10% of the specified value. For example, "about 20" includes ± 10 % of 20, or from 18 to 22. Preferably, the term "about" refers to a range of values ± 5 % of the specified value.

As used herein, the term "CrtB" or "phytoene synthase" refers to a phytoene synthase enzyme (EC 2.5.1.32) encoded by a *crtB* gene which catalyzes the condensation of two molecules of geranylgeranyl diphosphate (GGPP) to give phytoene. According to the organism, the nomenclature of the above identified enzyme and encoding gene may vary. However, for the sake of clarity, in the present specification, these terms are used independently from the origin of the enzymes or genes. Preferably, the term "CrtB" may refer to any of the sequences identified in table 1 below:

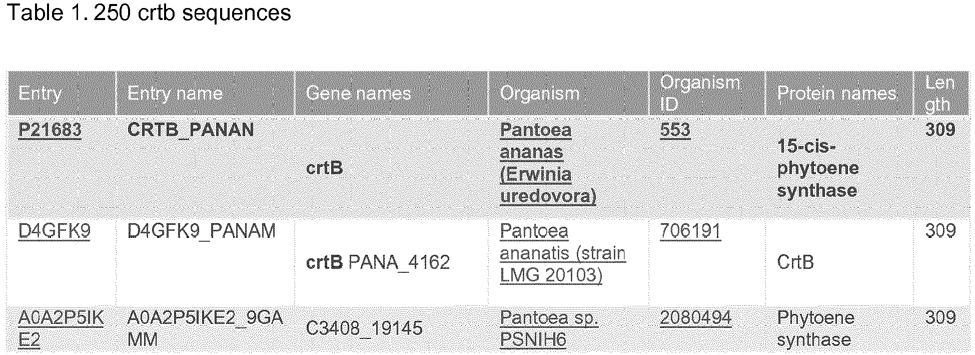

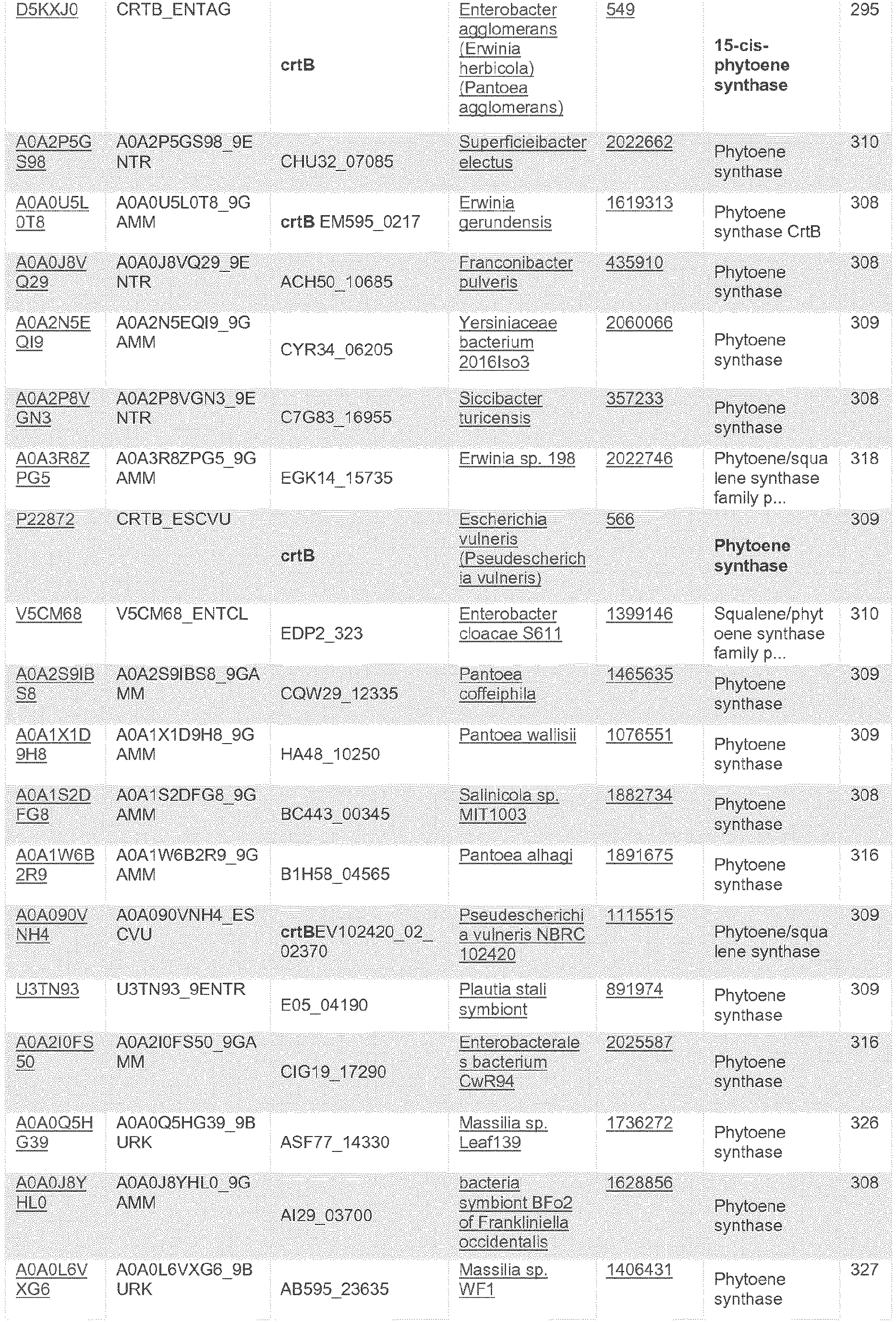

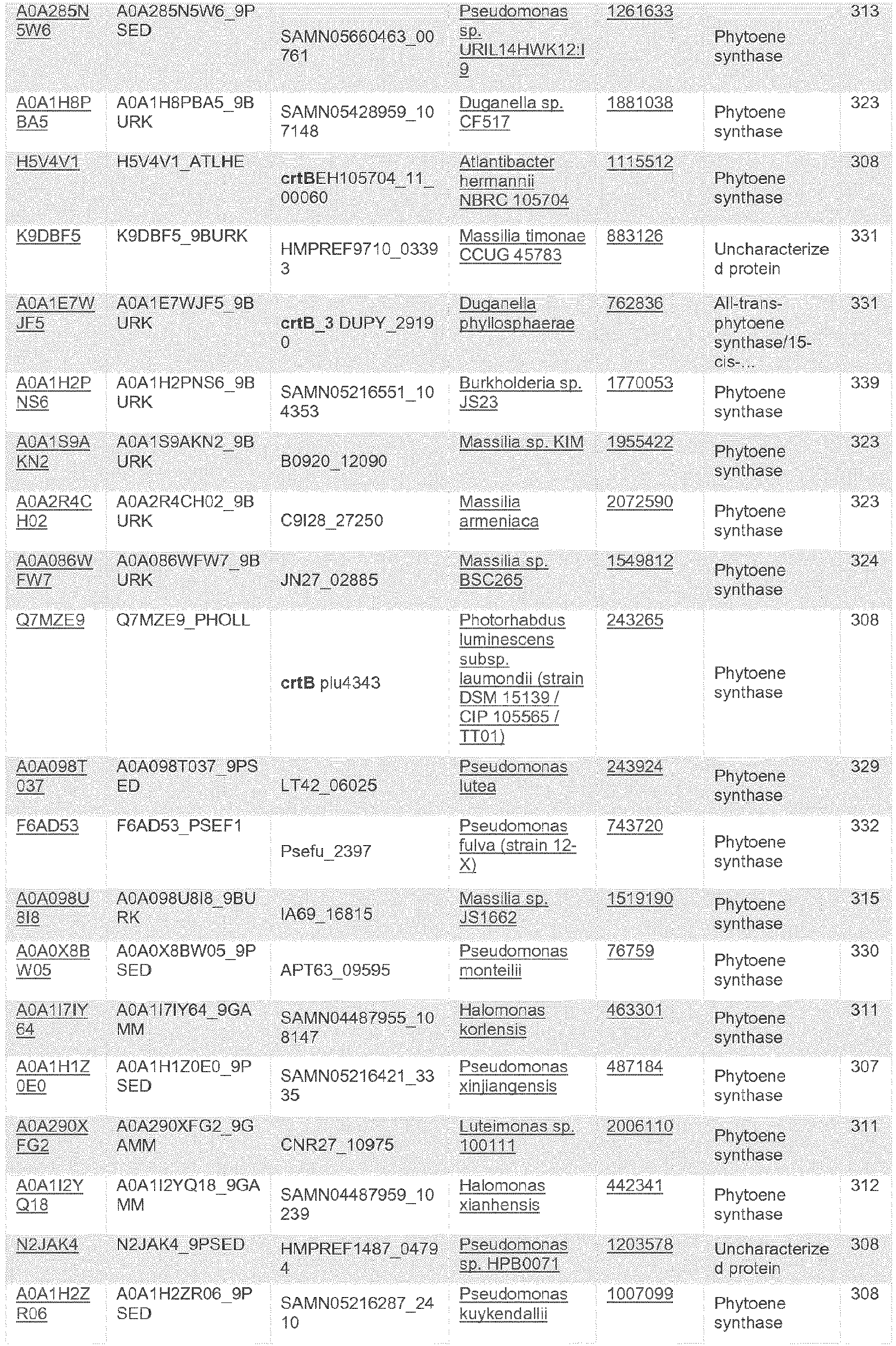

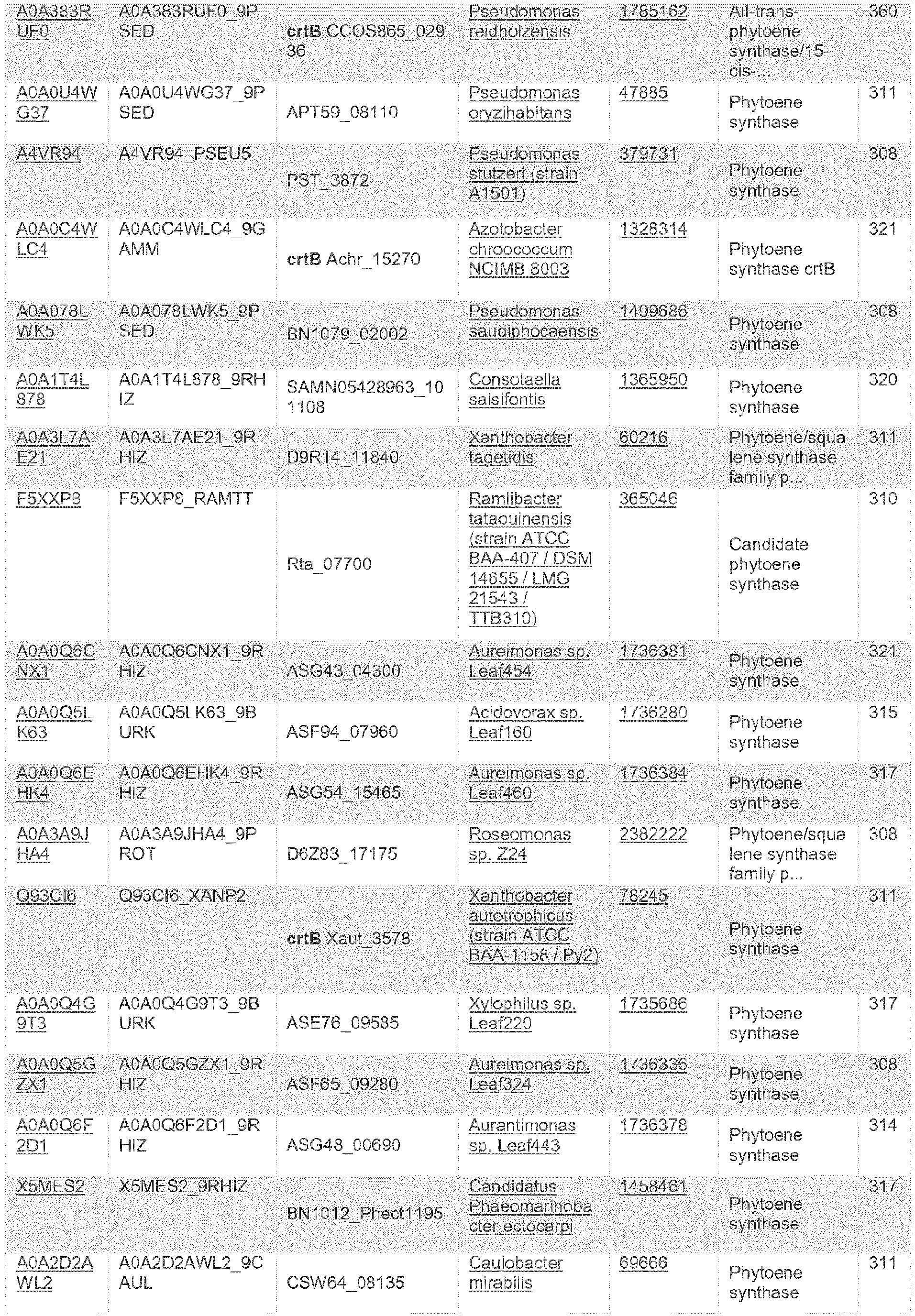

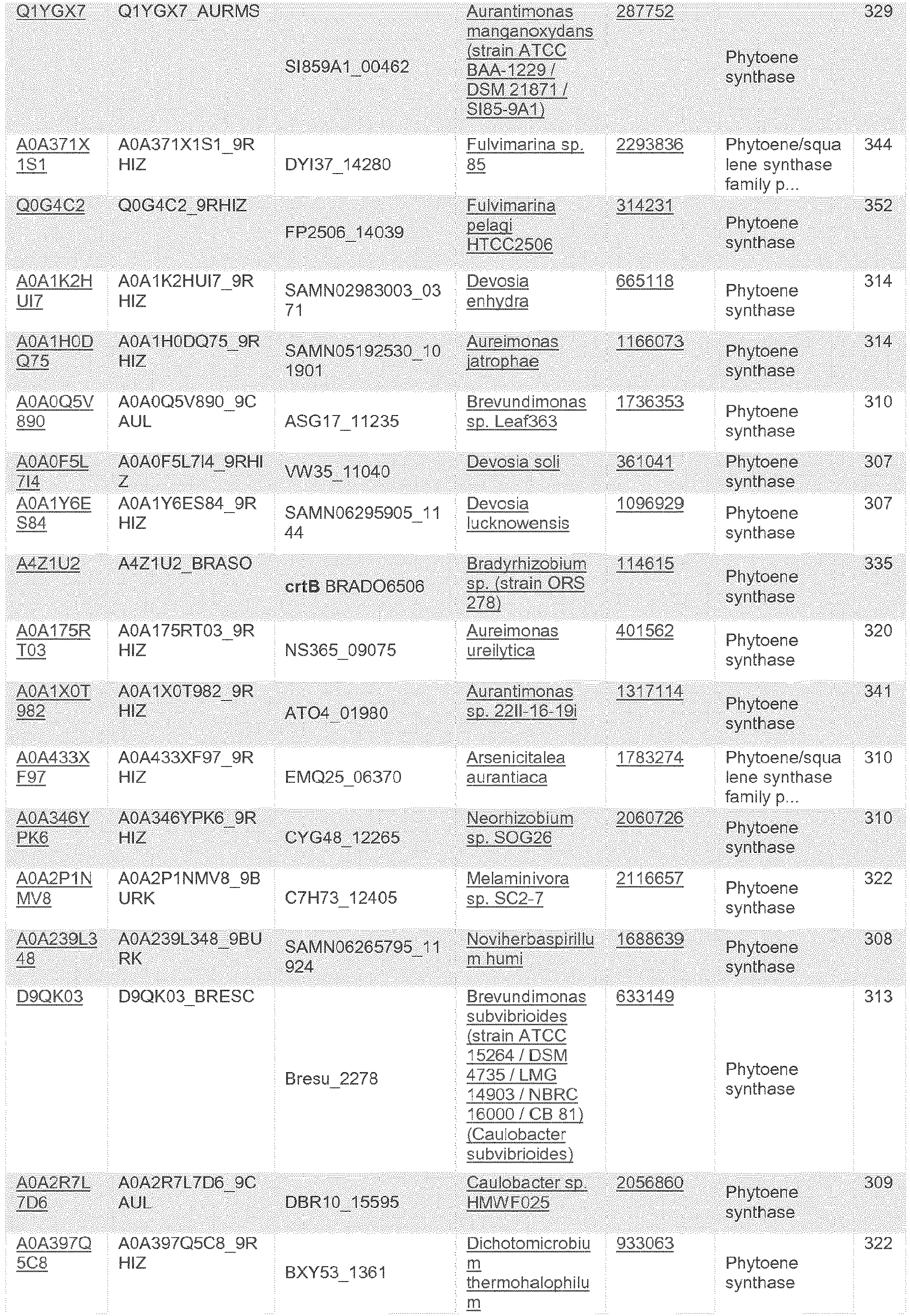

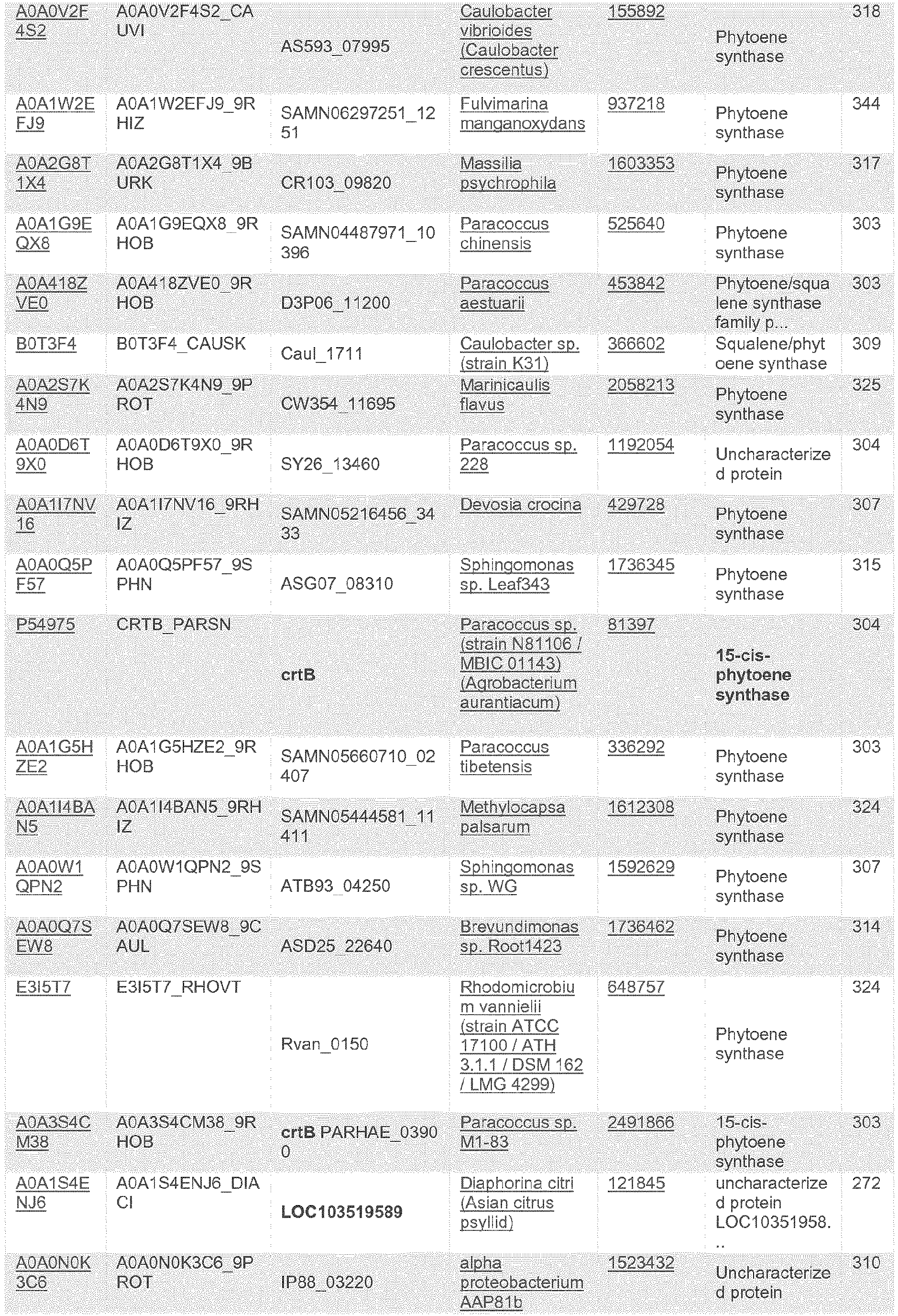

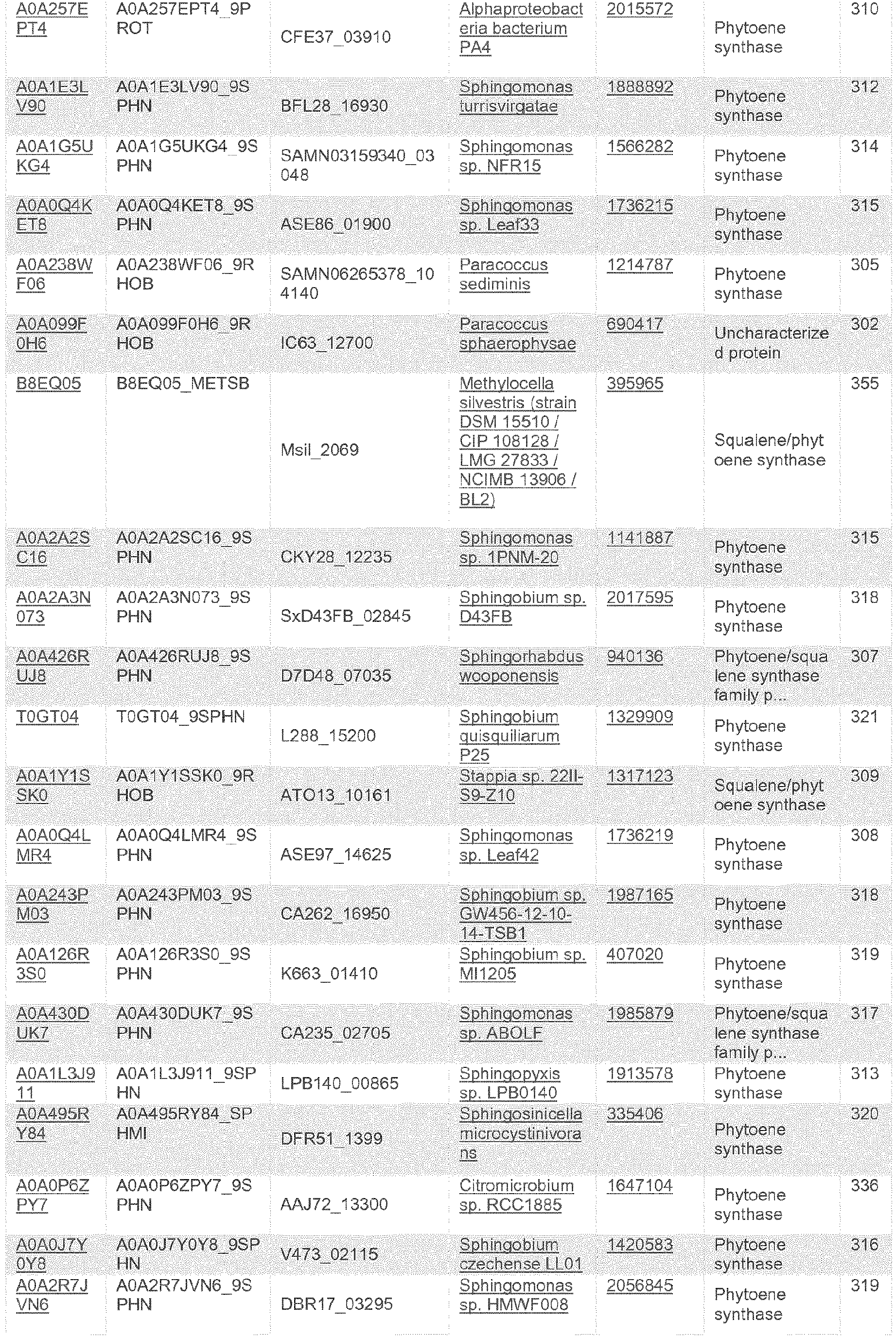

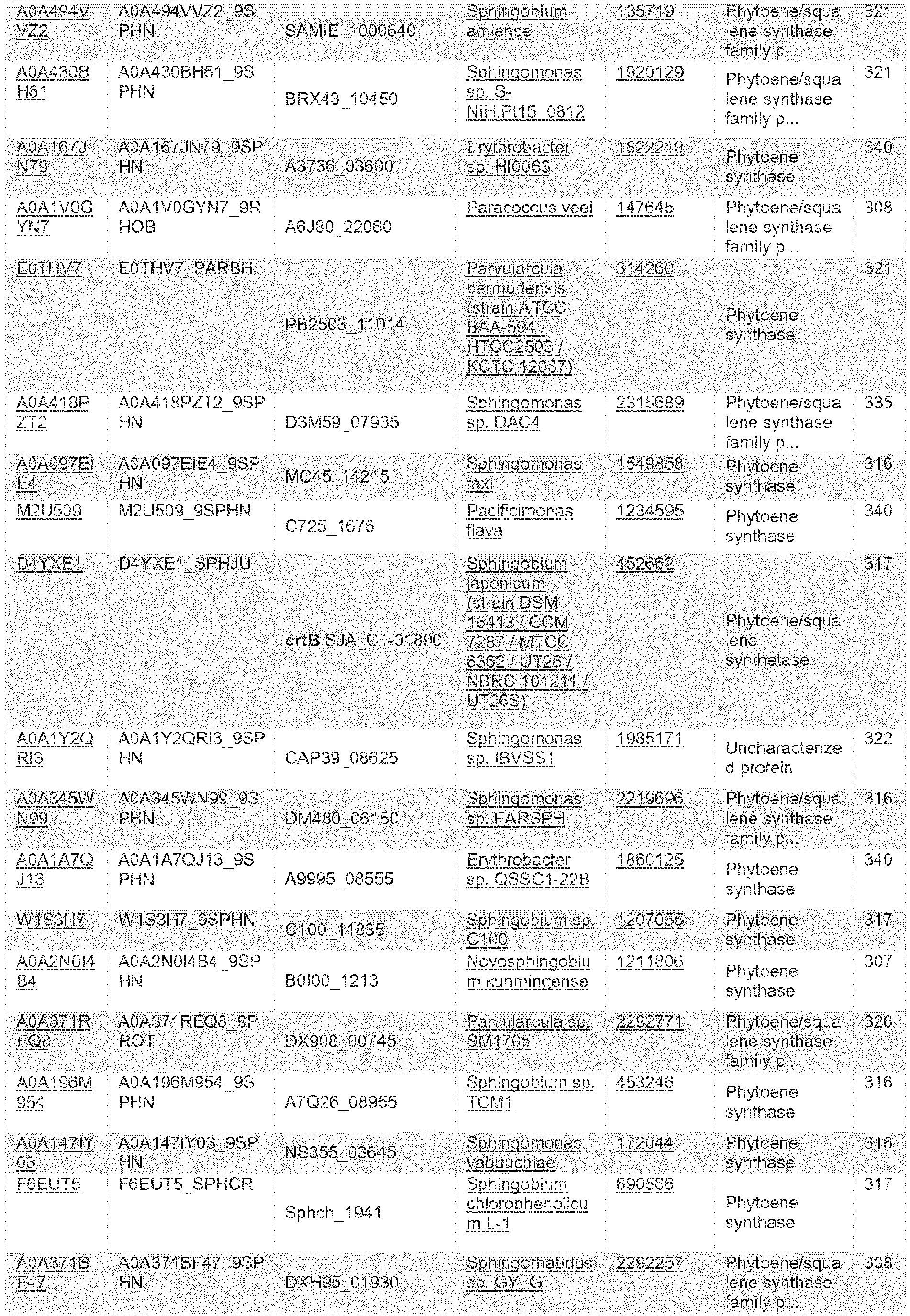

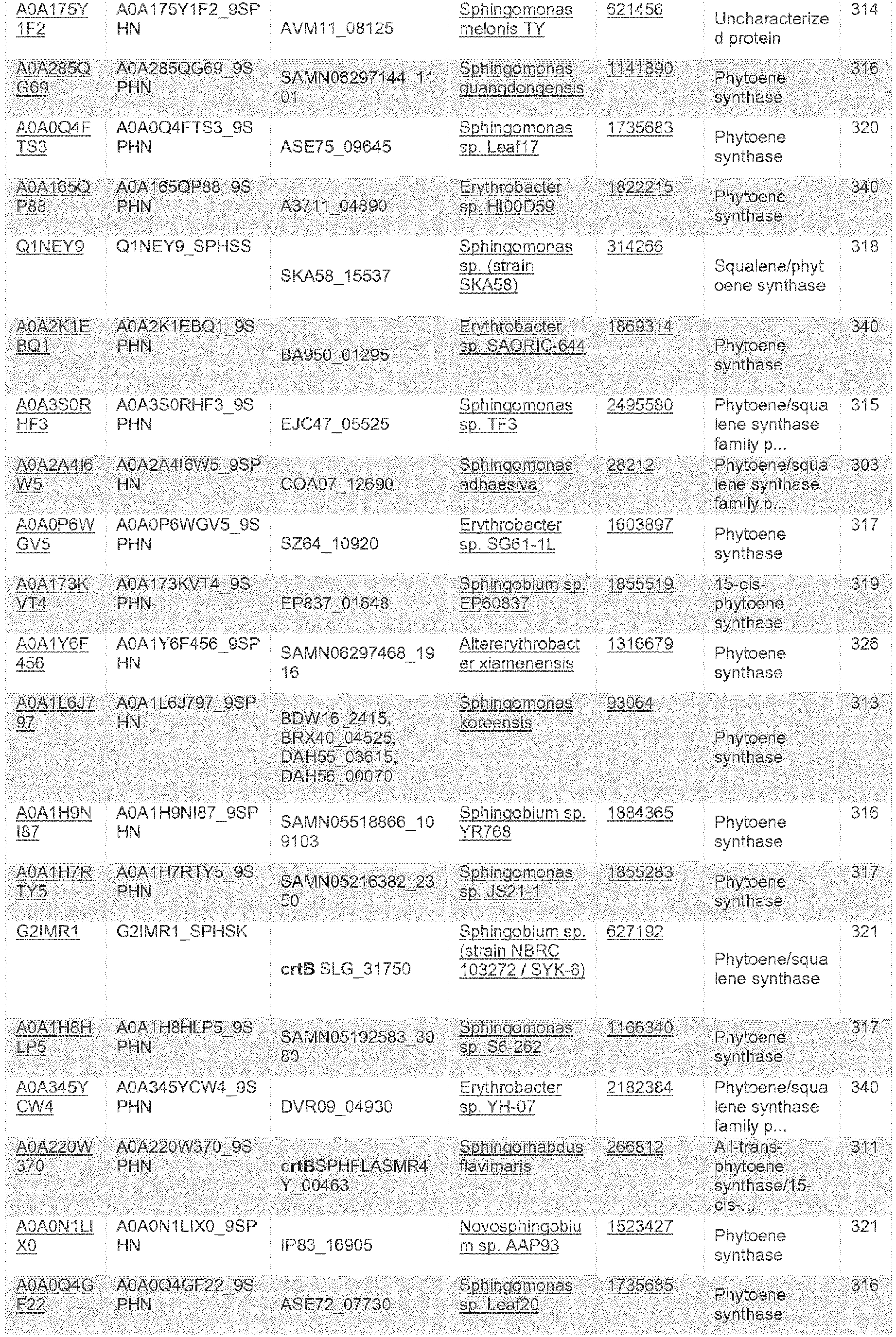

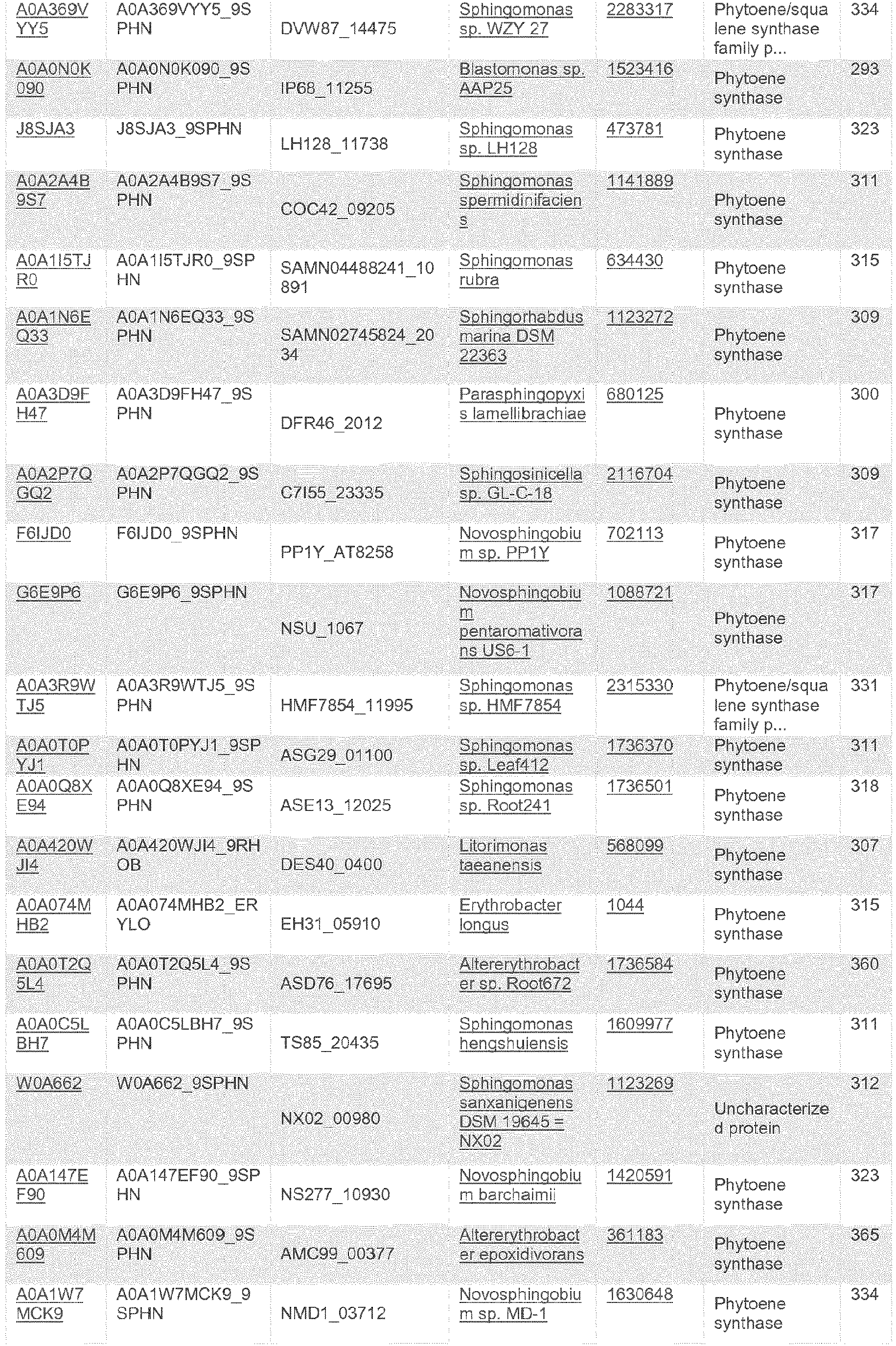

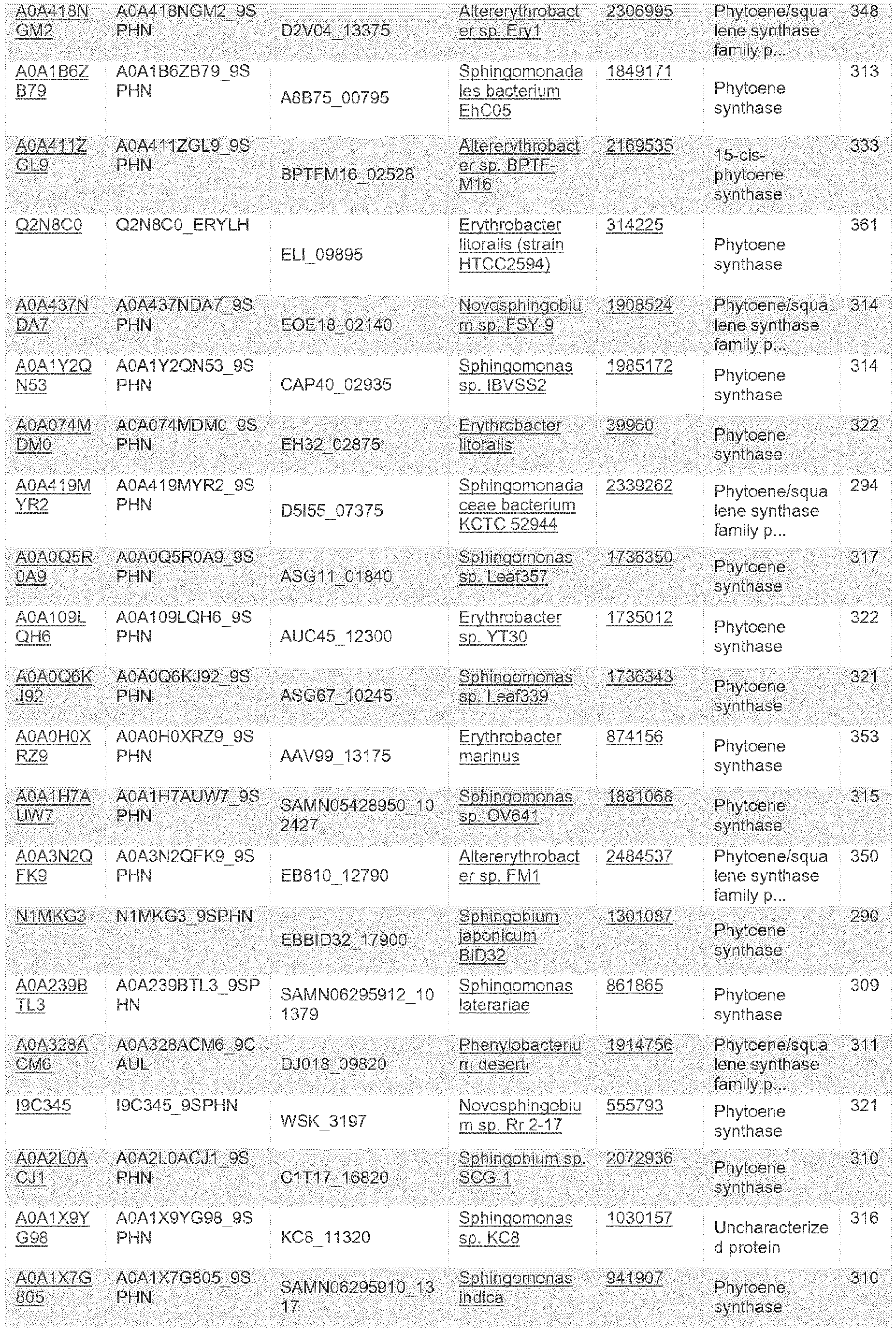

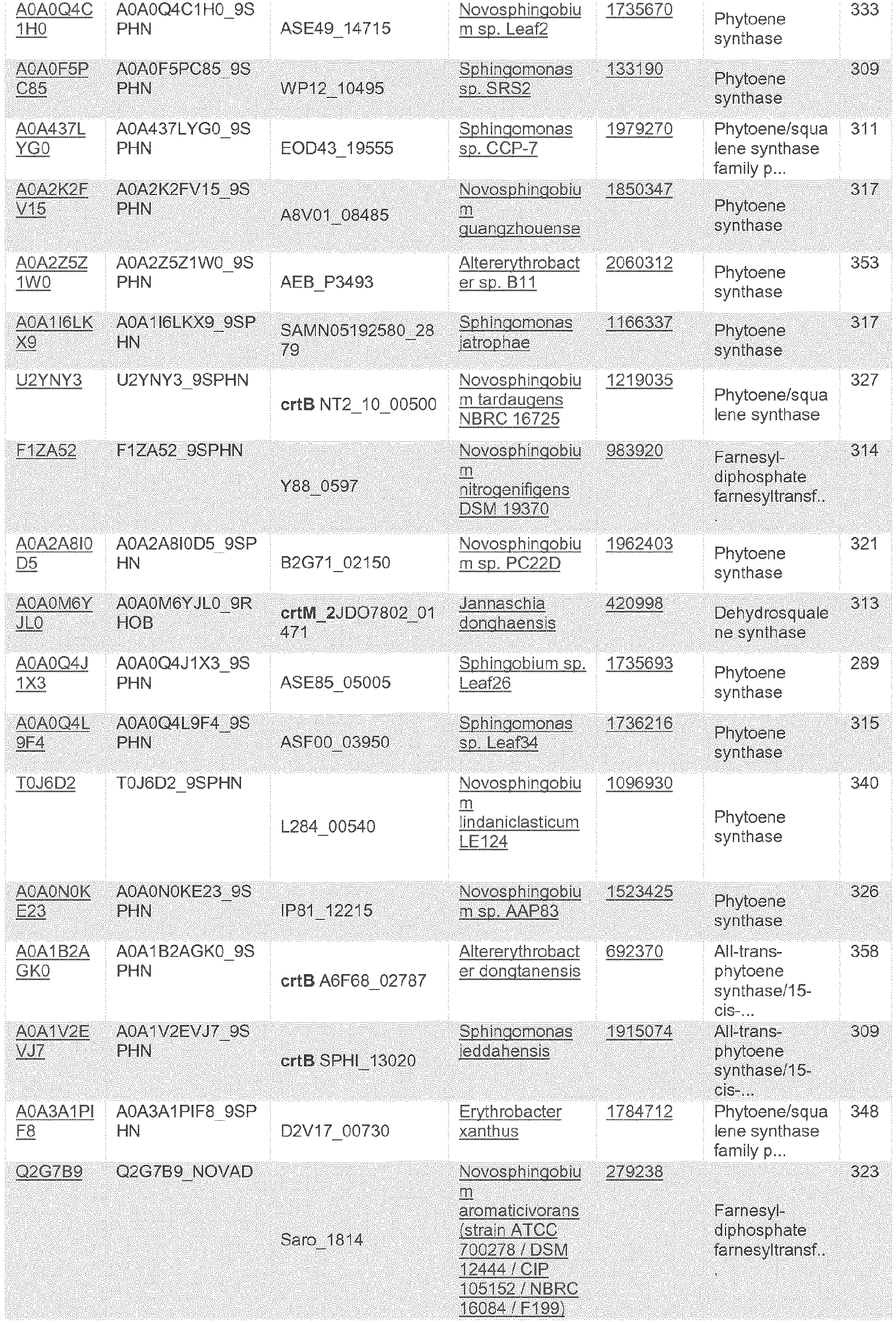

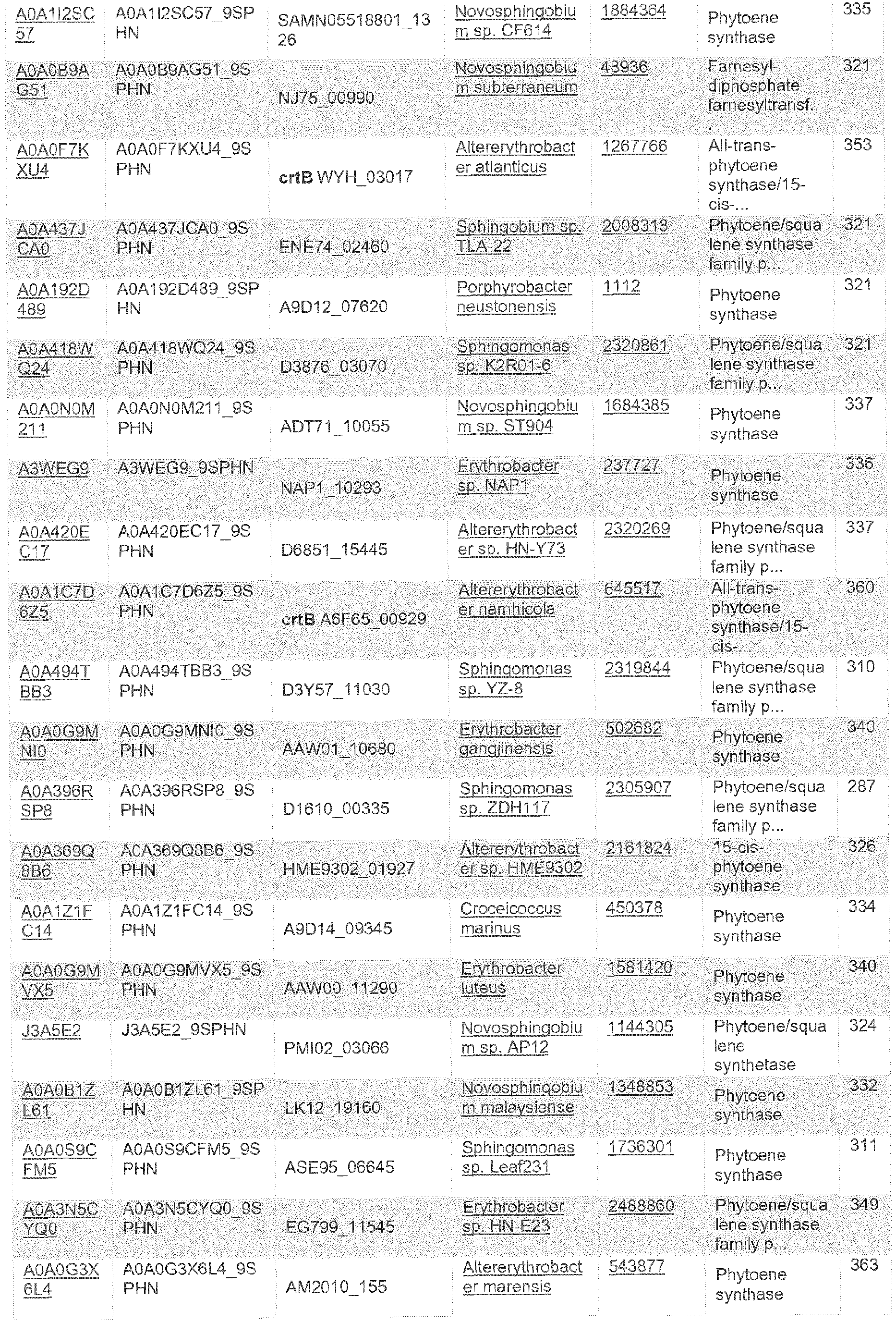

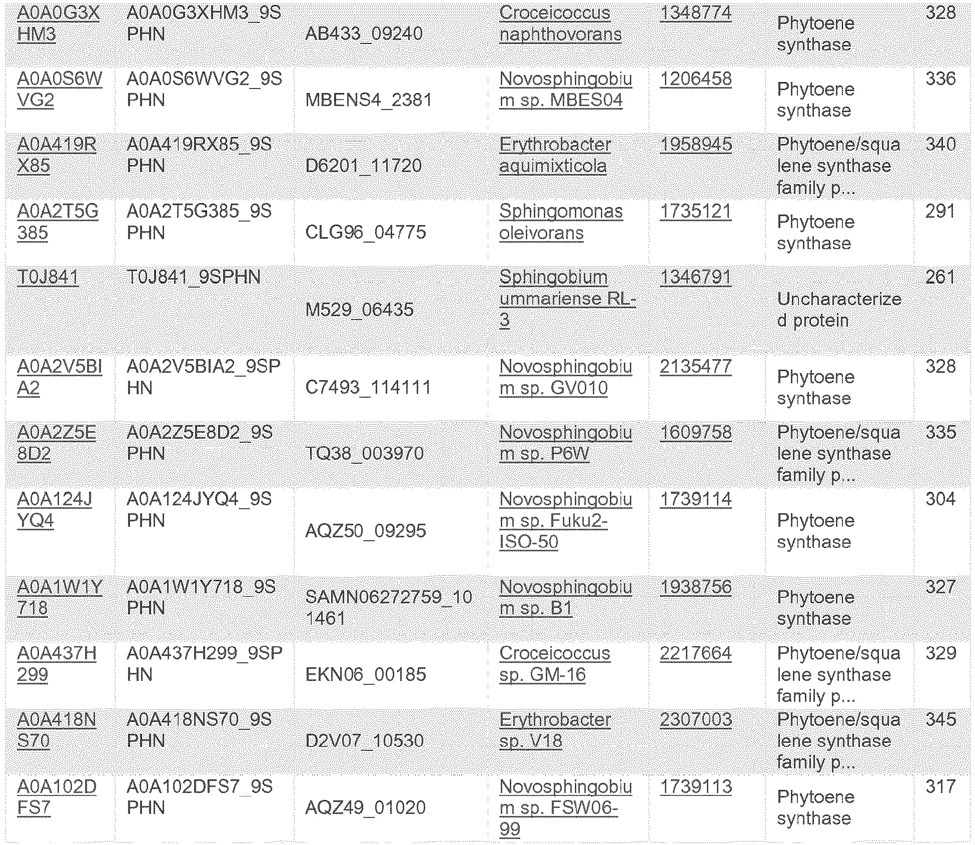

Preferably, the present invention refers to the following CrtB sequences corresponding to the phytoene synthase of *Pantoea ananatis* (acessions AXU24940; AHG94989; WP_013027995; WP_110015463; WP_105080745; WP_042676315; WP_105077524; WP_041931046 or WP_028722638), or to the phytoene synthase of *Pantoea stewarti* (AAN85600), or to the phytoene synthase of *Pantoea conspicua* (WP_094119067), or to the phytoene synthase of *Pantoea agglomerans* (AF289043), or to the phytoene synthase of *Pantoea eucalypti* (WP_140915947), or to the phytoene synthase of *Pantoea vagans* (WP_135910885), or to the phytoene synthase of *Pantoea deleyi* (WP_140916915) or more generally to the phytoene synthase of *Pantoea sp.* defined through the GenBank accession HAB23577.

As used herein, the term "phytoene" refers to any phytoene isomer, and preferably to 15-cis phytoene.

The references herein are included here in its full extent.

### Description

The regulation of plastids is a core process in plants that remains poorly defined. In this invention, we have shown that the supply of phytoene is a primary determinant of chloroplast-to-chromoplast differentiation. Based on our results, we propose that a metabolic threshold switch mechanism promotes the loss of chloroplast identity when phytoene is supplied in excess. This result also allows explaining why previous attempts using plant PSY enzymes (Maass, Arango et al. 2009, Yuan, Owsiany et al. 2015), which presumably did not reach this activation threshold, have not resulted in chromoplast differentiation in leaves. Our results are consistent with the existence of a two-step process responsible for the transformation of leaf chloroplasts into chromoplasts. First, inducing the production of a sufficiently large amount of phytoene elicits the initial changes, and secondly, increased accumulation of carotenoids due to sustained phytoene availability but also to phytoene-triggered changes in chloroplast membranes completes the differentiation of chloroplasts into chromoplasts. While our conclusions are based on an artificial system (i.e. the expression of a bacterial gene in chloroplast containing cells), the similarity of transcriptomic profiles between this process and fruit ripening strongly points to a general mechanism for chloroplasts to become chromoplasts without the need of a developmental signal. We propose that an initial boost of phytoene production might be necessary to break chloroplast identity in primary photosynthetic tissues such as leaves. However, it might not be required in organs or/and stages in which chloroplast identity is weak (e.g. when photosynthesis is not a functional requirement as in green fruits) or non-existent (e.g. in dark-grown calli, tubers, or roots). Consistent with this conclusion, constitutive overexpression of tomato or Arabidopsis genes encoding PSY enzymes resulted in chromoplast-like structures arising in mature green fruit of tomato (Fraser et al. 2007) and non-photosynthetic tissues of Arabidopsis (Maass et al. 2009) but had no effect in the leaves of these transgenic tomato and Arabidopsis plants (Fraser et al. 2007; Maass et al. 2009). A lower competency of leaf chloroplasts to develop into chromoplasts would also explain why upregulation of ORANGE (OR) triggers carotenoid overaccumulation and chromoplast differentiation in tomato fruit, potato tubers, cauliflower curds, or Arabidopsis calli, but does not work in the leaves of any of these plants (Li et al. 2001; Lopez et al. 2008; Yuan et al. 2015; Yazdani et al. 2019).

Phytoene-induced loss of chloroplast identity might be caused by direct effects of this uncolored carotenoid on the photosynthetic pigment-protein complexes responsible for harvesting sunlight and transferring excitation energy to the photosystems (Domonkos et al. 2013; Liguori et al. 2017). Another possibility is an indirect effect derived from retrograde signals generated when phytoene levels override the threshold. These unknown signals would trigger changes in nuclear gene expression presumably causing the remodeling or pre-conditioning of chloroplast membrane structures in a first phase of the chromoplast differentiation process.

In a second phase, which likely overlaps with the first one, conversion of phytoene into downstream carotenoids in pre-conditioned chloroplasts is required to eventually generate a chromoplast. Without pre-conditioning, carotenoids levels can increase but chloroplasts stay photosynthetically active and chromoplasts do not differentiate, (Roig-Villanova et al. 2007; Bou-Torrent et al. 2015). Moreover, without carotenoids downstream of phytoene, pre-conditioned chloroplasts remain unchanged and chromoplasts do not differentiate. It is likely that carotenoid accumulation occurs concomitantly with the remodeling of the internal plastid structures and that both factors synergistically activate each other. The new structures created following the disassembly of the photosynthetic grana and thylakoids likely contribute to reach high carotenoid levels by accommodating increasing amounts of carotenoids and by preventing their degradation (Sadali et al. 2019; Jarvis and Lopez-Juez 2013; Sun et al. 2018; Egea et al. 2010). They might additionally enhance carotenoid production by stimulating the activity of endogenous carotenoid biosynthetic enzymes (including PSY), many of which are membrane-associated (Ruiz-Sola and Rodriguez-Concepcion 2012).

The structural changes associated with the crtB-mediated chloroplast to chromoplast transformation involve reorganization of the plastidial proteome but also require global reprogramming of nuclear gene expression and primary metabolism. We also show that shut down of photosynthesis relies on enhanced respiration for an enhanced supply of energy and carbon precursors to synthesize carotenoids. It is likely that implementing these changes relies on retrograde signals produced by transforming plastids. Carotenoids can function as precursors for the biosynthesis of signaling molecules that regulate many developmental processes in plants, including plastid development (Avendano-Vazquez et al. 2014; Van Norman et al. 2014; Hou et al. 2016; Wang et al. 2019). The observation that Arabidopsis mutants defective in carotenoid cleavage dioxygenase activity in the cytosol and the plastids are not affected in the crtB-dependent leaf chromoplast differentiation process suggests that signals independent of these enzymes or of carotenoids are responsible for eliciting the changes in nuclear gene expression and cell metabolism supporting chromoplast biogenesis.

Other studies have shown that increasing the supply of phytoene and/or promoting the metabolic activity of the carotenoid pathway can induce the differentiation of leucoplasts (i.e., plastids devoid of pigments) to chromoplasts in non-colored tissues such as calli, tubers, and roots (Lopez, Van Eck et al. 2008, Maass, Arango et al. 2009, Bai, Rivera et al. 2014, Yuan, Owsiany et al. 2015, Schaub, Rodriguez-Franco et al. 2018). We discovered that producing high enough phytoene levels is a necessary first step towards transforming leaf chloroplasts into carotenoid-overaccumulating chromoplasts.

Recent work also showed that transplastomic plants with a synthetic metabolic pathway for producing the algae carotenoid astaxanthin differentiated leaf plastids that lost most of their endogenous carotenoids and chlorophylls and hence their chloroplast identity (Lu, Stegemann et al. 2017). No information was reported on the levels of phytoene in these lines. However, it is possible that the substitution of endogenous carotenoids by asthaxanthin might interfere with the activity of photosynthetic pigment-protein complexes similarly to phytoene overaccumulation. Chromoplast biogenesis has long been thought to be differently regulated by tissue-specific mechanisms subjected to a tight developmental control (Egea, Barsan et al. 2010). However, these emerging descriptions, together with results reported here, clearly point toward a common regulatory mechanism linking the activity of the carotenoid pathway (i.e. metabolic rather than developmental cues) to the regulation of chromoplast biogenesis.

In summary, we reveal a tool that, for the first time, allows the differentiation of chromoplasts from leaf chloroplasts. This very simple tool that we describe here to induce chromoplast biogenesis on demand can be applied to improve the nutritional quality of crops or other biotechnological applications. For example, our system could be used to increase carotenoid and tocopherol contents in leaf vegetables and/or forage crops for animal feed and/or cell cultures by placing crtB expression under the control of a specific agrochemical such as Routine (Okada, Nemoto et al. 2017), or other induction systems known in the prior-art AlcR/AlcA (ethanol inducible); GR fusions, GVG, and pOp/LhGR (dexamethasone inducible); XVE/OlexA (beta-estradiol inducible); or heat shock induction (Borghi, 2010). These and other agrochemicals or methods could be applied before harvest to induce chloroplast-to-chromoplast differentiation once reaching maximum crop biomass. Furthermore, we foresee that future synthetic biology approaches could be used to couple the activity of new biosynthetic pathways with controlled chromoplast biogenesis to enhance the production capacity of chromoplast located added-value chemicals in plants.

Therefore, a first aspect of the invention refers to a method of inducing chloroplast-to-chromoplast differentiation biogenesis in cell cultures, leaf vegetables, green fruits and/or forage crops that comprises the delivery, by preferably, but not limited to, agroinfiltration or any alternative method such as microbombardment or the use of viral vectors, of a recombinant plasmid or expression cassette in the leaf vegetables, green fruits or forage crops, wherein said construct is characterized in that it comprises a heterologous nucleic acid encoding a polypeptide exhibiting phytoene synthase activity and/or may overexpress an endogenous nucleic acid encoding a polypeptide exhibiting phytoene synthase activity, wherein said polypeptide exhibiting phytoene synthase activity further comprises a plastid-targeting signal in its N-terminus, and wherein said construct is capable of delivering an amount of phytoene in the chloroplasts of the plant tissue sufficient to induce chloroplast to chromoplast differentiation in said plant tissue, preferably said construct is capable of delivering at least about **0.01 µg/mg DW** of phytoene in the chloroplasts of the plant tissue.

In the context of the present invention, the stament "an amount of phytoene in the chloroplasts of the plant tissue sufficient to induce chloroplast to chromoplast differentiation in said plant tissue" can be the amount that results in the reduction of at least 50% of chlorophyll content in leaves (w/w), **or** 50% reduciton of photosynthesis-related parameters such as effective quantum yield at PSII (φPSII) **or** non-photochemical quenching (NPQ) when measured against the time where no phytoene is applied on the green tissues or cells of interest. Depending on the plant, tissue or cell of interest, as illustrated below with the examples of spinach, brussel sprouts, forage crops or avocado, the construct can be modified using adecuate promoters and phytoene synthases able to achieve the above mentioned reduction in the above enumerated measures of chloroplast photosynthetic capacity (including but not restricted to, chlorophyll content, effective quantum yield or non-photochemical quenching).

The skilled person will be able to determine the concentration of chlorophyll in leaves using methods as the one published by Schertz in 1928 (F.M. Schertz (1928) The quantitative deteermination of chlorophyll. Plant Physiology 1928 Jul;3 (3):323-334), or more inmediate methods as new as the one described by Cortazar (Cortazar, 2015 Cortazar B., et al. Quantification of plant chlorophyll content using google glass. Lab on Chip 2015; (15) 1708). Likewise, effective quantum yield measurement and non-photochemical quenching (NPQ) can be executed with currently available instruments (as described herein using a MAXI-PAM fluorometer) or alternatively following the teachings of Murchie et al. (2013), Murchie et. Al. Chlorophyll fluoreescene analysis: a guide to good proactice and understanding some neew applications. Journal of experimental Botany: 64 (13) 3983).

A second aspect of the invention refers to a method to increase carotenoid and tocopherol contents in cell cultures, leaf vegetables, green fruits and/or forage crops that comprises the delivery, by preferably, but not limited to, agroinfiltration or any alternative method such as microbombardment or the use of viral vectors, of a recombinant plasmid or expression cassette in the leaf vegetables, green fruits and/or forage crops, wherein said construct is characterized in that it comprises a heterologous nucleic acid encoding a polypeptide exhibiting phytoene synthase activity and/or may overexpress an endogenous nucleic acid encoding a polypeptide exhibiting phytoene synthase activity, wherein said polypeptide exhibiting phytoene synthase activity further comprises a plastid-targeting signal and wherein said construct is capable of delivering an amount of phytoene in the chloroplasts of the plant tissue sufficient to induce chloroplast to chromoplast differentiation in said plant tissue, preferably said construct is capable of delivering at least about **0.01 µg/mg DW** of phytoene in the chloroplasts of the plant tissue.

A third aspect of the invention refers to a method to enhance the production capacity of lipophilic chemicals in plants, the method comprising the delivery, by preferably, but not limited to, agroinfiltration or any alternative method such as microbombardment or the use of viral vectors, of a recombinant plasmid or expression cassette in cell cultures, leaf vegetables, green fruits and forage crops, wherein said construct is characterized in that it comprises a heterologous nucleic acid encoding a polypeptide exhibiting phytoene synthase activity and/or may overexpress an endogenous nucleic acid encoding a polypeptide exhibiting phytoene synthase activity, wherein said polypeptide exhibiting phytoene synthase activity further comprises a plastid-targeting signal and wherein said construct is capable of delivering an amount of phytoene in the chloroplasts of the plant tissue sufficient to induce chloroplast to chromoplast differentiation in said plant tissue, preferably said construct is capable of delivering at least about **0.01 µg/mg DW** of phytoene in the chloroplasts of the plant tissue.

A fourth aspect of the invention refers to a modified cell culture, leaf vegetable, green fruit and/or forage crop, or to a composition comprising said modified cell culture, leaf vegetable, green fruit and/or forage crop, wherein the modified cell culture, leaf vegetable, green fruit and/or forage crop comprises a recombinant plasmid or expression cassette (construct) that results in a enhanced phytoene synthase activity and accummulation within chloroplasts either by constitutive or inducible expression, that is, either inserted in the plant genome or through a transient method of expression, wherein said recombinant plasmid or expression cassette is characterized in that it comprises a heterologous nucleic acid encoding a polypeptide exhibiting phytoene synthase activity and/or may overexpress an endogenous nucleic acid encoding a polypeptide exhibiting phytoene synthase activity, wherein said polypeptide exhibiting phytoene synthase activity further comprises a plastid-targeting signal in its N-terminus, and wherein said construct is capable of delivering an amount of phytoene in the chloroplasts of the plant tissue sufficient to induce chloroplast to chromoplast differentiation in said plant tissue, preferably said construct is capable of delivering at least about **0.01 µg/mg DW** of phytoene in the chloroplasts of the plant tissue.

In the context of the present invention, plasmid construct is preferably understood as (p)crtB or crtbB or any plasmid encoding a phytoene synthase included in the list above and which is able to translocate the phytoene generating enzyme to the plant plastids of interest.

In the context of the present invention, (p)crtB is understood as 35S:(p)crtB-pGWB605 (SEQ ID NO 1) or as 35S:(p)crtB-pGWB405 (SEQ ID NO 2) two constructs driving *Pantoea ananatis* phytoene synthase expression with a functionally linked plastid-targeting signal of hydroxymethylbutenyl 4-diphosphate synthase from *Arabidopsis thaliana* able to translocate crtB to plastids and difering only in the selection marker/antibiotic and further identified as SEQ No ID 1 and 2.

>(p)crtB_stop_pGWB605
CTTTTCACGCCCTTTTAAATATCCGATTATTCTAATAAACGCTCTTTTCTCTTAGGTTTACCCGCCAATATATCCTGTC
AAACACTGATAGTTTAAACTGAAGGCGGGAAACGACAATCTGATCCAAGCTCAAGCTGCTCTAGCATTCGCCATTC
AGGCTGCGCAACTGTTGGGAAGGGCGATCGGTGCGGGCCTCTTCGCTATTACGCCAGCTGGCGAAAGGGGGATG
TGCTGCAAGGCGATTAAGTTGGGTAACGCCAGGGTTTTCCCAGTCACGACGTTGTAAAACGACGGCCAGTGCCAA
GCTTGCATGCCTGCAGGTCCCCAGATTAGCCTTTTCAATTTCAGAAAGAATGCTAACCCACAGATGGTTAGAGAGG
CTTACGCAGCAGGTCTCATCAAGACGATCTACCCGAGCAATAATCTCCAGGAAATCAAATACCTTCCCAAGAAGGT
TAAAGATGCAGTCAAAAGATTCAGGACTAACTGCATCAAGAACACAGAGAAAGATATATTTCTCAAGATCAGAAG
TACTATTCCAGTATGGACGATTCAAGGCTTGCTTCACAAACCAAGGCAAGTAATAGAGATTGGAGTCTCTAAAAAG
GTAGTTCCCACTGAATCAAAGGCCATGGAGTCAAAGATTCAAATAGAGGACCTAACAGAACTCGCCGTAAAGACT
GGCGAACAGTTCATACAGAGTCTCTTACGACTCAATGACAAGAAGAAAATCTTCGTCAACATGGTGGAGCACGAC
ACACTTGTCTACTCCAAAAATATCAAAGATACAGTCTCAGAAGACCAAAGGGCAATTGAGACTTTTCAACAAAGGG
TAATATCCGGAAACCTCCTCGGATTCCATTGCCCAGCTATCTGTCACTTTATTGTGAAGATAGTGGAAAAGGAAGG
TGGCTCCTACAAATGCCATCATTGCGATAAAGGAAAGGCCATCGTTGAAGATGCCTCTGCCGACAGTGGTCCCAA
AGATGGACCCCCACCCACGAGGAGCATCGTGGAAAAAGAAGACGTTCCAACCACGTCTTCAAAGCAAGTGGATTG
ATGTGATATCTCCACTGACGTAAGGGATGACGCACAATCCCACTATCCTTCGCAAGACCCTTCCTCTATATAAGGAA
GTTCATTTCATTTGGAGAGAACACGGGGGACTCTAGAGTTATCAACAAGTTTGTACAAAAAAGCAGGCTATGGCG
ACTGGAGTATTGCCAGCTCCGGTTTCTGGGATCAAGATACCGGATTCGAAAGTCGGGTTTGGTAAAAGCATGAA
TCTTGTGAGAATTTGTGATGTTAGGAGTCTAAGATCTGCTAGGAGAAGAGTTTCGGTTATCCGGAATATGAATA
ATCCGTCGTTACTCAATCATGCGGTCGAAACGATGGCAGTTGGCTCGAAAAGTTTTGCGACAGCCTCAAAGTTATT
TGATGCAAAAACCCGGCGCAGCGTACTGATGCTCTACGCCTGGTGCCGCCATTGTGACGATGTTATTGACGATCAG
ACGCTGGGCTTTCAGGCCCGGCAGCCTGCCTTACAAACGCCCGAACAACGTCTGATGCAACTTGAGATGAAAACGC
GCCAGGCCTATGCAGGATCGCAGATGCACGAACCGGCGTTTGCGGCTTTTCAGGAAGTGGCTATGGCTCATGATA
TCGCCCCGGCTTACGCGTTTGATCATCTGGAAGGCTTCGCCATGGATGTACGCGAAGCGCAATACAGCCAACTGGA
TGATACGCTGCGCTATTGCTATCACGTTGCAGGCGTTGTCGGCTTGATGATGGCGCAAATCATGGGCGTGCGGGAT
AACGCCACGCTGGACCGCGCCTGTGACCTTGGGCTGGCATTTCAGTTGACCAATATTGCTCGCGATATTGTGGACG
ATGCGCATGCGGGCCGCTGTTATCTGCCGGCAAGCTGGCTGGAGCATGAAGGTCTGAACAAAGAGAATTATGCGG
CACCTGAAAACCGTCAGGCGCTGAGCCGTATCGCCCGTCGTTTGGTGCAGGAAGCAGAACCTTACTATTTGTCTGC
CACAGCCGGCCTGGCAGGGTTGCCCCTGCGTTCCGCCTGGGCAATCGCTACGGCGAAGCAGGTTTACCGGAAAAT
AGGTGTCAAAGTTGAACAGGCCGGTCAGCAAGCCTGGGATCAGCGGCAGTCAACGACCACGCCCGAAAAATTAAC
GCTGCTGCTGGCCGCCTCTGGTCAGGCCCTTACTTCCCGGATGCGGGCTCATCCTCCCCGCCCTGCGCATCTCTGGC
AGCGCCCGCTCTAGACCCAGCTTTCTTGTACAAAGTGGTTCGATCTAGAGGATCCATGGTGAGCAAGGGCGAGGA
GCTGTTCACCGGGGTGGTGCCCATCCTGGTCGAGCTGGACGGCGACGTGAACGGCCACAAGTTCAGCGTGTCCG
GCGAGGGCGAGGGCGATGCCACCTACGGCAAGCTGACCCTGAAGTTCATCTGCACCACCGGCAAGCTGCCCGTG
CCCTGGCCCACCCTCGTGACCACCTTCACCTACGGCGTGCAGTGCTTCAGCCGCTACCCCGACCACATGAAGCAGC
ACGACTTCTTCAAGTCCGCCATGCCCGAAGGCTACGTCCAGGAGCGCACCATCTTCTTCAAGGACGACGGCAACTA
CAAGACCCGCGCCGAGGTGAAGTTCGAGGGCGACACCCTGGTGAACCGCATCGAGCTGAAGGGCATCGACTTCA
AGGAGGACGGCAACATCCTGGGGCACAAGCTGGAGTACAACTACAACAGCCACAACGTCTATATCATGGCCGAC
AAGCAGAAGAACGGCATCAAGGTGAACTTCAAGATCCGCCACAACATCGAGGACGGCAGCGTGCAGCTCGCCGA
CCACTACCAGCAGAACACCCCCATCGGCGACGGCCCCGTGCTGCTGCCCGACAACCACTACCTGAGCACCCAGTCC
GCCCTGAGCAAAGACCCCAACGAGAAGCGCGATCACATGGTCCTGCTGGAGTTCGTGACCGCCGCCGGGATCACT
CACGGCATGGACGAGCTGTACAAGTAAAGCGGCCCGAGCTCGAATTTCCCCGATCGTTCAAACATTTGGCAATAA
AGTTTCTTAAGATTGAATCCTGTTGCCGGTCTTGCGATGATTATCATATAATTTCTGTTGAATTACGTTAAGCATGTA
ATAATTAACATGTAATGCATGACGTTATTTATGAGATGGGTTTTTATGATTAGAGTCCCGCAATTATACATTTAATA
CGCGATAGAAAACAAAATATAGCGCGCAAACTAGGATAAATTATCGCGCGCGGTGTCATCTATGTTACTAGATCG
GGAATTGGTTCCGGAACCAATTCGTAATCATGGTCATAGCTGTTTCCTGTGTGAAATTGTTATCCGCTCACAATTCC
ACACAACATACGAGCCGGAAGCATAAAGTGTAAAGCCTGGGGTGCCTAATGAGTGAGCTAACTCACATTAGGCTG
AATTAGGCGCGCCTATTTCTATTAATTCCCGATCTAGTAACATAGATGACACCGCGCGCGATAATTTATCCTAGTTT
GCGCGCTATATTTTGTTTTCTATCGCGTATTAAATGTATAATTGCGGGACTCTAATCATAAAAACCCATCTCATAAAT
AACGTCATGCATTACATGTTAATTATTACATGCTTAACGTAATTCAACAGAAATTATATGATAATCATCGCAAGACC
GGCAACAGGATTCAATCTTAAGAAACTTTATTGCCAAATGTTTGAACGATCGGGGAAATTCGAGCTGGGGACGTC
CCCAGCTCGGTACCCCCTCCTGGGCACGGTCGTACGCGATCCGCCGCGGTTCCACCTCACCATTGCGGGTATGCCA
GTTGAGTTCGATGAGTTCCCGTACCAGCTCCAACTCGGGCGATGCCACGCGCACTCCGTTCAGATCTCGGTGACGG

GCAGGACCGGACGGGGCGGTACCGGCAGGCTGAAGTCCAGCTGCCAGAAACCCACGTCATGCCAGTTCCCGTGC
TTGAAGCCGGCCGCCCGCAGCATGCCGCGGGGGGCATATCCGAGCGCCTCGTGCATGCGCACGCTCGGGTCGTT
GGGCAGCCCGATGACAGCGACCACGCTCTTGAAGCCCTGTGCCTCCAGGGACTTCAGCAGGTGGGTGTAGAGCG
TGGAGCCCAGTCCCGTCCGCTGGTGGCGGGGGGAGACGTACACGGTCGACTCGGCCGTCCAGTCGTAGGCGTTG
CGTGCCTTCCAGGGGCCCGCGTAGGCGATGCCGGCGACCTCGCCGTCCACCTCGGCGACGAGCCAGGGATAGCG
CTCCCGCAGACGGACGAGGTCGTCCGTCCACTCCTGCGGTTCCTGCGGCTCGGTACGGAAGTTGACCGTGCTTGTC
TCGATGTAGTGGTTGACGATGGTGCAGACCGCCGGCATGTCCGCCTCGGTGGCACGGCGGATGTCGGCCGGGCG
TCGTTCTGGGCTCATATCTTATTGCCCCCGGGATCAGATCTGGATTGAGAGTGAATATGAGACTCTAATTGGATA
CCGAGGGGAATTTATGGAACGTCAGTGGAGCATTTTTGACAAGAAATATTTGCTAGCTGATAGTGACCTTAGGCG
ACTTTTGAACGCGCAATAATGGTTTCTGACGTATGTGCTTAGCTCATTAAACTCCAGAAACCCGCGGCTGAGTGGC
TCCTTCAACGTTGCGGTTCTGTCAGTTCCAAACGTAAAACGGCTTGTCCCGCGTCATCGGCGGGGGTCATAACGTG
ACTCCCTTAATTCTCCGCTCATGATCTTGATCCCCTGCGCCATCAGATCCTTGGCGGCAAGAAAGCCATCCAGTTTA
CTTTGCAGGGCTTCCCAACCTTACCAGAGGGCGCCCCAGCTGGCAATTCCGGTTCGCTTGCTGTCCATAAAACCGC
CCAGTCTAGCTATCGCCATGTAAGCCCACTGCAAGCTACCTGCTTTCTCTTTGCGCTTGCGTTTTCCCTTGTCCAGAT
AGCCCAGTAGCTGACATTCATCCGGGGTCAGCACCGTTTCTGCGGACTGGCTTTCTACGTGTTCCGCTTCCTTTAGC
AGCCCTTGCGCCCTGAGTGCTTGCGGCAGCGTGAAGCTGATCCGTCGAGATTAATAGAAATAAATTCAGCCTAATT
CGGCGTTAATTCAGTACATTAAAAACGTCCGCAATGTGTTATTAAGTTGTCTAAGCGTCAATTTGTTTACACCACAA
TATATCCTGCCACCAGCCAGCCAACAGCTCCCCGACCGGCAGCTCGGCACAAAATCACCACTCGATACAGGCAGCC
CATCAGTCCGGGACGGCGTCAGCGGGAGAGCCGTTGTAAGGCGGCAGACTTTGCTCATGTTACCGATGCTATTCG
GAAGAACGGCAACTAAGCTGCCGGGTTTGAAACACGGATGATCTCGCGGAGGGTAGCATGTTGATTGTAACGAT
GACAGAGCGTTGCTGCCTGTGATCAATTCGGGCACGAACCCAGTGGACATAAGCCTGTTCGGTTCGTAAGCTGTA
ATGCAAGTAGCGTATGCGCTCACGCAACTGGTCCAGAACCTTGACCGAACGCAGCGGTGGTAACGGCGCAGTGG
CGGTTTTCATGGCTTGTTATGACTGTTTTTTTGGGGTACAGTCTATGCCTCGGGCATCCAAGCAGCAAGCGCGTTAC
GCCGTGGGTCGATGTTTGATGTTATGGAGCAGCAACGATGTTACGCAGCAGGGCAGTCGCCCTAAAACAAAGTTA
AACATCATGGGGGAAGCGGTGATCGCCGAAGTATCGACTCAACTATCAGAGGTAGTTGGCGTCATCGAGCGCCAT
CTCGAACCGACGTTGCTGGCCGTACATTTGTACGGCTCCGCAGTGGATGGCGGCCTGAAGCCACACAGTGATATT
GATTTGCTGGTTACGGTGACCGTAAGGCTTGATGAAACAACGCGGCGAGCTTTGATCAACGACCTTTTGGAAACTT
CGGCTTCCCCTGGAGAGAGCGAGATTCTCCGCGCTGTAGAAGTCACCATTGTTGTGCACGACGACATCATTCCGTG
GCGTTATCCAGCTAAGCGCGAACTGCAATTTGGAGAATGGCAGCGCAATGACATTCTTGCAGGTATCTTCGAGCC
AGCCACGATCGACATTGATCTGGCTATCTTGCTGACAAAAGCAAGAGAACATAGCGTTGCCTTGGTAGGTCCAGC
GGCGGAGGAACTCTTTGATCCGGTTCCTGAACAGGATCTATTTGAGGCGCTAAATGAAACCTTAACGCTATGGAA
CTCGCCGCCCGACTGGGCTGGCGATGAGCGAAATGTAGTGCTTACGTTGTCCCGCATTTGGTACAGCGCAGTAAC
CGGCAAAATCGCGCCGAAGGATGTCGCTGCCGACTGGGCAATGGAGCGCCTGCCGGCCCAGTATCAGCCCGTCAT
ACTTGAAGCTAGACAGGCTTATCTTGGACAAGAAGAAGATCGCTTGGCCTCGCGCGCAGATCAGTTGGAAGAATT
TGTCCACTACGTGAAAGGCGAGATCACCAAGGTAGTCGGCAAATAATGTCTAGCTAGAAATTCGTTCAAGCCGAC
GCCGCTTCGCGGCGCGGCTTAACTCAAGCGTTAGATGCACTAAGCACATAATTGCTCACAGCCAAACTATCAGGTC
AAGTCTGCTTTTATTATTTTTAAGCGTGCATAATAAGCCCTACACAAATTGGGAGATATATCATGCATGACCAAAAT
CCCTTAACGTGAGTTTTCGTTCCACTGAGCGTCAGACCCCGTAGAAAAGATCAAAGGATCTTCTTGAGATCCTTTTT
TTCTGCGCGTAATCTGCTGCTTGCAAACAAAAAAACCACCGCTACCAGCGGTGGTTTGTTTGCCGGATCAAGAGCT
ACCAACTCTTTTTCCGAAGGTAACTGGCTTCAGCAGAGCGCAGATACCAAATACTGTCCTTCTAGTGTAGCCGTAG
TTAGGCCACCACTTCAAGAACTCTGTAGCACCGCCTACATACCTCGCTCTGCTAATCCTGTTACCAGTGGCTGCTGC
CAGTGGCGATAAGTCGTGTCTTACCGGGTTGGACTCAAGACGATAGTTACCGGATAAGGCGCAGCGGTCGGGCT
GAACGGGGGGTTCGTGCACACAGCCCAGCTTGGAGCGAACGACCTACACCGAACTGAGATACCTACAGCGTGAG
CTATGAGAAAGCGCCACGCTTCCCGAAGGGAGAAAGGCGGACAGGTATCCGGTAAGCGGCAGGGTCGGAACAG
GAGAGCGCACGAGGGAGCTTCCAGGGGGAAACGCCTGGTATCTTTATAGTCCTGTCGGGTTTCGCCACCTCTGAC
TTGAGCGTCGATTTTTGTGATGCTCGTCAGGGGGGCGGAGCCTATGGAAAAACGCCAGCAACGCGGCCTTTTTAC
GGTTCCTGGCCTTTTGCTGGCCTTTTGCTCACATGTTCTTTCCTGCGTTATCCCCTGATTCTGTGGATAACCGTATTA
CCGCCTTTGAGTGAGCTGATACCGCTCGCCGCAGCCGAACGACCGAGCGCAGCGAGTCAGTGAGCGAGGAAGCG
GAAGAGCGCCTGATGCGGTATTTTCTCCTTACGCATCTGTGCGGTATTTCACACCGCATATGGTGCACTCTCAGTAC
AATCTGCTCTGATGCCGCATAGTTAAGCCAGTATACACTCCGCTATCGCTACGTGACTGGGTCATGGCTGCGCCCC
GACACCCGCCAACACCCGCTGACGCGCCCTGACGGGCTTGTCTGCTCCCGGCATCCGCTTACAGACAAGCTGTGAC
CGTCTCCGGGAGCTGCATGTGTCAGAGGTTTTCACCGTCATCACCGAAACGCGCGAGGCAGGGTGCCTTGATGTG
GGCGCCGGCGGTCGAGTGGCGACGGCGCGGCTTGTCCGCGCCCTGGTAGATTGCCTGGCCGTAGGCCAGCCATT

TTTGAGCGGCCAGCGGCCGCGATAGGCCGACGCGAAGCGGCGGGCGTAGGGAGCGCAGCGACCGAAGGGTA
GGCGCTTTTTGCAGCTCTTCGGCTGTGCGCTGGCCAGACAGTTATGCACAGGCCAGGCGGGTTTTAAGAGTTTTAA
TAAGTTTTAAAGAGTTTTAGGCGGAAAAATCGCCTTTTTTCTCTTTTATATCAGTCACTTACATGTGTGACCGGTTCC
CAATGTACGGCTTTGGGTTCCCAATGTACGGGTTCCGGTTCCCAATGTACGGCTTTGGGTTCCCAATGTACGTGCT
ATCCACAGGAAAGAGACCTTTTCGACCTTTTTCCCCTGCTAGGGCAATTTGCCCTAGCATCTGCTCCGTACATTAGG
AACCGGCGGATGCTTCGCCCTCGATCAGGTTGCGGTAGCGCATGACTAGGATCGGGCCAGCCTGCCCCGCCTCCT
CCTTCAAATCGTACTCCGGCAGGTCATTTGACCCGATCAGCTTGCGCACGGTGAAACAGAACTTCTTGAACTCTCC
GGCGCTGCCACTGCGTTCGTAGATCGTCTTGAACAACCATCTGGCTTCTGCCTTGCCTGCGGCGCGGCGTGCCAGG
CGGTAGAGAAAACGGCCGATGCCGGGATCGATCAAAAAGTAATCGGGGTGAACCGTCAGCACGTCCGGGTTCTT
GCCTTCTGTGATCTCGCGGTACATCCAATCAGCTAGCTCGATCTCGATGTACTCCGGCCGCCCGGTTTCGCTCTTTA
CGATCTTGTAGCGGCTAATCAAGGCTTCACCCTCGGATACCGTCACCAGGCGGCCGTTCTTGGCCTTCTTCGTACG
CTGCATGGCAACGTGCGTGGTGTTTAACCGAATGCAGGTTTCTACCAGGTCGTCTTTCTGCTTTCCGCCATCGGCTC
GCCGGCAGAACTTGAGTACGTCCGCAACGTGTGGACGGAACACGCGGCCGGGCTTGTCTCCCTTCCCTTCCCGGT
ATCGGTTCATGGATTCGGTTAGATGGGAAACCGCCATCAGTACCAGGTCGTAATCCCACACACTGGCCATGCCGG
CCGGCCCTGCGGAAACCTCTACGTGCCCGTCTGGAAGCTCGTAGCGGATCACCTCGCCAGCTCGTCGGTCACGCTT
CGACAGACGGAAAACGGCCACGTCCATGATGCTGCGACTATCGCGGGTGCCCACGTCATAGAGCATCGGAACGA
AAAAATCTGGTTGCTCGTCGCCCTTGGGCGGCTTCCTAATCGACGGCGCACCGGCTGCCGGCGGTTGCCGGGATT
CTTTGCGGATTCGATCAGCGGCCGCTTGCCACGATTCACCGGGGCGTGCTTCTGCCTCGATGCGTTGCCGCTGGGC
GGCCTGCGCGGCCTTCAACTTCTCCACCAGGTCATCACCCAGCGCCGCGCCGATTTGTACCGGGCCGGATGGTTTG
CGACCGCTCACGCCGATTCCTCGGGCTTGGGGGTTCCAGTGCCATTGCAGGGCCGGCAGACAACCCAGCCGCTTA
CGCCTGGCCAACCGCCCGTTCCTCCACACATGGGGCATTCCACGGCGTCGGTGCCTGGTTGTTCTTGATTTTCCATG
CCGCCTCCTTTAGCCGCTAAAATTCATCTACTCATTTATTCATTTGCTCATTTACTCTGGTAGCTGCGCGATGTATTC
AGATAGCAGCTCGGTAATGGTCTTGCCTTGGCGTACCGCGTACATCTTCAGCTTGGTGTGATCCTCCGCCGGCAAC
TGAAAGTTGACCCGCTTCATGGCTGGCGTGTCTGCCAGGCTGGCCAACGTTGCAGCCTTGCTGCTGCGTGCGCTCG
GACGGCCGGCACTTAGCGTGTTTGTGCTTTTGCTCATTTTCTCTTTACCTCATTAACTCAAATGAGTTTTGATTTAAT
TTCAGCGGCCAGCGCCTGGACCTCGCGGGCAGCGTCGCCCTCGGGTTCTGATTCAAGAACGGTTGTGCCGGCGGC
GGCAGTGCCTGGGTAGCTCACGCGCTGCGTGATACGGGACTCAAGAATGGGCAGCTCGTACCCGGCCAGCGCCT
CGGCAACCTCACCGCCGATGCGCGTGCCTTTGATCGCCCGCGACACGACAAAGGCCGCTTGTAGCCTTCCATCCGT
GACCTCAATGCGCTGCTTAACCAGCTCCACCAGGTCGGCGGTGGCCCATATGTCGTAAGGGCTTGGCTGCACCGG
AATCAGCACGAAGTCGGCTGCCTTGATCGCGGACACAGCCAAGTCCGCCGCCTGGGGCGCTCCGTCGATCACTAC
GAAGTCGCGCCGGCCGATGGCCTTCACGTCGCGGTCAATCGTCGGGCGGTCGATGCCGACAACGGTTAGCGGTT
GATCTTCCCGCACGGCCGCCCAATCGCGGGCACTGCCCTGGGGATCGGAATCGACTAACAGAACATCGGCCCCGG
CGAGTTGCAGGGCGCGGGCTAGATGGGTTGCGATGGTCGTCTTGCCTGACCCGCCTTTCTGGTTAAGTACAGCGA
TAACCTTCATGCGTTCCCCTTGCGTATTTGTTTATTTACTCATCGCATCATATACGCAGCGACCGCATGACGCAAGCT
GTTTTACTCAAATACACATCACCTTTTTAGACGGCGGCGCTCGGTTTCTTCAGCGGCCAAGCTGGCCGGCCAGGCC
GCCAGCTTGGCATCAGACAAACCGGCCAGGATTTCATGCAGCCGCACGGTTGAGACGTGCGCGGGCGGCTCGAA
CACGTACCCGGCCGCGATCATCTCCGCCTCGATCTCTTCGGTAATGAAAAACGGTTCGTCCTGGCCGTCCTGGTGC
GGTTTCATGCTTGTTCCTCTTGGCGTTCATTCTCGGCGGCCGCCAGGGCGTCGGCCTCGGTCAATGCGTCCTCACG
GAAGGCACCGCGCCGCCTGGCCTCGGTGGGCGTCACTTCCTCGCTGCGCTCAAGTGCGCGGTACAGGGTCGAGC
GATGCACGCCAAGCAGTGCAGCCGCCTCTTTCACGGTGCGGCCTTCCTGGTCGATCAGCTCGCGGGCGTGCGCGA
TCTGTGCCGGGGTGAGGGTAGGGCGGGGGCCAAACTTCACGCCTCGGGCCTTGGCGGCCTCGCGCCCGCTCCGG
GTGCGGTCGATGATTAGGGAACGCTCGAACTCGGCAATGCCGGCGAACACGGTCAACACCATGCGGCCGGCCGG
CGTGGTGGTGTCGGCCCACGGCTCTGCCAGGCTACGCAGGCCCGCGCCGGCCTCCTGGATGCGCTCGGCAATGTC
CAGTAGGTCGCGGGTGCTGCGGGCCAGGCGGTCTAGCCTGGTCACTGTCACAACGTCGCCAGGGCGTAGGTGGT
CAAGCATCCTGGCCAGCTCCGGGCGGTCGCGCCTGGTGCCGGTGATCTTCTCGGAAAACAGCTTGGTGCAGCCGG
CCGCGTGCAGTTCGGCCCGTTGGTTGGTCAAGTCCTGGTCGTCGGTGCTGACGCGGGCATAGCCCAGCAGGCCAG
CGGCGGCGCTCTTGTTCATGGCGTAATGTCTCCGGTTCTAGTCGCAAGTATTCTACTTTATGCGACTAAAACACGC
GACAAGAAAACGCCAGGAAAAGGGCAGGGCGGCAGCCTGTCGCGTAACTTAGGACTTGTGCGACATGTCGTTTT
CAGAAGACGGCTGCACTGAACGTCAGAAGCCGACTGCACTATAGCAGCGGAGGGGTTGGATCAAAGTACTTTGA
TCCCGAGGGGAACCCTGTGGTTGGCATGCACATACAAATGGACGAACGGATAAAC

>(p)crtB_stop_pGWB405
CTTTTCACGCCCTTTTAAATATCCGATTATTCTAATAAACGCTCTTTTCTCTTAGGTTTACCCGCCAATATATCCTGTC
AAACACTGATAGTTTAAACTGAAGGCGGGAAACGACAATCTGATCCAAGCTCAAGCTGCTCTAGCATTCGCCATTC
AGGCTGCGCAACTGTTGGGAAGGGCGATCGGTGCGGGCCTCTTCGCTATTACGCCAGCTGGCGAAAGGGGGATG
TGCTGCAAGGCGATTAAGTTGGGTAACGCCAGGGTTTTCCCAGTCACGACGTTGTAAAACGACGGCCAGTGCCAA
GCTTGCATGCCTGCAGGTCCCCAGATTAGCCTTTTCAATTTCAGAAAGAATGCTAACCCACAGATGGTTAGAGAGG
CTTACGCAGCAGGTCTCATCAAGACGATCTACCCGAGCAATAATCTCCAGGAAATCAAATACCTTCCCAAGAAGGT
TAAAGATGCAGTCAAAAGATTCAGGACTAACTGCATCAAGAACACAGAGAAAGATATATTTCTCAAGATCAGAAG
TACTATTCCAGTATGGACGATTCAAGGCTTGCTTCACAAACCAAGGCAAGTAATAGAGATTGGAGTCTCTAAAAAG
GTAGTTCCCACTGAATCAAAGGCCATGGAGTCAAAGATTCAAATAGAGGACCTAACAGAACTCGCCGTAAAGACT
GGCGAACAGTTCATACAGAGTCTCTTACGACTCAATGACAAGAAGAAAATCTTCGTCAACATGGTGGAGCACGAC
ACACTTGTCTACTCCAAAAATATCAAAGATACAGTCTCAGAAGACCAAAGGGCAATTGAGACTTTTCAACAAAGGG
TAATATCCGGAAACCTCCTCGGATTCCATTGCCCAGCTATCTGTCACTTTATTGTGAAGATAGTGGAAAAGGAAGG
TGGCTCCTACAAATGCCATCATTGCGATAAAGGAAAGGCCATCGTTGAAGATGCCTCTGCCGACAGTGGTCCCAA
AGATGGACCCCCACCCACGAGGAGCATCGTGGAAAAAGAAGACGTTCCAACCACGTCTTCAAAGCAAGTGGATTG
ATGTGATATCTCCACTGACGTAAGGGATGACGCACAATCCCACTATCCTTCGCAAGACCCTTCCTCTATATAAGGAA
GTTCATTTCATTTGGAGAGAACACGGGGGACTCTAGAGTTATCAACAAGTTTGTACAAAAAAGCAGGCTATGGCG
ACTGGAGTATTGCCAGCTCCGGTTTCTGGGATCAAGATACCGGATTCGAAAGTCGGGTTTGGTAAAAGCATGAA
TCTTGTGAGAATTTGTGATGTTAGGAGTCTAAGATCTGCTAGGAGAAGAGTTTCGGTTATCCGGAATATGAATA
ATCCGTCGTTACTCAATCATGCGGTCGAAACGATGGCAGTTGGCTCGAAAAGTTTTGCGACAGCCTCAAAGTTATT
TGATGCAAAAACCCGGCGCAGCGTACTGATGCTCTACGCCTGGTGCCGCCATTGTGACGATGTTATTGACGATCAG
ACGCTGGGCTTTCAGGCCCGGCAGCCTGCCTTACAAACGCCCGAACAACGTCTGATGCAACTTGAGATGAAAACGC
GCCAGGCCTATGCAGGATCGCAGATGCACGAACCGGCGTTTGCGGCTTTTCAGGAAGTGGCTATGGCTCATGATA
TCGCCCCGGCTTACGCGTTTGATCATCTGGAAGGCTTCGCCATGGATGTACGCGAAGCGCAATACAGCCAACTGGA
TGATACGCTGCGCTATTGCTATCACGTTGCAGGCGTTGTCGGCTTGATGATGGCGCAAATCATGGGCGTGCGGGAT
AACGCCACGCTGGACCGCGCCTGTGACCTTGGGCTGGCATTTCAGTTGACCAATATTGCTCGCGATATTGTGGACG
ATGCGCATGCGGGCCGCTGTTATCTGCCGGCAAGCTGGCTGGAGCATGAAGGTCTGAACAAAGAGAATTATGCGG
CACCTGAAAACCGTCAGGCGCTGAGCCGTATCGCCCGTCGTTTGGTGCAGGAAGCAGAACCTTACTATTTGTCTGC
CACAGCCGGCCTGGCAGGGTTGCCCCTGCGTTCCGCCTGGGCAATCGCTACGGCGAAGCAGGTTTACCGGAAAAT
AGGTGTCAAAGTTGAACAGGCCGGTCAGCAAGCCTGGGATCAGCGGCAGTCAACGACCACGCCCGAAAAATTAAC
GCTGCTGCTGGCCGCCTCTGGTCAGGCCCTTACTTCCCGGATGCGGGCTCATCCTCCCCGCCCTGCGCATCTCTGGC
AGCGCCCGCTCTAGACCCAGCTTTCTTGTACAAAGTGGTTCGATCTAGAGGATCCATGGTGAGCAAGGGCGAGGA
GCTGTTCACCGGGGTGGTGCCCATCCTGGTCGAGCTGGACGGCGACGTGAACGGCCACAAGTTCAGCGTGTCCG
GCGAGGGCGAGGGCGATGCCACCTACGGCAAGCTGACCCTGAAGTTCATCTGCACCACCGGCAAGCTGCCCGTG
CCCTGGCCCACCCTCGTGACCACCTTCACCTACGGCGTGCAGTGCTTCAGCCGCTACCCCGACCACATGAAGCAGC
ACGACTTCTTCAAGTCCGCCATGCCCGAAGGCTACGTCCAGGAGCGCACCATCTTCTTCAAGGACGACGGCAACTA
CAAGACCCGCGCCGAGGTGAAGTTCGAGGGCGACACCCTGGTGAACCGCATCGAGCTGAAGGGCATCGACTTCA
AGGAGGACGGCAACATCCTGGGGCACAAGCTGGAGTACAACTACAACAGCCACAACGTCTATATCATGGCCGAC
AAGCAGAAGAACGGCATCAAGGTGAACTTCAAGATCCGCCACAACATCGAGGACGGCAGCGTGCAGCTCGCCGA
CCACTACCAGCAGAACACCCCCATCGGCGACGGCCCCGTGCTGCTGCCCGACAACCACTACCTGAGCACCCAGTCC
GCCCTGAGCAAAGACCCCAACGAGAAGCGCGATCACATGGTCCTGCTGGAGTTCGTGACCGCCGCCGGGATCACT
CACGGCATGGACGAGCTGTACAAGTAAAGCGGCCCGAGCTCGAATTTCCCCGATCGTTCAAACATTTGGCAATAA
AGTTTCTTAAGATTGAATCCTGTTGCCGGTCTTGCGATGATTATCATATAATTTCTGTTGAATTACGTTAAGCATGTA
ATAATTAACATGTAATGCATGACGTTATTTATGAGATGGGTTTTTATGATTAGAGTCCCGCAATTATACATTTAATA
CGCGATAGAAAACAAAATATAGCGCGCAAACTAGGATAAATTATCGCGCGCGGTGTCATCTATGTTACTAGATCG
GGAATTGGTTCCGGAACCAATTCGTAATCATGGTCATAGCTGTTTCCTGTGTGAAATTGTTATCCGCTCACAATTCC
ACACAACATACGAGCCGGAAGCATAAAGTGTAAAGCCTGGGGTGCCTAATGAGTGAGCTAACTCACATTAGGCTG
AATTAGGCGCGCCTATTTCTGAATTCCCGATCTAGTAACATAGATGACACCGCGCGCGATAATTTATCCTAGTTTGC
GCGCTATATTTTGTTTTCTATCGCGTATTAAATGTATAATTGCGGGACTCTAATCATAAAAACCCATCTCATAAATAA
CGTCATGCATTACATGTTAATTATTACATGCTTAACGTAATTCAACAGAAATTATATGATAATCATCGCAAGACCGG
CAACAGGATTCAATCTTAAGAAACTTTATTGCCAAATGTTTGAACGATCGGGGATCATCCGGGTCTGTGGCGGGA
ACTCCACGAAAATATCCGAACGCAGCAAGATATCGCGGTGCATCTCGGTCTTGCCTGGGCAGTCGCCGCCGACGC
CGTTGATGTGGACGCCGGGCCCGATCATATTGTCGCTCAGGATCGTGGCGTTGTGCTTGTCGGCCGTTGCTGTCGT

AATGATATCGGCACCTTCGACCGCCTGTTCCGCAGAGATCCCGTGGGCGAAGAACTCCAGCATGAGATCCCCGCG
CTGGAGGATCATCCAGCCGGCGTCCCGGAAAACGATTCCGAAGCCCAACCTTTCATAGAAGGCGGCGGTGGAATC
GAAATCTCGTGATGGCAGGTTGGGCGTCGCTTGGTCGGTCATTTCGAACCCCAGAGTCCCGCTCAGAAGAACTCG
TCAAGAAGGCGATAGAAGGCGATGCGCTGCGAATCGGGAGCGGCGATACCGTAAAGCACGAGGAAGCGGTCAG
CCCATTCGCCGCCAAGCTCTTCAGCAATATCACGGGTAGCCAACGCTATGTCCTGATAGCGGTCCGCCACACCCAG
CCGGCCACAGTCGATGAATCCAGAAAAGCGGCCATTTTCCACCATGATATTCGGCAAGCAGGCATCGCCATGGGT
CACGACGAGATCATCGCCGTCGGGCATGCGCGCCTTGAGCCTGGCGAACAGTTCGGCTGGCGCGAGCCCCTGAT
GCTCTTCGTCCAGATCATCCTGATCGACAAGACCGGCTTCCATCCGAGTACGTGCTCGCTCGATGCGATGTTTCGCT
TGGTGGTCGAATGGGCAGGTAGCCGGATCAAGCGTATGCAGCCGCCGCATTGCATCAGCCATGATGGATACTTTC
TCGGCAGGAGCAAGGTGAGATGACAGGAGATCCTGCCCCGGCACTTCGCCCAATAGCAGCCAGTCCCTTCCCGCT
TCAGTGACAACGTCGAGCACAGCTGCGCAAGGAACGCCCGTCGTGGCCAGCCACGATAGCCGCGCTGCCTCGTCC
TGCAGTTCATTCAGGGCACCGGACAGGTCGGTCTTGACAAAAAGAACCGGGCGCCCCTGCGCTGACAGCCGGAA
CACGGCGGCATCAGAGCAGCCGATTGTCTGTTGTGCCCAGTCATAGCCGAATAGCCTCTCCACCCAAGCGGCCGG
AGAACCTGCGTGCAATCCATCTTGTTCAATCATGCGAAACGATCCAGATCCGGTGCAGATTATTTGGATTGAGAGT
GAATATGAGACTCTAATTGGATACCGAGGGGAATTTATGGAACGTCAGTGGAGCATTTTTGACAAGAAATATTTG
CTAGCTGATAGTGACCTTAGGCGACTTTTGAACGCGCAATAATGGTTTCTGACGTATGTGCTTAGCTCATTAAACTC
CAGAAACCCGCGGCTGAGTGGCTCCTTCAACGTTGCGGTTCTGTCAGTTCCAAACGTAAAACGGCTTGTCCCGCGT
CATCGGCGGGGGTCATAACGTGACTCCCTTAATTCTCCGCTCATGATGAATTCAGAAATAAATTCAGCCTAATTCG
GCGTTAATTCAGTACATTAAAAACGTCCGCAATGTGTTATTAAGTTGTCTAAGCGTCAATTTGTTTACACCACAATA
TATCCTGCCACCAGCCAGCCAACAGCTCCCCGACCGGCAGCTCGGCACAAAATCACCACTCGATACAGGCAGCCCA
TCAGTCCGGGACGGCGTCAGCGGGAGAGCCGTTGTAAGGCGGCAGACTTTGCTCATGTTACCGATGCTATTCGGA
AGAACGGCAACTAAGCTGCCGGGTTTGAAACACGGATGATCTCGCGGAGGGTAGCATGTTGATTGTAACGATGA
CAGAGCGTTGCTGCCTGTGATCAATTCGGGCACGAACCCAGTGGACATAAGCCTGTTCGGTTCGTAAGCTGTAAT
GCAAGTAGCGTATGCGCTCACGCAACTGGTCCAGAACCTTGACCGAACGCAGCGGTGGTAACGGCGCAGTGGCG
GTTTTCATGGCTTGTTATGACTGTTTTTTTGGGGTACAGTCTATGCCTCGGGCATCCAAGCAGCAAGCGCGTTACGC
CGTGGGTCGATGTTTGATGTTATGGAGCAGCAACGATGTTACGCAGCAGGGCAGTCGCCCTAAAACAAAGTTAAA
CATCATGGGGGAAGCGGTGATCGCCGAAGTATCGACTCAACTATCAGAGGTAGTTGGCGTCATCGAGCGCCATCT
CGAACCGACGTTGCTGGCCGTACATTTGTACGGCTCCGCAGTGGATGGCGGCCTGAAGCCACACAGTGATATTGA
TTTGCTGGTTACGGTGACCGTAAGGCTTGATGAAACAACGCGGCGAGCTTTGATCAACGACCTTTTGGAAACTTCG
GCTTCCCCTGGAGAGAGCGAGATTCTCCGCGCTGTAGAAGTCACCATTGTTGTGCACGACGACATCATTCCGTGGC
GTTATCCAGCTAAGCGCGAACTGCAATTTGGAGAATGGCAGCGCAATGACATTCTTGCAGGTATCTTCGAGCCAG
CCACGATCGACATTGATCTGGCTATCTTGCTGACAAAAGCAAGAGAACATAGCGTTGCCTTGGTAGGTCCAGCGG
CGGAGGAACTCTTTGATCCGGTTCCTGAACAGGATCTATTTGAGGCGCTAAATGAAACCTTAACGCTATGGAACTC
GCCGCCCGACTGGGCTGGCGATGAGCGAAATGTAGTGCTTACGTTGTCCCGCATTTGGTACAGCGCAGTAACCGG
CAAAATCGCGCCGAAGGATGTCGCTGCCGACTGGGCAATGGAGCGCCTGCCGGCCCAGTATCAGCCCGTCATACT
TGAAGCTAGACAGGCTTATCTTGGACAAGAAGAAGATCGCTTGGCCTCGCGCGCAGATCAGTTGGAAGAATTTGT
CCACTACGTGAAAGGCGAGATCACCAAGGTAGTCGGCAAATAATGTCTAGCTAGAAATTCGTTCAAGCCGACGCC
GCTTCGCGGCGCGGCTTAACTCAAGCGTTAGATGCACTAAGCACATAATTGCTCACAGCCAAACTATCAGGTCAAG
TCTGCTTTTATTATTTTTAAGCGTGCATAATAAGCCCTACACAAATTGGGAGATATATCATGCATGACCAAAATCCC
TTAACGTGAGTTTTCGTTCCACTGAGCGTCAGACCCCGTAGAAAAGATCAAAGGATCTTCTTGAGATCCTTTTTTTC
TGCGCGTAATCTGCTGCTTGCAAACAAAAAAACCACCGCTACCAGCGGTGGTTTGTTTGCCGGATCAAGAGCTACC
AACTCTTTTTCCGAAGGTAACTGGCTTCAGCAGAGCGCAGATACCAAATACTGTCCTTCTAGTGTAGCCGTAGTTA
GGCCACCACTTCAAGAACTCTGTAGCACCGCCTACATACCTCGCTCTGCTAATCCTGTTACCAGTGGCTGCTGCCAG
TGGCGATAAGTCGTGTCTTACCGGGTTGGACTCAAGACGATAGTTACCGGATAAGGCGCAGCGGTCGGGCTGAA
CGGGGGGTTCGTGCACACAGCCCAGCTTGGAGCGAACGACCTACACCGAACTGAGATACCTACAGCGTGAGCTAT
GAGAAAGCGCCACGCTTCCCGAAGGGAGAAAGGCGGACAGGTATCCGGTAAGCGGCAGGGTCGGAACAGGAGA
GCGCACGAGGGAGCTTCCAGGGGGAAACGCCTGGTATCTTTATAGTCCTGTCGGGTTTCGCCACCTCTGACTTGA
GCGTCGATTTTTGTGATGCTCGTCAGGGGGGCGGAGCCTATGGAAAAACGCCAGCAACGCGGCCTTTTTACGGTT
CCTGGCCTTTTGCTGGCCTTTTGCTCACATGTTCTTTCCTGCGTTATCCCCTGATTCTGTGGATAACCGTATTACCGC
CTTTGAGTGAGCTGATACCGCTCGCCGCAGCCGAACGACCGAGCGCAGCGAGTCAGTGAGCGAGGAAGCGGAAG
AGCGCCTGATGCGGTATTTTCTCCTTACGCATCTGTGCGGTATTTCACACCGCATATGGTGCACTCTCAGTACAATC
TGCTCTGATGCCGCATAGTTAAGCCAGTATACACTCCGCTATCGCTACGTGACTGGGTCATGGCTGCGCCCCGACA
CCCGCCAACACCCGCTGACGCGCCCTGACGGGCTTGTCTGCTCCCGGCATCCGCTTACAGACAAGCTGTGACCGTC

TCCGGGAGCTGCATGTGTCAGAGGTTTTCACCGTCATCACCGAAACGCGCGAGGCAGGGTGCCTTGATGTGGGCG
CCGGCGGTCGAGTGGCGACGGCGCGGCTTGTCCGCGCCCTGGTAGATTGCCTGGCCGTAGGCCAGCCATTTTTGA
GCGGCCAGCGGCCGCGATAGGCCGACGCGAAGCGGCGGGGCGTAGGGAGCGCAGCGACCGAAGGGTAGGCGC
TTTTTGCAGCTCTTCGGCTGTGCGCTGGCCAGACAGTTATGCACAGGCCAGGCGGGTTTTAAGAGTTTTAATAAGT
TTTAAAGAGTTTTAGGCGGAAAAATCGCCTTTTTTCTCTTTTATATCAGTCACTTACATGTGTGACCGGTTCCCAATG
TACGGCTTTGGGTTCCCAATGTACGGGTTCCGGTTCCCAATGTACGGCTTTGGGTTCCCAATGTACGTGCTATCCAC
AGGAAAGAGACCTTTTCGACCTTTTTCCCCTGCTAGGGCAATTTGCCCTAGCATCTGCTCCGTACATTAGGAACCG
GCGGATGCTTCGCCCTCGATCAGGTTGCGGTAGCGCATGACTAGGATCGGGCCAGCCTGCCCCGCCTCCTCCTTCA
AATCGTACTCCGGCAGGTCATTTGACCCGATCAGCTTGCGCACGGTGAAACAGAACTTCTTGAACTCTCCGGCGCT
GCCACTGCGTTCGTAGATCGTCTTGAACAACCATCTGGCTTCTGCCTTGCCTGCGGCGCGGCGTGCCAGGCGGTAG
AGAAAACGGCCGATGCCGGGATCGATCAAAAAGTAATCGGGGTGAACCGTCAGCACGTCCGGGTTCTTGCCTTCT
GTGATCTCGCGGTACATCCAATCAGCTAGCTCGATCTCGATGTACTCCGGCCGCCCGGTTTCGCTCTTTACGATCTT
GTAGCGGCTAATCAAGGCTTCACCCTCGGATACCGTCACCAGGCGGCCGTTCTTGGCCTTCTTCGTACGCTGCATG
GCAACGTGCGTGGTGTTTAACCGAATGCAGGTTTCTACCAGGTCGTCTTTCTGCTTTCCGCCATCGGCTCGCCGGC
AGAACTTGAGTACGTCCGCAACGTGTGGACGGAACACGCGGCCGGGCTTGTCTCCCTTCCCTTCCCGGTATCGGTT
CATGGATTCGGTTAGATGGGAAACCGCCATCAGTACCAGGTCGTAATCCCACACACTGGCCATGCCGGCCGGCCC
TGCGGAAACCTCTACGTGCCCGTCTGGAAGCTCGTAGCGGATCACCTCGCCAGCTCGTCGGTCACGCTTCGACAG
ACGGAAAACGGCCACGTCCATGATGCTGCGACTATCGCGGGTGCCCACGTCATAGAGCATCGGAACGAAAAAATC
TGGTTGCTCGTCGCCCTTGGGCGGCTTCCTAATCGACGGCGCACCGGCTGCCGGCGGTTGCCGGGATTCTTTGCG
GATTCGATCAGCGGCCGCTTGCCACGATTCACCGGGGCGTGCTTCTGCCTCGATGCGTTGCCGCTGGGCGGCCTG
CGCGGCCTTCAACTTCTCCACCAGGTCATCACCCAGCGCCGCGCCGATTTGTACCGGGCCGGATGGTTTGCGACCG
CTCACGCCGATTCCTCGGGCTTGGGGGTTCCAGTGCCATTGCAGGGCCGGCAGACAACCCAGCCGCTTACGCCTG
GCCAACCGCCCGTTCCTCCACACATGGGGCATTCCACGGCGTCGGTGCCTGGTTGTTCTTGATTTTCCATGCCGCCT
CCTTTAGCCGCTAAAATTCATCTACTCATTTATTCATTTGCTCATTTACTCTGGTAGCTGCGCGATGTATTCAGATAG
CAGCTCGGTAATGGTCTTGCCTTGGCGTACCGCGTACATCTTCAGCTTGGTGTGATCCTCCGCCGGCAACTGAAAG
TTGACCCGCTTCATGGCTGGCGTGTCTGCCAGGCTGGCCAACGTTGCAGCCTTGCTGCTGCGTGCGCTCGGACGG
CCGGCACTTAGCGTGTTTGTGCTTTTGCTCATTTTCTCTTTACCTCATTAACTCAAATGAGTTTTGATTTAATTTCAGC
GGCCAGCGCCTGGACCTCGCGGGCAGCGTCGCCCTCGGGTTCTGATTCAAGAACGGTTGTGCCGGCGGCGGCAG
TGCCTGGGTAGCTCACGCGCTGCGTGATACGGGACTCAAGAATGGGCAGCTCGTACCCGGCCAGCGCCTCGGCAA
CCTCACCGCCGATGCGCGTGCCTTTGATCGCCCGCGACACGACAAAGGCCGCTTGTAGCCTTCCATCCGTGACCTC
AATGCGCTGCTTAACCAGCTCCACCAGGTCGGCGGTGGCCCATATGTCGTAAGGGCTTGGCTGCACCGGAATCAG
CACGAAGTCGGCTGCCTTGATCGCGGACACAGCCAAGTCCGCCGCCTGGGGCGCTCCGTCGATCACTACGAAGTC
GCGCCGGCCGATGGCCTTCACGTCGCGGTCAATCGTCGGGCGGTCGATGCCGACAACGGTTAGCGGTTGATCTTC
CCGCACGGCCGCCCAATCGCGGGCACTGCCCTGGGGATCGGAATCGACTAACAGAACATCGGCCCCGGCGAGTT
GCAGGGCGCGGGCTAGATGGGTTGCGATGGTCGTCTTGCCTGACCCGCCTTTCTGGTTAAGTACAGCGATAACCT
TCATGCGTTCCCCTTGCGTATTTGTTTATTTACTCATCGCATCATATACGCAGCGACCGCATGACGCAAGCTGTTTTA
CTCAAATACACATCACCTTTTTAGACGGCGGCGCTCGGTTTCTTCAGCGGCCAAGCTGGCCGGCCAGGCCGCCAGC
TTGGCATCAGACAAACCGGCCAGGATTTCATGCAGCCGCACGGTTGAGACGTGCGCGGGCGGCTCGAACACGTA
CCCGGCCGCGATCATCTCCGCCTCGATCTCTTCGGTAATGAAAAACGGTTCGTCCTGGCCGTCCTGGTGCGGTTTC
ATGCTTGTTCCTCTTGGCGTTCATTCTCGGCGGCCGCCAGGGCGTCGGCCTCGGTCAATGCGTCCTCACGGAAGGC
ACCGCGCCGCCTGGCCTCGGTGGGCGTCACTTCCTCGCTGCGCTCAAGTGCGCGGTACAGGGTCGAGCGATGCAC
GCCAAGCAGTGCAGCCGCCTCTTTCACGGTGCGGCCTTCCTGGTCGATCAGCTCGCGGGCGTGCGCGATCTGTGC
CGGGGTGAGGGTAGGGCGGGGGCCAAACTTCACGCCTCGGGCCTTGGCGGCCTCGCGCCCGCTCCGGGTGCGGT
CGATGATTAGGGAACGCTCGAACTCGGCAATGCCGGCGAACACGGTCAACACCATGCGGCCGGCCGGCGTGGTG
GTGTCGGCCCACGGCTCTGCCAGGCTACGCAGGCCCGCGCCGGCCTCCTGGATGCGCTCGGCAATGTCCAGTAGG
TCGCGGGTGCTGCGGGCCAGGCGGTCTAGCCTGGTCACTGTCACAACGTCGCCAGGGCGTAGGTGGTCAAGCAT
CCTGGCCAGCTCCGGGCGGTCGCGCCTGGTGCCGGTGATCTTCTCGGAAAACAGCTTGGTGCAGCCGGCCGCGTG
CAGTTCGGCCCGTTGGTTGGTCAAGTCCTGGTCGTCGGTGCTGACGCGGGCATAGCCCAGCAGGCCAGCGGCGG
CGCTCTTGTTCATGGCGTAATGTCTCCGGTTCTAGTCGCAAGTATTCTACTTTATGCGACTAAAACACGCGACAAGA
AAACGCCAGGAAAAGGGCAGGGCGGCAGCCTGTCGCGTAACTTAGGACTTGTGCGACATGTCGTTTTCAGAAGA
CGGCTGCACTGAACGTCAGAAGCCGACTGCACTATAGCAGCGGAGGGGTTGGATCAAAGTACTTTGATCCCGAG
GGGAACCCTGTGGTTGGCATGCACATACAAATGGACGAACGGATAAAC

In the context of the present invention, crtbB is understood as 35S:crtB-pGWB605 (SEQ ID NO 4) or as 35S:crtB-pGWB405 (SEQ ID NO 3) two constructs driving *Pantoea ananatis* phytoene synthase expression without a functionally linked plastid-targeting signal, but where internal crtB protein localization signals are still able to translocate sufficient phytoene synthase activity to plastids and further identified as SEQ No ID 3 and 4. 
>crtB_stop_pGWB405
CTTTTCACGCCCTTTTAAATATCCGATTATTCTAATAAACGCTCTTTTCTCTTAGGTTTACCCGCCAATATATCCTGTC
AAACACTGATAGTTTAAACTGAAGGCGGGAAACGACAATCTGATCCAAGCTCAAGCTGCTCTAGCATTCGCCATTC
AGGCTGCGCAACTGTTGGGAAGGGCGATCGGTGCGGGCCTCTTCGCTATTACGCCAGCTGGCGAAAGGGGGATG
TGCTGCAAGGCGATTAAGTTGGGTAACGCCAGGGTTTTCCCAGTCACGACGTTGTAAAACGACGGCCAGTGCCAA
GCTTGCATGCCTGCAGGTCCCCAGATTAGCCTTTTCAATTTCAGAAAGAATGCTAACCCACAGATGGTTAGAGAGG
CTTACGCAGCAGGTCTCATCAAGACGATCTACCCGAGCAATAATCTCCAGGAAATCAAATACCTTCCCAAGAAGGT
TAAAGATGCAGTCAAAAGATTCAGGACTAACTGCATCAAGAACACAGAGAAAGATATATTTCTCAAGATCAGAAG
TACTATTCCAGTATGGACGATTCAAGGCTTGCTTCACAAACCAAGGCAAGTAATAGAGATTGGAGTCTCTAAAAAG
GTAGTTCCCACTGAATCAAAGGCCATGGAGTCAAAGATTCAAATAGAGGACCTAACAGAACTCGCCGTAAAGACT
GGCGAACAGTTCATACAGAGTCTCTTACGACTCAATGACAAGAAGAAAATCTTCGTCAACATGGTGGAGCACGAC
ACACTTGTCTACTCCAAAAATATCAAAGATACAGTCTCAGAAGACCAAAGGGCAATTGAGACTTTTCAACAAAGGG
TAATATCCGGAAACCTCCTCGGATTCCATTGCCCAGCTATCTGTCACTTTATTGTGAAGATAGTGGAAAAGGAAGG
TGGCTCCTACAAATGCCATCATTGCGATAAAGGAAAGGCCATCGTTGAAGATGCCTCTGCCGACAGTGGTCCCAA
AGATGGACCCCCACCCACGAGGAGCATCGTGGAAAAAGAAGACGTTCCAACCACGTCTTCAAAGCAAGTGGATTG
ATGTGATATCTCCACTGACGTAAGGGATGACGCACAATCCCACTATCCTTCGCAAGACCCTTCCTCTATATAAGGAA
GTTCATTTCATTTGGAGAGAACACGGGGGACTCTAGAGTTATCAACAAGTTTGTACAAAAAAGCAGGCTTCATGA
ATAATCCGTCGTTACTCAATCATGCGGTCGAAACGATGGCAGTTGGCTCGAAAAGTTTTGCGACAGCCTCAAAGTT
ATTTGATGCAAAAACCCGGCGCAGCGTACTGATGCTCTACGCCTGGTGCCGCCATTGTGACGATGTTATTGACGAT
CAGACGCTGGGCTTTCAGGCCCGGCAGCCTGCCTTACAAACGCCCGAACAACGTCTGATGCAACTTGAGATGAAAA
CGCGCCAGGCCTATGCAGGATCGCAGATGCACGAACCGGCGTTTGCGGCTTTTCAGGAAGTGGCTATGGCTCATG
ATATCGCCCCGGCTTACGCGTTTGATCATCTGGAAGGCTTCGCCATGGATGTACGCGAAGCGCAATACAGCCAACT
GGATGATACGCTGCGCTATTGCTATCACGTTGCAGGCGTTGTCGGCTTGATGATGGCGCAAATCATGGGCGTGCG
GGATAACGCCACGCTGGACCGCGCCTGTGACCTTGGGCTGGCATTTCAGTTGACCAATATTGCTCGCGATATTGTG
GACGATGCGCATGCGGGCCGCTGTTATCTGCCGGCAAGCTGGCTGGAGCATGAAGGTCTGAACAAAGAGAATTAT
GCGGCACCTGAAAACCGTCAGGCGCTGAGCCGTATCGCCCGTCGTTTGGTGCAGGAAGCAGAACCTTACTATTTGT
CTGCCACAGCCGGCCTGGCAGGGTTGCCCCTGCGTTCCGCCTGGGCAATCGCTACGGCGAAGCAGGTTTACCGGA
AAATAGGTGTCAAAGTTGAACAGGCCGGTCAGCAAGCCTGGGATCAGCGGCAGTCAACGACCACGCCCGAAAAAT
TAACGCTGCTGCTGGCCGCCTCTGGTCAGGCCCTTACTTCCCGGATGCGGGCTCATCCTCCCCGCCCTGCGCATCTC
TGGCAGCGCCCGCTCTAGACCCAGCTTTCTTGTACAAAGTGGTTCGATCTAGAGGATCCATGGTGAGCAAGGGCG
AGGAGCTGTTCACCGGGGTGGTGCCCATCCTGGTCGAGCTGGACGGCGACGTGAACGGCCACAAGTTCAGCGTG
TCCGGCGAGGGCGAGGGCGATGCCACCTACGGCAAGCTGACCCTGAAGTTCATCTGCACCACCGGCAAGCTGCCC
GTGCCCTGGCCCACCCTCGTGACCACCTTCACCTACGGCGTGCAGTGCTTCAGCCGCTACCCCGACCACATGAAGC
AGCACGACTTCTTCAAGTCCGCCATGCCCGAAGGCTACGTCCAGGAGCGCACCATCTTCTTCAAGGACGACGGCA
ACTACAAGACCCGCGCCGAGGTGAAGTTCGAGGGCGACACCCTGGTGAACCGCATCGAGCTGAAGGGCATCGAC
TTCAAGGAGGACGGCAACATCCTGGGGCACAAGCTGGAGTACAACTACAACAGCCACAACGTCTATATCATGGCC
GACAAGCAGAAGAACGGCATCAAGGTGAACTTCAAGATCCGCCACAACATCGAGGACGGCAGCGTGCAGCTCGC
CGACCACTACCAGCAGAACACCCCCATCGGCGACGGCCCCGTGCTGCTGCCCGACAACCACTACCTGAGCACCCA
GTCCGCCCTGAGCAAAGACCCCAACGAGAAGCGCGATCACATGGTCCTGCTGGAGTTCGTGACCGCCGCCGGGAT
CACTCACGGCATGGACGAGCTGTACAAGTAAAGCGGCCCGAGCTCGAATTTCCCCGATCGTTCAAACATTTGGCA
ATAAAGTTTCTTAAGATTGAATCCTGTTGCCGGTCTTGCGATGATTATCATATAATTTCTGTTGAATTACGTTAAGC
ATGTAATAATTAACATGTAATGCATGACGTTATTTATGAGATGGGTTTTTATGATTAGAGTCCCGCAATTATACATT
TAATACGCGATAGAAAACAAAATATAGCGCGCAAACTAGGATAAATTATCGCGCGCGGTGTCATCTATGTTACTA
GATCGGGAATTGGTTCCGGAACCAATTCGTAATCATGGTCATAGCTGTTTCCTGTGTGAAATTGTTATCCGCTCACA
ATTCCACACAACATACGAGCCGGAAGCATAAAGTGTAAAGCCTGGGGTGCCTAATGAGTGAGCTAACTCACATTA
 
GGCTGAATTAGGCGCGCCTATTTCTGAATTCCCGATCTAGTAACATAGATGACACCGCGCGCGATAATTTATCCTA
GTTTGCGCGCTATATTTTGTTTTCTATCGCGTATTAAATGTATAATTGCGGGACTCTAATCATAAAAACCCATCTCAT
AAATAACGTCATGCATTACATGTTAATTATTACATGCTTAACGTAATTCAACAGAAATTATATGATAATCATCGCAA
GACCGGCAACAGGATTCAATCTTAAGAAACTTTATTGCCAAATGTTTGAACGATCGGGGATCATCCGGGTCTGTGG
CGGGAACTCCACGAAAATATCCGAACGCAGCAAGATATCGCGGTGCATCTCGGTCTTGCCTGGGCAGTCGCCGCC
GACGCCGTTGATGTGGACGCCGGGCCCGATCATATTGTCGCTCAGGATCGTGGCGTTGTGCTTGTCGGCCGTTGC
TGTCGTAATGATATCGGCACCTTCGACCGCCTGTTCCGCAGAGATCCCGTGGGCGAAGAACTCCAGCATGAGATCC
CCGCGCTGGAGGATCATCCAGCCGGCGTCCCGGAAAACGATTCCGAAGCCCAACCTTTCATAGAAGGCGGCGGTG
GAATCGAAATCTCGTGATGGCAGGTTGGGCGTCGCTTGGTCGGTCATTTCGAACCCCAGAGTCCCGCTCAGAAGA
ACTCGTCAAGAAGGCGATAGAAGGCGATGCGCTGCGAATCGGGAGCGGCGATACCGTAAAGCACGAGGAAGCG
GTCAGCCCATTCGCCGCCAAGCTCTTCAGCAATATCACGGGTAGCCAACGCTATGTCCTGATAGCGGTCCGCCACA
CCCAGCCGGCCACAGTCGATGAATCCAGAAAAGCGGCCATTTTCCACCATGATATTCGGCAAGCAGGCATCGCCA
TGGGTCACGACGAGATCATCGCCGTCGGGCATGCGCGCCTTGAGCCTGGCGAACAGTTCGGCTGGCGCGAGCCC
CTGATGCTCTTCGTCCAGATCATCCTGATCGACAAGACCGGCTTCCATCCGAGTACGTGCTCGCTCGATGCGATGTT
TCGCTTGGTGGTCGAATGGGCAGGTAGCCGGATCAAGCGTATGCAGCCGCCGCATTGCATCAGCCATGATGGATA
CTTTCTCGGCAGGAGCAAGGTGAGATGACAGGAGATCCTGCCCCGGCACTTCGCCCAATAGCAGCCAGTCCCTTC
CCGCTTCAGTGACAACGTCGAGCACAGCTGCGCAAGGAACGCCCGTCGTGGCCAGCCACGATAGCCGCGCTGCCT
CGTCCTGCAGTTCATTCAGGGCACCGGACAGGTCGGTCTTGACAAAAAGAACCGGGCGCCCCTGCGCTGACAGCC
GGAACACGGCGGCATCAGAGCAGCCGATTGTCTGTTGTGCCCAGTCATAGCCGAATAGCCTCTCCACCCAAGCGG
CCGGAGAACCTGCGTGCAATCCATCTTGTTCAATCATGCGAAACGATCCAGATCCGGTGCAGATTATTTGGATTGA
GAGTGAATATGAGACTCTAATTGGATACCGAGGGGAATTTATGGAACGTCAGTGGAGCATTTTTGACAAGAAATA
TTTGCTAGCTGATAGTGACCTTAGGCGACTTTTGAACGCGCAATAATGGTTTCTGACGTATGTGCTTAGCTCATTAA
ACTCCAGAAACCCGCGGCTGAGTGGCTCCTTCAACGTTGCGGTTCTGTCAGTTCCAAACGTAAAACGGCTTGTCCC
GCGTCATCGGCGGGGGTCATAACGTGACTCCCTTAATTCTCCGCTCATGATGAATTCAGAAATAAATTCAGCCTAA
TTCGGCGTTAATTCAGTACATTAAAAACGTCCGCAATGTGTTATTAAGTTGTCTAAGCGTCAATTTGTTTACACCAC
AATATATCCTGCCACCAGCCAGCCAACAGCTCCCCGACCGGCAGCTCGGCACAAAATCACCACTCGATACAGGCA
GCCCATCAGTCCGGGACGGCGTCAGCGGGAGAGCCGTTGTAAGGCGGCAGACTTTGCTCATGTTACCGATGCTAT
TCGGAAGAACGGCAACTAAGCTGCCGGGTTTGAAACACGGATGATCTCGCGGAGGGTAGCATGTTGATTGTAAC
GATGACAGAGCGTTGCTGCCTGTGATCAATTCGGCACGAACCCAGTGGACATAAGCCTGTTCGGTTCGTAAGCT
GTAATGCAAGTAGCGTATGCGCTCACGCAACTGGTCCAGAACCTTGACCGAACGCAGCGGTGGTAACGGCGCAGT
GGCGGTTTTCATGGCTTGTTATGACTGTTTTTTTGGGGTACAGTCTATGCCTCGGGCATCCAAGCAGCAAGCGCGT
TACGCCGTGGGTCGATGTTTGATGTTATGGAGCAGCAACGATGTTACGCAGCAGGGCAGTCGCCCTAAAACAAAG
TTAAACATCATGGGGGAAGCGGTGATCGCCGAAGTATCGACTCAACTATCAGAGGTAGTTGGCGTCATCGAGCGC
CATCTCGAACCGACGTTGCTGGCCGTACATTTGTACGGCTCCGCAGTGGATGGCGGCCTGAAGCCACACAGTGAT
ATTGATTTGCTGGTTACGGTGACCGTAAGGCTTGATGAAACAACGCGGCGAGCTTTGATCAACGACCTTTTGGAA
ACTTCGGCTTCCCCTGGAGAGAGCGAGATTCTCCGCGCTGTAGAAGTCACCATTGTTGTGCACGACGACATCATTC
CGTGGCGTTATCCAGCTAAGCGCGAACTGCAATTTGGAGAATGGCAGCGCAATGACATTCTTGCAGGTATCTTCG
AGCCAGCCACGATCGACATTGATCTGGCTATCTTGCTGACAAAAGCAAGAGAACATAGCGTTGCCTTGGTAGGTC
CAGCGGCGGAGGAACTCTTTGATCCGGTTCCTGAACAGGATCTATTTGAGGCGCTAAATGAAACCTTAACGCTATG
GAACTCGCCGCCCGACTGGGCTGGCGATGAGCGAAATGTAGTGCTTACGTTGTCCCGCATTTGGTACAGCGCAGT
AACCGGCAAAATCGCGCCGAAGGATGTCGCTGCCGACTGGGCAATGGAGCGCCTGCCGGCCCAGTATCAGCCCG
TCATACTTGAAGCTAGACAGGCTTATCTTGGACAAGAAGAAGATCGCTTGGCCTCGCGCGCAGATCAGTTGGAAG
AATTTGTCCACTACGTGAAAGGCGAGATCACCAAGGTAGTCGGCAAATAATGTCTAGCTAGAAATTCGTTCAAGCC
GACGCCGCTTCGCGGCGCGGCTTAACTCAAGCGTTAGATGCACTAAGCACATAATTGCTCACAGCCAAACTATCAG
GTCAAGTCTGCTTTTATTATTTTTAAGCGTGCATAATAAGCCCTACACAAATTGGGAGATATATCATGCATGACCAA
AATCCCTTAACGTGAGTTTTCGTTCCACTGAGCGTCAGACCCCGTAGAAAAGATCAAAGGATCTTCTTGAGATCCTT
TTTTTCTGCGCGTAATCTGCTGCTTGCAAACAAAAAAACCACCGCTACCAGCGGTGGTTTGTTTGCCGGATCAAGA
GCTACCAACTCTTTTTCCGAAGGTAACTGGCTTCAGCAGAGCGCAGATACCAAATACTGTCCTTCTAGTGTAGCCGT
AGTTAGGCCACCACTTCAAGAACTCTGTAGCACCGCCTACATACCTCGCTCTGCTAATCCTGTTACCAGTGGCTGCT
GCCAGTGGCGATAAGTCGTGTCTTACCGGGTTGGACTCAAGACGATAGTTACCGGATAAGGCGCAGCGGTCGGG
CTGAACGGGGGGTTCGTGCACACAGCCCAGCTTGGAGCGAACGACCTACACCGAACTGAGATACCTACAGCGTG
AGCTATGAGAAAGCGCCACGCTTCCCGAAGGGAGAAAGGCGGACAGGTATCCGGTAAGCGGCAGGGTCGGAAC
AGGAGAGCGCACGAGGGAGCTTCCAGGGGGAAACGCCTGGTATCTTTATAGTCCTGTCGGGTTTCGCCACCTCTG
 
ACTTGAGCGTCGATTTTTGTGATGCTCGTCAGGGGGGCGGAGCCTATGGAAAAACGCCAGCAACGCGGCCTTTTT
ACGGTTCCTGGCCTTTTGCTGGCCTTTTGCTCACATGTTCTTTCCTGCGTTATCCCCTGATTCTGTGGATAACCGTAT
TACCGCCTTTGAGTGAGCTGATACCGCTCGCCGCAGCCGAACGACCGAGCGCAGCGAGTCAGTGAGCGAGGAAG
CGGAAGAGCGCCTGATGCGGTATTTTCTCCTTACGCATCTGTGCGGTATTTCACACCGCATATGGTGCACTCTCAGT
ACAATCTGCTCTGATGCCGCATAGTTAAGCCAGTATACACTCCGCTATCGCTACGTGACTGGGTCATGGCTGCGCC
CCGACACCCGCCAACACCCGCTGACGCGCCCTGACGGGCTTGTCTGCTCCCGGCATCCGCTTACAGACAAGCTGTG
ACCGTCTCCGGGAGCTGCATGTGTCAGAGGTTTTCACCGTCATCACCGAAACGCGCGAGGCAGGGTGCCTTGATG
TGGGCGCCGGCGGTCGAGTGGCGACGGCGCGGCTTGTCCGCGCCCTGGTAGATTGCCTGGCCGTAGGCCAGCCA
TTTTTGAGCGGCCAGCGGCCGCGATAGGCCGACGCGAAGCGGCGGGGCGTAGGGAGCGCAGCGACCGAAGGGT
AGGCGCTTTTTGCAGCTCTTCGGCTGTGCGCTGGCCAGACAGTTATGCACAGGCCAGGCGGGTTTTAAGAGTTTTA
ATAAGTTTTAAAGAGTTTTAGGCGGAAAAATCGCCTTTTTTCTCTTTTATATCAGTCACTTACATGTGTGACCGGTTC
CCAATGTACGGCTTTGGGTTCCCAATGTACGGGTTCCGGTTCCCAATGTACGGCTTTGGGTTCCCAATGTACGTGC
TATCCACAGGAAAGAGACCTTTTCGACCTTTTTCCCCTGCTAGGGCAATTTGCCCTAGCATCTGCTCCGTACATTAG
GAACCGGCGGATGCTTCGCCCTCGATCAGGTTGCGGTAGCGCATGACTAGGATCGGGCCAGCCTGCCCCGCCTCC
TCCTTCAAATCGTACTCCGGCAGGTCATTTGACCCGATCAGCTTGCGCACGGTGAAACAGAACTTCTTGAACTCTCC
GGCGCTGCCACTGCGTTCGTAGATCGTCTTGAACAACCATCTGGCTTCTGCCTTGCCTGCGGCGCGGCGTGCCAGG
CGGTAGAGAAAACGGCCGATGCCGGGATCGATCAAAAAGTAATCGGGGTGAACCGTCAGCACGTCCGGGTTCTT
GCCTTCTGTGATCTCGCGGTACATCCAATCAGCTAGCTCGATCTCGATGTACTCCGGCCGCCCGGTTTCGCTCTTTA
CGATCTTGTAGCGGCTAATCAAGGCTTCACCCTCGGATACCGTCACCAGGCGGCCGTTCTTGGCCTTCTTCGTACG
CTGCATGGCAACGTGCGTGGTGTTTAACCGAATGCAGGTTTCTACCAGGTCGTCTTTCTGCTTTCCGCCATCGGCTC
GCCGGCAGAACTTGAGTACGTCCGCAACGTGTGGACGGAACACGCGGCCGGGCTTGTCTCCCTTCCCTTCCCGGT
ATCGGTTCATGGATTCGGTTAGATGGGAAACCGCCATCAGTACCAGGTCGTAATCCCACACACTGGCCATGCCGG
CCGGCCCTGCGGAAACCTCTACGTGCCCGTCTGGAAGCTCGTAGCGGATCACCTCGCCAGCTCGTCGGTCACGCTT
CGACAGACGGAAAACGGCCACGTCCATGATGCTGCGACTATCGCGGGTGCCCACGTCATAGAGCATCGGAACGA
AAAAATCTGGTTGCTCGTCGCCCTTGGGCGGCTTCCTAATCGACGGCGCACCGGCTGCCGGCGGTTGCCGGGATT
CTTTGCGGATTCGATCAGCGGCCGCTTGCCACGATTCACCGGGGCGTGCTTCTGCCTCGATGCGTTGCCGCTGGGC
GGCCTGCGCGGCCTTCAACTTCTCCACCAGGTCATCACCCAGCGCCGCGCCGATTTGTACCGGGCCGGATGGTTTG
CGACCGCTCACGCCGATTCCTCGGGCTTGGGGGTTCCAGTGCCATTGCAGGGCCGGCAGACAACCCAGCCGCTTA
CGCCTGGCCAACCGCCCGTTCCTCCACACATGGGGCATTCCACGGCGTCGGTGCCTGGTTGTTCTTGATTTTCCATG
CCGCCTCCTTTAGCCGCTAAAATTCATCTACTCATTTATTCATTTGCTCATTTACTCTGGTAGCTGCGCGATGTATTC
AGATAGCAGCTCGGTAATGGTCTTGCCTTGGCGTACCGCGTACATCTTCAGCTTGGTGTGATCCTCCGCCGGCAAC
TGAAAGTTGACCCGCTTCATGGCTGGCGTGTCTGCCAGGCTGGCCAACGTTGCAGCCTTGCTGCTGCGTGCGCTCG
GACGGCCGGCACTTAGCGTGTTTGTGCTTTTGCTCATTTTCTCTTTACCTCATTAACTCAAATGAGTTTTGATTTAAT
TTCAGCGGCCAGCGCCTGGACCTCGCGGGCAGCGTCGCCCTCGGGTTCTGATTCAAGAACGGTTGTGCCGGCGGC
GGCAGTGCCTGGGTAGCTCACGCGCTGCGTGATACGGGACTCAAGAATGGGCAGCTCGTACCCGGCCAGCGCCT
CGGCAACCTCACCGCCGATGCGCGTGCCTTTGATCGCCCGCGACACGACAAAGGCCGCTTGTAGCCTTCCATCCGT
GACCTCAATGCGCTGCTTAACCAGCTCCACCAGGTCGGCGGTGGCCCATATGTCGTAAGGGCTTGGCTGCACCGG
AATCAGCACGAAGTCGGCTGCCTTGATCGCGGACACAGCCAAGTCCGCCGCCTGGGGCGCTCCGTCGATCACTAC
GAAGTCGCGCCGGCCGATGGCCTTCACGTCGCGGTCAATCGTCGGGCGGTCGATGCCGACAACGGTTAGCGGTT
GATCTTCCCGCACGGCCGCCCAATCGCGGGCACTGCCCTGGGGATCGGAATCGACTAACAGAACATCGGCCCCGG
CGAGTTGCAGGGCGCGGGCTAGATGGGTTGCGATGGTCGTCTTGCCTGACCCGCCTTTCTGGTTAAGTACAGCGA
TAACCTTCATGCGTTCCCCTTGCGTATTTGTTTATTTACTCATCGCATCATATACGCAGCGACCGCATGACGCAAGCT
GTTTTACTCAAATACACATCACCTTTTTAGACGGCGGCGCTCGGTTTCTTCAGCGGCCAAGCTGGCCGGCCAGGCC
GCCAGCTTGGCATCAGACAAACCGGCCAGGATTTCATGCAGCCGCACGGTTGAGACGTGCGCGGGCGGCTCGAA
CACGTACCCGGCCGCGATCATCTCCGCCTCGATCTCTTCGGTAATGAAAAACGGTTCGTCCTGGCCGTCCTGGTGC
GGTTTCATGCTTGTTCCTCTTGGCGTTCATTCTCGGCGGCCGCCAGGGCGTCGGCCTCGGTCAATGCGTCCTCACG
GAAGGCACCGCGCCGCCTGGCCTCGGTGGGCGTCACTTCCTCGCTGCGCTCAAGTGCGCGGTACAGGGTCGAGC
GATGCACGCCAAGCAGTGCAGCCGCCTCTTTCACGGTGCGGCCTTCCTGGTCGATCAGCTCGCGGGCGTGCGCGA
TCTGTGCCGGGGTGAGGGTAGGGCGGGGGCCAAACTTCACGCCTCGGGCCTTGGCGGCCTCGCGCCCGCTCCGG
GTGCGGTCGATGATTAGGGAACGCTCGAACTCGGCAATGCCGGCGAACACGGTCAACACCATGCGGCCGGCCGG
CGTGGTGGTGTCGGCCCACGGCTCTGCCAGGCTACGCAGGCCCGCGCCGGCCTCCTGGATGCGCTCGGCAATGTC
CAGTAGGTCGCGGGTGCTGCGGGCCAGGCGGTCTAGCCTGGTCACTGTCACAACGTCGCCAGGGCGTAGGTGGT
CAAGCATCCTGGCCAGCTCCGGGCGGTCGCGCCTGGTGCCGGTGATCTTCTCGGAAAACAGCTTGGTGCAGCCGG
 
CCGCGTGCAGTTCGGCCCGTTGGTTGGTCAAGTCCTGGTCGTCGGTGCTGACGCGGGCATAGCCCAGCAGGCCAG
CGGCGGCGCTCTTGTTCATGGCGTAATGTCTCCGGTTCTAGTCGCAAGTATTCTACTTTATGCGACTAAAACACGC
GACAAGAAAACGCCAGGAAAAGGGCAGGGCGGCAGCCTGTCGCGTAACTTAGGACTTGTGCGACATGTCGTTTT
CAGAAGACGGCTGCACTGAACGTCAGAAGCCGACTGCACTATAGCAGCGGAGGGGTTGGATCAAAGTACTTTGA
TCCCGAGGGGAACCCTGTGGTTGGCATGCACATACAAATGGACGAACGGATAAAC
 
>crtB_stop_pGWB605
CTTTTCACGCCCTTTTAAATATCCGATTATTCTAATAAACGCTCTTTTCTCTTAGGTTTACCCGCCAATATATCCTGTC
AAACACTGATAGTTTAAACTGAAGGCGGGAAACGACAATCTGATCCAAGCTCAAGCTGCTCTAGCATTCGCCATTC
AGGCTGCGCAACTGTTGGGAAGGGCGATCGGTGCGGGCCTCTTCGCTATTACGCCAGCTGGCGAAAGGGGGATG
TGCTGCAAGGCGATTAAGTTGGGTAACGCCAGGGTTTTCCCAGTCACGACGTTGTAAAACGACGGCCAGTGCCAA
GCTTGCATGCCTGCAGGTCCCCAGATTAGCCTTTTCAATTTCAGAAAGAATGCTAACCCACAGATGGTTAGAGAGG
CTTACGCAGCAGGTCTCATCAAGACGATCTACCCGAGCAATAATCTCCAGGAAATCAAATACCTTCCCAAGAAGGT
TAAAGATGCAGTCAAAAGATTCAGGACTAACTGCATCAAGAACACAGAGAAAGATATATTTCTCAAGATCAGAAG
TACTATTCCAGTATGGACGATTCAAGGCTTGCTTCACAAACCAAGGCAAGTAATAGAGATTGGAGTCTCTAAAAAG
GTAGTTCCCACTGAATCAAAGGCCATGGAGTCAAAGATTCAAATAGAGGACCTAACAGAACTCGCCGTAAAGACT
GGCGAACAGTTCATACAGAGTCTCTTACGACTCAATGACAAGAAGAAAATCTTCGTCAACATGGTGGAGCACGAC
ACACTTGTCTACTCCAAAAATATCAAAGATACAGTCTCAGAAGACCAAAGGGCAATTGAGACTTTTCAACAAAGGG
TAATATCCGGAAACCTCCTCGGATTCCATTGCCCAGCTATCTGTCACTTTATTGTGAAGATAGTGGAAAAGGAAGG
TGGCTCCTACAAATGCCATCATTGCGATAAAGGAAAGGCCATCGTTGAAGATGCCTCTGCCGACAGTGGTCCCAA
AGATGGACCCCCACCCACGAGGAGCATCGTGGAAAAAGAAGACGTTCCAACCACGTCTTCAAAGCAAGTGGATTG
ATGTGATATCTCCACTGACGTAAGGGATGACGCACAATCCCACTATCCTTCGCAAGACCCTTCCTCTATATAAGGAA
GTTCATTTCATTTGGAGAGAACACGGGGGACTCTAGAGTTATCAACAAGTTTGTACAAAAAAGCAGGCTTCATGA
ATAATCCGTCGTTACTCAATCATGCGGTCGAAACGATGGCAGTTGGCTCGAAAAGTTTTGCGACAGCCTCAAAGTT
ATTTGATGCAAAAACCCGGCGCAGCGTACTGATGCTCTACGCCTGGTGCCGCCATTGTGACGATGTTATTGACGAT
CAGACGCTGGGCTTTCAGGCCCGGCAGCCTGCCTTACAAACGCCCGAACAACGTCTGATGCAACTTGAGATGAAAA
CGCGCCAGGCCTATGCAGGATCGCAGATGCACGAACCGGCGTTTGCGGCTTTTCAGGAAGTGGCTATGGCTCATG
ATATCGCCCCGGCTTACGCGTTTGATCATCTGGAAGGCTTCGCCATGGATGTACGCGAAGCGCAATACAGCCAACT
GGATGATACGCTGCGCTATTGCTATCACGTTGCAGGCGTTGTCGGCTTGATGATGGCGCAAATCATGGGCGTGCG
GGATAACGCCACGCTGGACCGCGCCTGTGACCTTGGGCTGGCATTTCAGTTGACCAATATTGCTCGCGATATTGTG
GACGATGCGCATGCGGGCCGCTGTTATCTGCCGGCAAGCTGGCTGGAGCATGAAGGTCTGAACAAAGAGAATTAT
GCGGCACCTGAAAACCGTCAGGCGCTGAGCCGTATCGCCCGTCGTTTGGTGCAGGAAGCAGAACCTTACTATTTGT
CTGCCACAGCCGGCCTGGCAGGGTTGCCCCTGCGTTCCGCCTGGGCAATCGCTACGGCGAAGCAGGTTTACCGGA
AAATAGGTGTCAAAGTTGAACAGGCCGGTCAGCAAGCCTGGGATCAGCGGCAGTCAACGACCACGCCCGAAAAT
TAACGCTGCTGCTGGCCGCCTCTGGTCAGGCCCTTACTTCCCGGATGCGGGCTCATCCTCCCCGCCCTGCGCATCTC
TGGCAGCGCCCGCTCTAGACCCAGCTTTCTTGTACAAAGTGGTTCGATCTAGAGGATCCATGGTGAGCAAGGGCG
AGGAGCTGTTCACCGGGGTGGTGCCCATCCTGGTCGAGCTGGACGGCGACGTGAACGGCCACAAGTTCAGCGTG
TCCGGCGAGGGCGAGGGCGATGCCACCTACGGCAAGCTGACCCTGAAGTTCATCTGCACCACCGGCAAGCTGCCC
GTGCCCTGGCCCACCCTCGTGACCACCTTCACCTACGGCGTGCAGTGCTTCAGCCGCTACCCCGACCACATGAAGC
AGCACGACTTCTTCAAGTCCGCCATGCCCGAAGGCTACGTCCAGGAGCGCACCATCTTCTTCAAGGACGACGGCA
ACTACAAGACCCGCGCCGAGGTGAAGTTCGAGGGCGACACCCTGGTGAACCGCATCGAGCTGAAGGGCATCGAC
TTCAAGGAGGACGGCAACATCCTGGGGCACAAGCTGGAGTACAACTACAACAGCCACAACGTCTATATCATGGCC
GACAAGCAGAAGAACGGCATCAAGGTGAACTTCAAGATCCGCCACAACATCGAGGACGGCAGCGTGCAGCTCGC
CGACCACTACCAGCAGAACACCCCCATCGGCGACGGCCCCGTGCTGCTGCCCGACAACCACTACCTGAGCACCCA
GTCCGCCCTGAGCAAAGACCCCAACGAGAAGCGCGATCACATGGTCCTGCTGGAGTTCGTGACCGCCGCCGGGAT
CACTCACGGCATGGACGAGCTGTACAAGTAAAGCGGCCCGAGCTCGAATTTCCCCGATCGTTCAAACATTTGGCA
ATAAAGTTTCTTAAGATTGAATCCTGTTGCCGGTCTTGCGATGATTATCATATAATTTCTGTTGAATTACGTTAAGC
ATGTAATAATTAACATGTAATGCATGACGTTATTTATGAGATGGGTTTTTATGATTAGAGTCCCGCAATTATACATT
TAATACGCGATAGAAAACAAAATATAGCGCGCAAACTAGGATAAATTATCGCGCGCGGTGTCATCTATGTTACTA
GATCGGGAATTGGTTCCGGAACCAATTCGTAATCATGGTCATAGCTGTTTCCTGTGTGAAATTGTTATCCGCTCACA
ATTCCACACAACATACGAGCCGGAAGCATAAAGTGTAAAGCCTGGGGTGCCTAATGAGTGAGCTAACTCACATTA
GGCTGAATTAGGCGCGCCTATTTCTATTAATTCCCGATCTAGTAACATAGATGACACCGCGCGCGATAATTTATCCT
 
AGTTTGCGCGCTATATTTTGTTTTCTATCGCGTATTAAATGTATAATTGCGGGACTCTAATCATAAAAACCCATCTCA
TAAATAACGTCATGCATTACATGTTAATTATTACATGCTTAACGTAATTCAACAGAAATTATATGATAATCATCGCA
AGACCGGCAACAGGATTCAATCTTAAGAAACTTTATTGCCAAATGTTTGAACGATCGGGGAAATTCGAGCTGGGG
ACGTCCCCAGCTCGGTACCCCTCCTGGGCACGGTCGTACGCGATCCGCCGCGGTTCCACCTCACCATTGCGGGTA
TGCCAGTTGAGTTCGATGAGTTCCCGTACCAGCTCCAACTCGGGCGATGCCACGCGCACTCCGTTCAGATCTCGGT
GACGGGCAGGACCGGACGGGGCGGTACCGGCAGGCTGAAGTCCAGCTGCCAGAAACCCACGTCATGCCAGTTCC
CGTGCTTGAAGCCGGCCGCCCGCAGCATGCCGCGGGGGGCATATCCGAGCGCCTCGTGCATGCGCACGCTCGGG
TCGTTGGGCAGCCCGATGACAGCGACCACGCTCTTGAAGCCCTGTGCCTCCAGGGACTTCAGCAGGTGGGTGTAG
AGCGTGGAGCCCAGTCCCGTCCGCTGGTGGCGGGGGGAGACGTACACGGTCGACTCGGCCGTCCAGTCGTAGGC
GTTGCGTGCCTTCCAGGGGCCCGCGTAGGCGATGCCGGCGACCTCGCCGTCCACCTCGGCGACGAGCCAGGGAT
AGCGCTCCCGCAGACGGACGAGGTCGTCCGTCCACTCCTGCGGTTCCTGCGGCTCGGTACGGAAGTTGACCGTGC
TTGTCTCGATGTAGTGGTTGACGATGGTGCAGACCGCCGGCATGTCCGCCTCGGTGGCACGGCGGATGTCGGCCG
GGCGTCGTTCTGGGCTCATATCTTATTGCCCCCCGGGATCAGATCTGGATTGAGAGTGAATATGAGACTCTAATTG
GATACCGAGGGGAATTTATGGAACGTCAGTGGAGCATTTTTGACAAGAAATATTTGCTAGCTGATAGTGACCTTA
GGCGACTTTTGAACGCGCAATAATGGTTTCTGACGTATGTGCTTAGCTCATTAAACTCCAGAAACCCGCGGCTGAG
TGGCTCCTTCAACGTTGCGGTTCTGTCAGTTCCAAACGTAAAACGGCTTGTCCCGCGTCATCGGCGGGGGTCATAA
CGTGACTCCCTTAATTCTCCGCTCATGATCTTGATCCCCTGCGCCATCAGATCCTTGGCGGCAAGAAAGCCATCCAG
TTTACTTTGCAGGGCTTCCCAACCTTACCAGAGGGCGCCCCAGCTGGCAATTCCGGTTCGCTTGCTGTCCATAAAAC
CGCCCAGTCTAGCTATCGCCATGTAAGCCCACTGCAAGCTACCTGCTTTCTCTTTGCGCTTGCGTTTTCCCTTGTCCA
GATAGCCCAGTAGCTGACATTCATCCGGGGTCAGCACCGTTTCTGCGGACTGGCTTTCTACGTGTTCCGCTTCCTTT
AGCAGCCCTTGCGCCCTGAGTGCTTGCGGCAGCGTGAAGCTGATCCGTCGAGATTAATAGAAATAAATTCAGCCT
AATTCGGCGTTAATTCAGTACATTAAAAACGTCCGCAATGTGTTATTAAGTTGTCTAAGCGTCAATTTGTTTACACC
ACAATATATCCTGCCACCAGCCAGCCAACAGCTCCCCGACCGGCAGCTCGGCACAAAATCACCACTCGATACAGGC
AGCCCATCAGTCCGGGACGGCGTCAGCGGGAGAGCCGTTGTAAGGCGGCAGACTTTGCTCATGTTACCGATGCTA
TTCGGAAGAACGGCAACTAAGCTGCCGGGTTTGAAACACGGATGATCTCGCGGAGGGTAGCATGTTGATTGTAAC
GATGACAGAGCGTTGCTGCCTGTGATCAATTCGGGCACGAACCCAGTGGACATAAGCCTGTTCGGTTCGTAAGCT
GTAATGCAAGTAGCGTATGCGCTCACGCAACTGGTCCAGAACCTTGACCGAACGCAGCGGTGGTAACGGCGCAGT
GGCGGTTTTCATGGCTTGTTATGACTGTTTTTTTGGGGTACAGTCTATGCCTCGGGCATCCAAGCAGCAAGCGCGT
TACGCCGTGGGTCGATGTTTGATGTTATGGAGCAGCAACGATGTTACGCAGCAGGGCAGTCGCCCTAAAACAAAG
TTAAACATCATGGGGGAAGCGGTGATCGCCGAAGTATCGACTCAACTATCAGAGGTAGTTGGCGTCATCGAGCGC
CATCTCGAACCGACGTTGCTGGCCGTACATTTGTACGGCTCCGCAGTGGATGGCGGCCTGAAGCCACACAGTGAT
ATTGATTTGCTGGTTACGGTGACCGTAAGGCTTGATGAAACAACGCGGCGAGCTTTGATCAACGACCTTTTGGAA
ACTTCGGCTTCCCCTGGAGAGAGCGAGATTCTCCGCGCTGTAGAAGTCACCATTGTTGTGCACGACGACATCATTC
CGTGGCGTTATCCAGCTAAGCGCGAACTGCAATTTGGAGAATGGCAGCGCAATGACATTCTTGCAGGTATCTTCG
AGCCAGCCACGATCGACATTGATCTGGCTATCTTGCTGACAAAAGCAAGAGAACATAGCGTTGCCTTGGTAGGTC
CAGCGGCGGAGGAACTCTTTGATCCGGTTCCTGAACAGGATCTATTTGAGGCGCTAAATGAAACCTTAACGCTATG
GAACTCGCCGCCCGACTGGGCTGGCGATGAGCGAAATGTAGTGCTTACGTTGTCCCGCATTTGGTACAGCGCAGT
AACCGGCAAAATCGCGCCGAAGGATGTCGCTGCCGACTGGGCAATGGAGCGCCTGCCGGCCCAGTATCAGCCCG
TCATACTTGAAGCTAGACAGGCTTATCTTGGACAAGAAGAAGATCGCTTGGCCTCGCGCGCAGATCAGTTGGAAG
AATTTGTCCACTACGTGAAAGGCGAGATCACCAAGGTAGTCGGCAAATAATGTCTAGCTAGAAATTCGTTCAAGCC
GACGCCGCTTCGCGGCGCGGCTTAACTCAAGCGTTAGATGCACTAAGCACATAATTGCTCACAGCCAAACTATCAG
GTCAAGTCTGCTTTTATTATTTTTAAGCGTGCATAATAAGCCCTACACAAATTGGGAGATATATCATGCATGACCAA
AATCCCTTAACGTGAGTTTTCGTTCCACTGAGCGTCAGACCCCGTAGAAAAGATCAAAGGATCTTCTTGAGATCCTT
TTTTTCTGCGCGTAATCTGCTGCTTGCAAACAAAAAAACCACCGCTACCAGCGGTGGTTTGTTTGCCGGATCAAGA
GCTACCAACTCTTTTTCCGAAGGTAACTGGCTTCAGCAGAGCGCAGATACCAAATACTGTCCTTCTAGTGTAGCCGT
AGTTAGGCCACCACTTCAAGAACTCTGTAGCACCGCCTACATACCTCGCTCTGCTAATCCTGTTACCAGTGGCTGCT
GCCAGTGGCGATAAGTCGTGTCTTACCGGGTTGGACTCAAGACGATAGTTACCGGATAAGGCGCAGCGGTCGGG
CTGAACGGGGGGTTCGTGCACACAGCCCAGCTTGGAGCGAACGACCTACACCGAACTGAGATACCTACAGCGTG
AGCTATGAGAAAGCGCCACGCTTCCCGAAGGGAGAAAGGCGGACAGGTATCCGGTAAGCGGCAGGGTCGGAAC
AGGAGAGCGCACGAGGGAGCTTCCAGGGGGAAACGCCTGGTATCTTTATAGTCCTGTCGGGTTTCGCCACCTCTG
ACTTGAGCGTCGATTTTTGTGATGCTCGTCAGGGGGGCGGAGCCTATGGAAAAACGCCAGCAACGCGGCCTTTTT
ACGGTTCCTGGCCTTTTGCTGGCCTTTTGCTCACATGTTCTTTCCTGCGTTATCCCCTGATTCTGTGGATAACCGTAT
TACCGCCTTTGAGTGAGCTGATACCGCTCGCCGCAGCCGAACGACCGAGCGCAGCGAGTCAGTGAGCGAGGAAG
 
CGGAAGAGCGCCTGATGCGGTATTTTCTCCTTACGCATCTGTGCGGTATTTCACACCGCATATGGTGCACTCTCAGT
ACAATCTGCTCTGATGCCGCATAGTTAAGCCAGTATACACTCCGCTATCGCTACGTGACTGGGTCATGGCTGCGCC
CCGACACCCGCCAACACCCGCTGACGCGCCCTGACGGGCTTGTCTGCTCCCGGCATCCGCTTACAGACAAGCTGTG
ACCGTCTCCGGGAGCTGCATGTGTCAGAGGTTTTCACCGTCATCACCGAAACGCGCGAGGCAGGGTGCCTTGATG
TGGGCGCCGGCGGTCGAGTGGCGACGGCGCGGCTTGTCCGCGCCCTGGTAGATTGCCTGGCCGTAGGCCAGCCA
TTTTTGAGCGGCCAGCGGCCGCGATAGGCCGACGCGAAGCGGCGGGGCGTAGGGAGCGCAGCGACCGAAGGGT
AGGCGCTTTTTGCAGCTCTTCGGCTGTGCGCTGGCCAGACAGTTATGCACAGGCCAGGCGGGTTTTAAGAGTTTTA
ATAAGTTTTAAAGAGTTTTAGGCGGAAAAATCGCCTTTTTTCTCTTTTATATCAGTCACTTACATGTGTGACCGGTTC
CCAATGTACGGCTTTGGGTTCCCAATGTACGGGTTCCGGTTCCCAATGTACGGCTTTGGGTTCCCAATGTACGTGC
TATCCACAGGAAAGAGACCTTTTCGACCTTTTTCCCCTGCTAGGGCAATTTGCCCTAGCATCTGCTCCGTACATTAG
GAACCGGCGGATGCTTCGCCCTCGATCAGGTTGCGGTAGCGCATGACTAGGATCGGGCCAGCCTGCCCCGCCTCC
TCCTTCAAATCGTACTCCGGCAGGTCATTTGACCCGATCAGCTTGCGCACGGTGAAACAGAACTTCTTGAACTCTCC
GGCGCTGCCACTGCGTTCGTAGATCGTCTTGAACAACCATCTGGCTTCTGCCTTGCCTGCGGCGCGGCGTGCCAGG
CGGTAGAGAAAACGGCCGATGCCGGGATCGATCAAAAAGTAATCGGGGTGAACCGTCAGCACGTCCGGGTTCTT
GCCTTCTGTGATCTCGCGGTACATCCAATCAGCTAGCTCGATCTCGATGTACTCCGGCCGCCCGGTTTCGCTCTTTA
CGATCTTGTAGCGGCTAATCAAGGCTTCACCCTCGGATACCGTCACCAGGCGGCCGTTCTTGGCCTTCTTCGTACG
CTGCATGGCAACGTGCGTGGTGTTTAACCGAATGCAGGTTTCTACCAGGTCGTCTTTCTGCTTTCCGCCATCGGCTC
GCCGGCAGAACTTGAGTACGTCCGCAACGTGTGGACGGAACACGCGGCCGGGCTTGTCTCCCTTCCCTTCCCGGT
ATCGGTTCATGGATTCGGTTAGATGGGAAACCGCCATCAGTACCAGGTCGTAATCCCACACACTGGCCATGCCGG
CCGGCCCTGCGGAAACCTCTACGTGCCCGTCTGGAAGCTCGTAGCGGATCACCTCGCCAGCTCGTCGGTCACGCTT
CGACAGACGGAAAACGGCCACGTCCATGATGCTGCGACTATCGCGGGTGCCCACGTCATAGAGCATCGGAACGA
AAAAATCTGGTTGCTCGTCGCCCTTGGGCGGCTTCCTAATCGACGGCGCACCGGCTGCCGGCGGTTGCCGGGATT
CTTTGCGGATTCGATCAGCGGCCGCTTGCCACGATTCACCGGGGCGTGCTTCTGCCTCGATGCGTTGCCGCTGGGC
GGCCTGCGCGGCCTTCAACTTCTCCACCAGGTCATCACCCAGCGCCGCGCCGATTTGTACCGGGCCGGATGGTTTG
CGACCGCTCACGCCGATTCCTCGGGCTTGGGGGTTCCAGTGCCATTGCAGGGCCGGCAGACAACCCAGCCGCTTA
CGCCTGGCCAACCGCCCGTTCCTCCACACATGGGGCATTCCACGGCGTCGGTGCCTGGTTGTTCTTGATTTTCCATG
CCGCCTCCTTTAGCCGCTAAAATTCATCTACTCATTTATTCATTTGCTCATTTACTCTGGTAGCTGCGCGATGTATTC
AGATAGCAGCTCGGTAATGGTCTTGCCTTGGCGTACCGCGTACATCTTCAGCTTGGTGTGATCCTCCGCCGGCAAC
TGAAAGTTGACCCGCTTCATGGCTGGCGTGTCTGCCAGGCTGGCCAACGTTGCAGCCTTGCTGCTGCGTGCGCTCG
GACGGCCGGCACTTAGCGTGTTTGTGCTTTTGCTCATTTTCTCTTTACCTCATTAACTCAAATGAGTTTTGATTTAAT
TTCAGCGGCCAGCGCCTGGACCTCGCGGGCAGCGTCGCCCTCGGGTTCTGATTCAAGAACGGTTGTGCCGGCGGC
GGCAGTGCCTGGGTAGCTCACGCGCTGCGTGATACGGGACTCAAGAATGGGCAGCTCGTACCCGGCCAGCGCCT
CGGCAACCTCACCGCCGATGCGCGTGCCTTTGATCGCCCGCGACACGACAAAGGCCGCTTGTAGCCTTCCATCCGT
GACCTCAATGCGCTGCTTAACCAGCTCCACCAGGTCGGCGGTGGCCCATATGTCGTAAGGGCTTGGCTGCACCGG
AATCAGCACGAAGTCGGCTGCCTTGATCGCGGACACAGCCAAGTCCGCCGCCTGGGGCGCTCCGTCGATCACTAC
GAAGTCGCGCCGGCCGATGGCCTTCACGTCGCGGTCAATCGTCGGGCGGTCGATGCCGACAACGGTTAGCGGTT
GATCTTCCCGCACGGCCGCCCAATCGCGGGCACTGCCCTGGGGATCGGAATCGACTAACAGAACATCGGCCCCGG
CGAGTTGCAGGGCGCGGGCTAGATGGGTTGCGATGGTCGTCTTGCCTGACCCGCCTTTCTGGTTAAGTACAGCGA
TAACCTTCATGCGTTCCCCTTGCGTATTTGTTTATTTACTCATCGCATCATATACGCAGCGACCGCATGACGCAAGCT
GTTTTACTCAAATACACATCACCTTTTTAGACGGCGGCGCTCGGTTTCTTCAGCGGCCAAGCTGGCCGGCCAGGCC
GCCAGCTTGGCATCAGACAAACCGGCCAGGATTTCATGCAGCCGCACGGTTGAGACGTGCGCGGGCGGCTCGAA
CACGTACCCGGCCGCGATCATCTCCGCCTCGATCTCTTCGGTAATGAAAAACGGTTCGTCCTGGCCGTCCTGGTGC
GGTTTCATGCTTGTTCCTCTTGGCGTTCATTCTCGGCGGCCGCCAGGGCGTCGGCCTCGGTCAATGCGTCCTCACG
GAAGGCACCGCGCCGCCTGGCCTCGGTGGGCGTCACTTCCTCGCTGCGCTCAAGTGCGCGGTACAGGGTCGAGC
GATGCACGCCAAGCAGTGCAGCCGCCTCTTTCACGGTGCGGCCTTCCTGGTCGATCAGCTCGCGGGCGTGCGCGA
TCTGTGCCGGGGTGAGGGTAGGGCGGGGGCCAAACTTCACGCCTCGGGCCTTGGCGGCCTCGCGCCCGCTCCGG
GTGCGGTCGATGATTAGGGAACGCTCGAACTCGGCAATGCCGGCGAACACGGTCAACACCATGCGGCCGGCCGG
CGTGGTGGTGTCGGCCCACGGCTCTGCCAGGCTACGCAGGCCCGCGCCGGCCTCCTGGATGCGCTCGGCAATGTC
CAGTAGGTCGCGGGTGCTGCGGGCCAGGCGGTCTAGCCTGGTCACTGTCACAACGTCGCCAGGGCGTAGGTGGT
CAAGCATCCTGGCCAGCTCCGGGCGGTCGCGCCTGGTGCCGGTGATCTTCTCGGAAAACAGCTTGGTGCAGCCGG
CCGCGTGCAGTTCGGCCCGTTGGTTGGTCAAGTCCTGGTCGTCGGTGCTGACGCGGGCATAGCCCAGCAGGCCAG
CGGCGGCGCTCTTGTTCATGGCGTAATGTCTCCGGTTCTAGTCGCAAGTATTCTACTTTATGCGACTAAAACACGC
GACAAGAAAACGCCAGGAAAAGGGCAGGGCGGCAGCCTGTCGCGTAACTTAGGACTTGTGCGACATGTCGTTTT
 
CAGAAGACGGCTGCACTGAACGTCAGAAGCCGACTGCACTATAGCAGCGGAGGGGTTGGATCAAAGTACTTTGA
TCCCGAGGGGAACCCTGTGGTTGGCATGCACATACAAATGGACGAACGGATAAAC
HDS signal peptide is shown in bold in the above sequences
crtB is shown in italics in the above sequences.
STOP codon is shown undeline in the above sequences.

In the context of the present invention, plastid-targeting signal in its N-terminus is preferably understood as the sequence of the hydroxymethylbutenyl 4-diphosphate synthase gene from *A*. *thaliana* cDNA, of which cloning procedures are detailed in Table 6 and in the Methods section below.

In the context of the present invention, a plasmid construct or an expression vector capable of accumulating at least about **0.01 µg/mg DW** of phytoene in the leaf vegetables and forage crops between 36 and 48 hpi, is understood as (p)crtB or crtbB, or any other plasmid encoding a phytoene synthase which is able to direct its translocation to the plant plastids of interest.

In a preferred embodiment of any of the above aspects, the polypeptide exhibiting phytoene synthase activity may be any known phytoene synthase capable of reaching the chloroplast, such as selected from known bacterial, algal or plant phytoene synthases. Preferably, the polypeptide exhibiting phytoene synthase activity is a CrtB, more preferably from *Pantoea ananatis* (GenBank accession number: P21683) also preferably any CrtB selected from any of the sequence identified in table 1). Also preferably, the CrtB gene expressed or overexpressed in the modified plant tissue of the invention may encode an endogenous phytoene synthase, a heterologous phytoene synthase or an improved variant of the endogenous phytoene synthase.

The polypeptide exhibiting phytoene synthase activity may also be any polypeptide exhibiting phytoene synthase activity and having at least 60%, preferably 65, 70, 75, 80, 85, 90, 95, 96, 97, 98 or 99%, identity to any phytoene synthase listed in table 1.

The CrtB gene used in the present invention may also encode an improved phytoene synthase, i.e. an enzyme that possesses at least one mutation in its sequence, in comparison with the amino acid sequence of the wild-type enzyme, said mutation leading to an increase of its activity, an increased specific catalytic activity, an increased specificity for the substrate, an increased protein or RNA stability and/or an increased intracellular concentration of the enzyme, or leading to a feedback inhibition resistant mutant.

In another preferred embodiment of any of the above aspects, examples of expression cassettes useful in the present invention comprise at least a CrtB gene operably linked to a plastid-targeting signal in its N-terminus and optionally one or more control sequences, typically comprising a transcriptional promoter and a transcription terminator, that direct the expression of said gene. A control sequence may include a promoter that is recognized by the host cell. The promoter may contain transcriptional control sequences that mediate the expression of the enzyme. The promoter may be any polynucleotide that shows transcriptional activity in the leaf vegetables or forage crops. The promoter may be a native or heterologous promoter.

Thus, in a preferred embodiment of any of the above aspects of the invention, the plasmid construct or expression vector further comprises a promoter inducing a high level of expression of the gene, i.e. a strong promoter, using elements stabilizing the corresponding messenger RNA or modifying Ribosome Binding Site (RBS) sequences and sequences surrounding them. The promoters suitable to be used in the present invention are known by the skilled person and can be constitutive or inducible, and native or heterologous.

In another embodiment of any of the above aspects, the plasmid construct or expression vector further comprises a specific promoter, i.e., one that can drive the expression of crtB and increase phytoene production in the tissue of interest, such as shown in the following nonlimiting examples:
1. if one intends to drive the accumulation of carotenoids in the early leaves of spinach, one would use spinach specific promoters PsaF (Wöstemeyer A., J. Plant Physiol. 160. 503 -508 (2003)) or PetH (Mohanty A., Indian Journal of Biochemistry and Biophysics (37); 447); or
2. if one intends to drive the accumulation of vitamin A in a forage crop such as rye grass, one could use the *LpRbcS or LpCAB* promoters *(*Panter et al, Agronomy 2017, 7(2), 36; https://doi.org/10.3390/agronomy7020036**)** using the teachings of Panter 2017 (Agronomy 7 (2) 36); or
3. if one intends to drive the appearance of yellowishyelowish, redish colours naturally occuring from the accummulation carotenoids in the mesocarp of avocado fruit one could use the type 3 metallothionein-like gene promoter, MT3-A and the teachings of patent US7732669, or
4. if one intends to promote the antioxidant properties properties or flavours and organoleptic characteristics of brussel sprouts by introducing new carotenoids on them, one can use the teachings of patent US20180195081 and the plastocyanin promoter of *Pisum sativum* to drive phytoene production on the tissue of interest.

Examples of suitable promoters able to drive expression in chlorophyll containing tissues further include but are not restricted to; pea rbc-3A (Gilmarin and Chua, 1990), arabidopsis CAB2 (Carre and Kay. 1995), ST-LS1 of *Solanum Tuberosum* (Dupre 1991, Plant J. 1,115) or alfalfa RAc (Potenza, 2004), or derivatives of such promoters.

Preferably, the promoter is a specific and strong constitutive promoter.

Preferably, the expression vector may be present in the leaves in 1 to 5, 20, 100 or 500 copies.

A control sequence may also comprise a transcription terminator, which is recognized by the cells of the leaf vegetables or forage crops to terminate transcription. The terminator is operably linked to the 3'-terminus of the gene. Any terminator that is functional in the cells of the leaf vegetables or forage crops may be used in the present invention.

The host cells of the leaf vegetables, green fruits or forage crops of the fourth aspect, may be transformed, transfected or transduced in a transient or stable manner. The recombinant plants of the invention may be obtained by any method known by the skilled person, such as electroporation, conjugation, transduction, competent cell transformation, protoplast transformation, protoplast fusion, biolistic "gene gun" transformation, PEG-mediated transformation, lipid-assisted transformation or transfection, chemically mediated transfection, lithium acetate -mediated transformation or liposome-mediated transformation.

The term "recombinant or modified plant" as used herein encompasses the modified leaf vegetables, green fruits or forage crops as well as any progeny that is not identical to the parent leaf vegetables or forage crops, in particular due to mutations that occur during replication.

To enhance the production of phytoene, the modified plant of the fourth aspect of the invention or of the methods of any of aspects one to three, may also be genetically modified in order to increase the production of geranylgeranyl diphosphate (GGPP). In particular, the modified leaf vegetables or forage crops of the invention may be genetically modified to increase the carbon flux to isopentenyl pyrophosphate (IPP) and dimethylallyl pyrophosphate (DMAPP) and/or to increase the conversion of IPP and DMAPP to geranylgeranyl diphosphate (GGPP). The carbon flux to IPP and DMAPP may be increased by enhancing the 2-C- methyl-D-erythritol 4-phosphate/I-deoxy-D-xylulose 5-phosphate (MEP/DXP) pathway. As used herein, the term "MEP pathway" or "MEP/DXP pathway" refers to the biosynthetic pathway leading to the formation of IPP and DMAPP from the condensation of pyruvate and D-glyceraldehyde 3 - phosphate to 1 -deoxy-D-xylulose 5- phosphate (DXP). This pathway involves the following enzymes: 1 -deoxy-D-xylulose 5- phosphate synthase (EC 2.2.1.7), 1-deoxy-D-xylulose 5-phosphate reductoisomerase (EC 1.1.1.267), 2-C-methyl-D-erythritol 4-phosphate cytidylyltransferase (EC 2.7.7.60), 4- diphosphocytidyl-2-C-methyl-D-erythritol kinase (EC 2.7.1.148), 2-C-methyl-D-erythritol 2,4-cyclodiphosphate synthase (EC 4.6.1.12), 4-hydroxy-3-methylbut-2-en-I- yl diphosphate synthase (EC 1.17.7.1), 4-hydroxy-3-methylbut-2-enyl diphosphate reductase (EC 1.17.1.2), and isopentenyl-diphosphate delta-isomerase (EC 5.3.3.2).

This pathway may be enhanced by any method known by the skilled person, for example by a method described in the patent application WO 2015/189428. In particular, this pathway may be enhanced by increasing at least one enzymatic activity selected from the group consisting of DXP synthase (DXS), DXP reductoisomerase (DXR), IspD, IspE, IspF, IspG, IspH and IPP isomerase activities (IDI), preferably by increasing at least DXP synthase and IPP isomerase activities.

An enzymatic activity (e.g. DXS, DXR, IspD, IspE, IspF, IspG, IspH, IDI or FPPS activity) may be increased as detailed above for the phytoene synthase activity, i.e. by overexpression of an endogenous gene or expression of a heterologous gene, and/or expression of an improved variant of the endogenous enzyme.

The term "DXS" or "DXP synthase" refers to the enzyme 1-deoxy-D-xylulose 5- phosphate synthase (EC 2.2.1.7) encoded by the dxs gene which catalyzes the condensation of pyruvate and D-glyceraldehyde 3-phosphate to 1-deoxy-D-xylulose 5- phosphate (DXP). The names of gene product, "DXP synthase", "DXS" or "DXPS", are used interchangeably in this application. The DXP synthase activity can be determined by using a radiolabeled substrate as described by Lois et al. (1998) or any other method known by the skilled person. The term "DXP reductoisomerase" or "DXR" refers to the enzyme 1-deoxy-D-xylulose 5-phosphate reductoisomerase (EC 1.1.1.267) encoded by the dxr gene. The term "IspD" refers to the enzyme 2-C-methyl-D-erythritol 4-phosphate cytidylyltransferase (EC 2.7.7.60) encoded by the ispD gene. The term "IspE" refers to the enzyme 4-diphosphocytidyl-2-C-methyl-D-erythritol kinase, (EC 2.7.1.148) encoded by the ispE gene. The term "IspF" refers to the enzyme 2-C-methyl-D-erythritol 2,4- cyclodiphosphate synthase (EC 4.6.1.12) encoded by the ispF gene. The term "IspG" refers to the enzyme 4-hydroxy-3-methylbut-2-en-I-yl diphosphate synthase (EC 1.17.7.1) encoded by the ispG gene. The term "IspH" refers to the enzyme 4-hydroxy-3-methylbut-2-enyl diphosphate reductase, also named hydroxymethylbutenyl pyrophosphate reductase, (EC 1.17.1.2) encoded by the ispH gene. The term "IDI", "IPP isomerase" or "isopentenyl pyrophosphate isomerase" refers to the enzyme isopentenyl- diphosphate delta-isomerase (EC 5.3.3.2) encoded by the idi gene that catalyzes the 1,3- allylic rearrangement of the homoallylic substrate isopentenyl (IPP) to its allylic isomer, dimethylallyl diphosphate (DMAPP). According to the organism, the nomenclature of the above identified enzymes and encoding genes may vary. However, for the sake of clarity, in the present specification, these terms are used independently from the origin of the enzymes or genes.

Preferably, at least one gene selected from the group consisting of dxs, dxr, ispD, ispE, ispF, ispG, ispH and idi genes, is overexpressed, more preferably at least dxs and/or idi genes, and even more preferably at least dxs and idi genes are overexpressed.

In an embodiment, said composition of the fourth aspect of the invention or of any of its preferred embodiments, is a cosmetic or pharmaceutical composition. In another embodiment, said composition is a nutraceutical or nutricosmetic composition, or a food or feed additive.

As used herein, the term "nutraceutical composition" refers to a composition comprising nutrients isolated or purified from food and having a beneficial effect on the health of the consumer. As used herein, the term "nutricosmetic composition" refers to a composition comprising nutritional oral ingredients and which is formulated and marketed specifically for beauty purposes.

In a particular embodiment, the cosmetic or pharmaceutical composition is a skin whitening, lightening or bleaching composition or a composition to prevent aging, oxidative or photo-oxidative damages.

Phytoene was also known to show anticarcinogenic and anti-inflammatory properties. Thus, in an embodiment, the composition is a pharmaceutical composition to be used in the treatment of cancer or in the treatment of inflammatory disorders.

Preferably, the composition of the invention is to be administered by topical or oral route.

The composition of the of the fourth aspect of the invention or of any of its preferred embodiments may obviously, depending on its use, comprise also other ingredients, such as cosmetic or pharmaceutical acceptable carriers, preservatives, antioxidants such as carotenoids, as well as pharmaceutically or cosmetically active ingredients. In a preferred embodiment, the composition further comprises a hydrophobic carrier, which may be selected from oils typically used in the cosmetic, pharmaceutical or food industry, such as vegetable, mineral or synthetic oils.

Further aspects and advantages of the present invention will be described in the following examples, which should be regarded as illustrative and not limiting.

### EXAMPLES

### Plant material and growth conditions

*N. tabacum* (Xanthi nc) (Li and Carrington 1995), *N. benthamiana* (RDR6i) (Schwach et al. 2005), and *A. thaliana* (Columbia-0 and Landsberg *erecta*) plants were grown under standard conditions as described previously (Llorente et al. 2016; Majer et al. 2017). For generating dark-induced leaf senescence, detached leaves were maintained inside dark, humid chambers until visible yellowing occurred. Collected plant material was frozen in liquid nitrogen, lyophilized, and then homogenized to a fine powder using a TissueLyser system (Qiagen) for further analyses.

### Gene constructs and transient expression assays

The viral vectors used in this study have been described (Majer et al. 2017), except for a plasmid with recombinant clone TEV-GFP, which was also constructed from plasmid pGTEVa (Bedoya et al. 2012). To generate the different crtB versions, we amplified by PCR the *Pantoea ananatis crtB* gene from plasmid pACCRT-EIB (Cunningham et al. 1993) and the plastid-targeting sequence of the hydroxymethylbutenyl 4-diphosphate synthase gene from *A. thaliana* cDNA (Gas et al. 2009). We also amplified the cDNA sequences encoding AtPSY from *A*. *thaliana* and SIPSY2 from *S*. *lycopersicum.* All primers used for cloning procedures are detailed in Table 2.

**Table 2. Primers used for cloning procedures**

| **Name** | **Sequence** |
|---|---|
| PacrtB_N-tag_attB1-F | 5´- GGGGACAAGTTTGTACAAAAAAGCAGGCTGGATGAATAATCCG TCGTTACTCAATC-3´ |
| PacrtB_C-tag_attB2-R | 5´- GGGGACCACTTTGTACAAGAAAGCTGGGTCTAGAGCGGGCGC TGCCAGAGATG-3´ |
| PacrtB_STOP_attB2-R | 5´- GGGGACCACTTTGTACAAGAAAGCTGGGTCTAGAGCGGGCGC TGCCAGAGATG-3´ |
| AtHDS_N-tag_attB1-F | 5´- GGGGACAAGTTTGTACAAAAAAGCAGGCTTCATGGCGACTGGA GTATTGCCAGCTC-3´ |
| crtB-5'-AtHDS-3'-R | 5´- GATTGAGTAACGACGGATTATTCATATTCCGGATAACCGAAACT CTTCTC-3´ |
| AtPSY_N-tag_attB1-F | 5´- GGGGACAAGTTTGTACAAAAAAGCAGGCTTCATGTCAAGCTTA GTAGCAAGTCCTTC-3´ |
| AtPSY_STOP_attB2-R | 5´- GGGGACCACTTTGTACAAGAAAGCTGGGTTCATATCGATAGTC |
| | TTGAACTTGAAG-3′ |
| SIPSY2_N-tag_attB1-F | 5´- GGGGACAAGTTTGTACAAAAAAGCAGGCTATGTCTGTTGCTTT GTTGTGGGTTG-3´ |
| SIPSY2_STOP_attB2-R | 5´- GGGGACCACTTTGTACAAGAAAGCTGGGTTCATGTCTTTGCTA GTGGGGAAGAAG-3´ |
| PacrtE_N-tag_attB1-F | 5´- GGGGACAAGTTTGTACAAAAAAGCAGGCTGGATGACGGTCTGC GCAAAAAAACAC-3´ |
| PacrtE_STOP_attB2-R | 5´- GGGGACCACTTTGTACAAGAAAGCTGGGTTTATACTGACGGCA GCGAGTTTTTTGTC-3´ |

PCR products were cloned using the Gateway system first into plasmid pDONR-207 and then into plasmid pGWB405 (Nakagawa et al. 2007) to generate 35S:crtB-pGWB405, 35S:(p)crtB-pGWB405, 35S:(p)crtB-GFP-pGWB405, 35S:AtPSY-pGWB405, and 35S:SIPSY2-pGWB405 or into plasmid pGWB506 (Nakagawa et al. 2007) to generate 35S:GFP-crtB-pGWB506.

For transient expression studies using viral vectors, leaves of 4 to 6 week-old *N. tabacum* and *A. thaliana* were mechanically inoculated with crude extracts from frozen-stored infected plant tissue and collected upon the appearance of the yellowing phenotype as described previously (Majer et al. 2017). For agroinfiltration experiments, leaves of 4 to 6 week-old *N. benthamiana* plants were infiltrated with *A. tumefaciens* strain GV3101 carrying plasmids of interest following the procedure described previously (Sparkes et al. 2006). Infiltration cultures were grown on LB medium at 28°C and used at an OD600 of 0.5, except for experiments performed with serial culture dilutions to express (p)crtB at different levels. For pharmacological inhibition of phytoene conversion, leaves were infiltrated with a 2 µM solution of norflurazon (NF) in water and 0.05 % Tween 20. The infiltration was performed next to leaf areas that had been agroinfiltrated with different constructs 24 h earlier. Infiltrated plants were returned to the same growth conditions after infiltration.

### Transmission electron microscopy

Transmission electron microscopy of plant leaves was performed as previously described (D'Andrea et al. 2018).

### Metabolite analyses

Carotenoids were analyzed as previously described (Llorente et al. 2016). Phytoene was quantified using a concentration curve with a commercial standard (Sigma). Lipids were extracted, separated by Thin Layer Chromatography (TLC), and methylated as described (Wang and Benning 2011). Primary metabolites were extracted as described previously (Lisec et al. 2006) using approximately 20 mg of lyophilized leaf tissue. Derivatization and gas chromatography-time of flight-mass spectrometry (GC-TOF-MS) analyses were carried out as described previously (Lisec et al. 2006). Metabolites were identified manually by TagFinder software using the reference library mass spectra and retention indices from the Golm Metabolome Database, http://gmd.mpimp-golm.mpg.de. The parameters used for the peak annotation of the 52 metabolites can be found in Table 3.

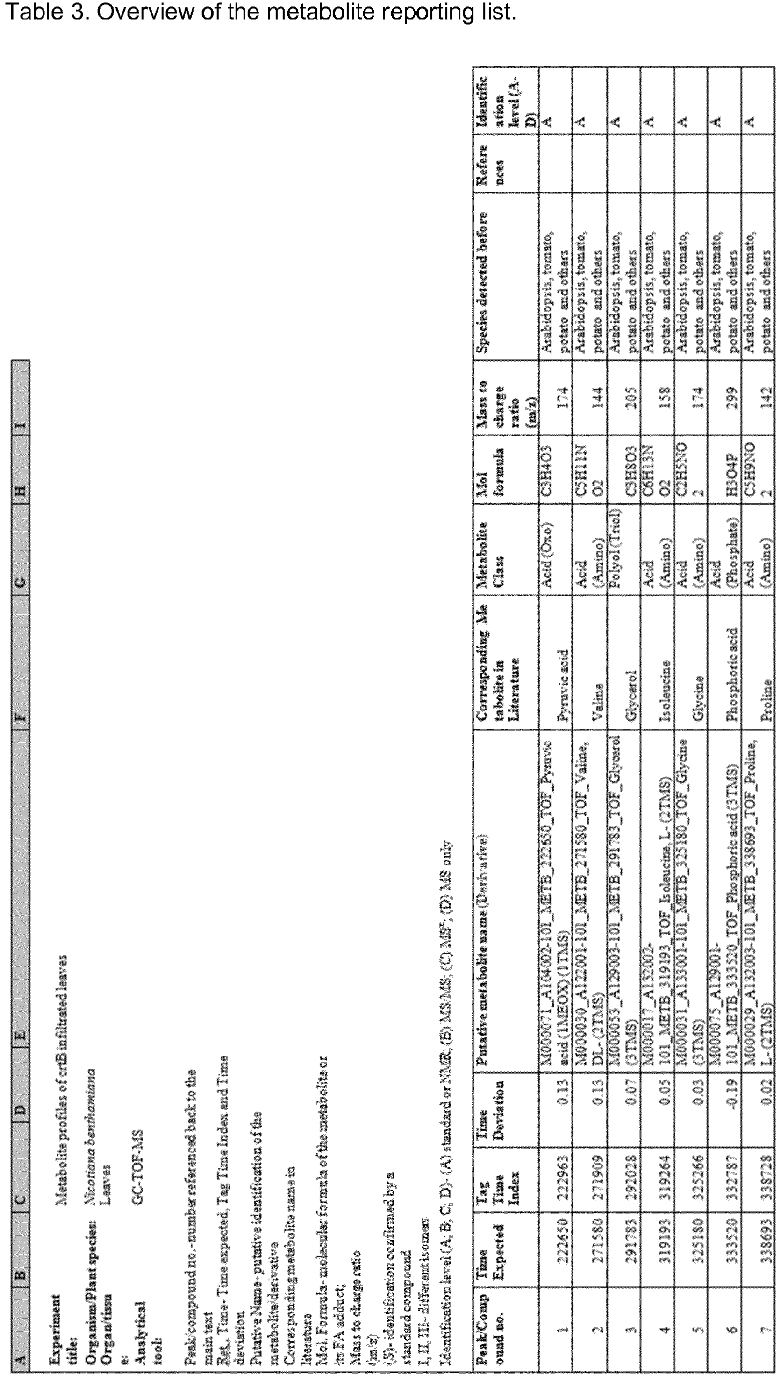

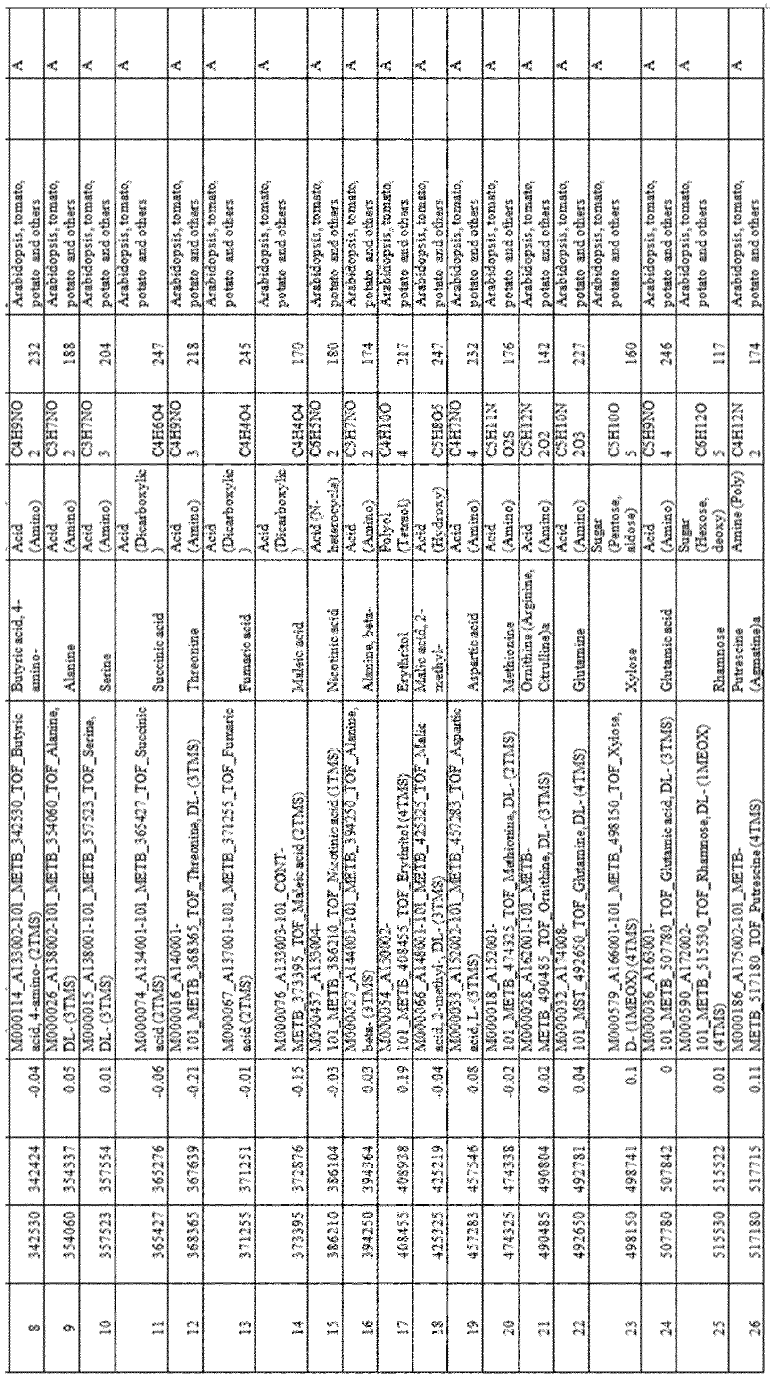

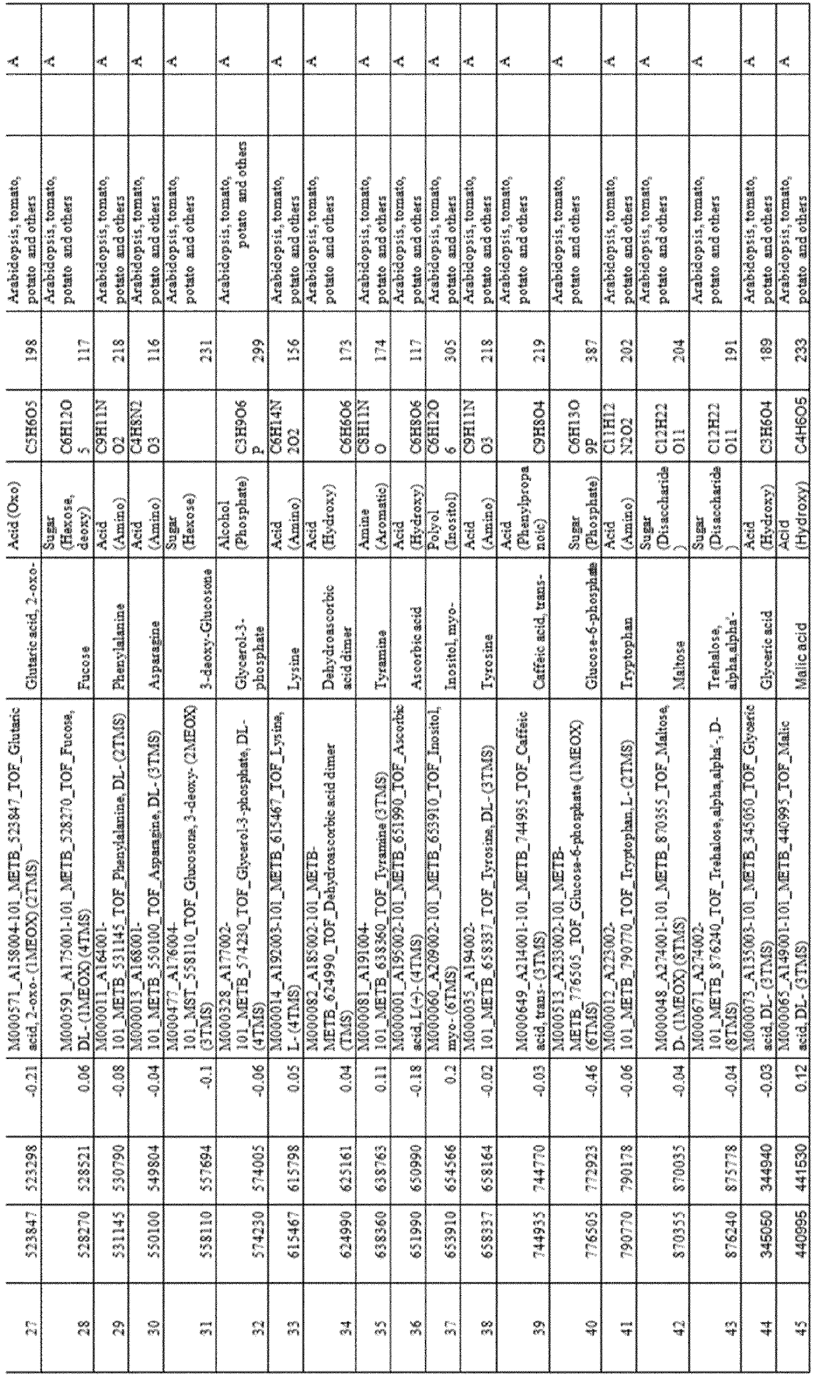

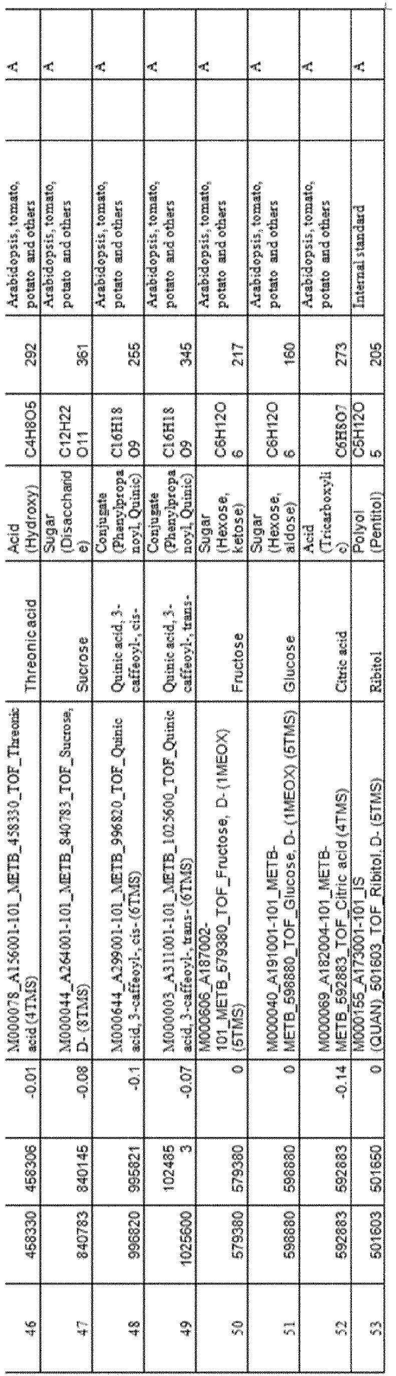

Data were normalized to the mean value of the GFP control samples (i.e. the value of all metabolites for GFP control samples was set to 1). The means and standard errors of five to six replicates at 96 hpi are presented in Table 4.

**Table 4. Relative metabolite levels in N. benthamiana leaf sections infiltrated with control GFP and (p)crtB constructs (for details see M&M section). Data is presented as means ± SE of five to six biological replicates normalized to the mean level of the control GFP samples. Asterisks denote significant differences (P < 0.05) to the GFP samples.**

| **Metabolite** | **GFP** | **crtB** |
|---|---|---|
| Pyruvate | 1±0.09 | 1.05±0.04 |
| Valine | 1±0.08 | 1.33±0.03 |
| Glycerol | 1±0.10 | 0.8±0.12 |
| Isoleucine | 1±0.11 | 1.38±0.1 |
| Glycine | 1±0.34 | 0.88±0.2 |
| Phosporic acid | 1±0.08 | 1.2±0.08 |
| Proline | 1±0.18 | 1.04±0.26 |
| GABA | 1±0.16 | 0.68±0.1 |
| Alanine | 1±0.15 | 1.41±0.49 |
| Serine | 1±0.14 | 1.01±0.11 |
| Succinate | 1±0.10 | 1.1±0.11 |
| Threonine | 1±0.06 | 1.24±0.11 |
| Fumarate | 1±0.11 | 0.85±0.07 |
| Maleate | 1±0.12 | 1.28±0.12 |
| Nicotinate | 1±0.07 | 1.08±0.1 |
| beta-alanine | 1±0.07 | 1.22±0.08 |
| Erythritol | 1±0.02 | 1.24±0.03 |
| 2-methyl-Malate | 1±0.04 | 1.09±0.04 |
| Aspartate | 1±0.03 | 1.35±0.02 |
| Methionine | 1±0.14 | 1.03±0.12 |
| Ornithine | 1±0.18 | 0.91±0.06 |
| Glutamine | 1±0.21 | 1.66±0.7 |
| Xylose | 1±0.09 | 1.13±0.04 |
| Glutamate | 1±0.15 | 1.55±0.17 |
| Rhamnose | 1±0.07 | 1.17±0.05 |
| Putrescine | 1±0.11 | 1.81±0.12 |
| 2-oxo-Glutarate | 1±0.13 | 1.42±0.11 |
| Fucose | 1±0.08 | 1.19±0.06 |
| Phenyalanine | 1±0.10 | 1.03±0.08 |
| Asparagine | 1±0.16 | 1.43±0.18 |
| 3-deoxy-Glucosone | 1±0.08 | 0.87±0.04 |
| Glycerol-3-P | 1±0.10 | 0.89±0.08 |
| Lysine | 1±0.15 | 0.99±0.05 |
| Dehydroascorbate | 1±0.11 | 0.7±0.08 |
| Tyramine | 1±0.08 | 1.02±0.05 |
| Ascorbate | 1±0.02 | 0.77±0.07 |
| myo-Inositol | 1±0.07 | 0.83±0.1 |
| Tyrosine | 1±0.13 | 1.53±0.15 |
| trans-Caffeate | 1±0.32 | 1.44±0.06 |
| Glucose-6-P | 1±0.06 | 1.1±0.04 |
| Tryptophan | 1±0.28 | 2.1±0.51 |
| Maltose | 1±0.10 | 0.92±0.09 |
| Trehalose | 1±0.09 | 0.95±0.07 |
| Glycerate | 1±0.16 | 0.72±0.11 |
| Malate | 1±0.09 | 1.33±0.1 |
| Threonate | 1±0.11 | 1.24±0.09 |
| Sucrose | 1±0.11 | 1.14±0.06 |
| Quinic acid, 3-caffeoyl-, cis- | 1±0.07 | 1.07±0.07 |
| Quinic acid, 3-caffeoyl-, trans- | 1±0.08 | 1.02±0.06 |
| Fructose | 1±0.10 | 0.56±0.04 |
| Glucose | 1±0.06 | 0.74±0.07 |
| Citrate | 1±0.09 | 2.28±0.31 |

### Transcript analyses

Total RNA was extracted from leaves with the Maxwell 16 LEV Plant RNA Kit (Promega) and quantified with a NanoDrop (Thermo Scientific) as described (Majer et al. 2017). For reverse transcription-quantitative PCR (RT-qPCR) analyses, the First Strand cDNA Synthesis Kit (Roche) was used to generate cDNA according to the manufacturer's instructions, with anchored oligo(dT)₁₈ primers and 1 µg of total RNA. Relative mRNA abundance was evaluated via quantitative PCR using LightCycler 480 SYBR Green I Master Mix (Roche) on a LightCycler 480 real-time PCR system (Roche). Gene expression analysis of *N. tabacum SAG12* gene was conducted using primers NtSAG12_qPCR-F (5'-A T T C A T G G G G C A G T A A A T G G-3') (SEQ ID NO 5) and NtSAG12_qPCR-R (5'-G A A G C G T C C A T A G C A A G T C C-3') (SEQ ID NO 6) and the L25 ribosomal protein gene for normalization (Schmidt and Delaney 2010) using primers NtRPL25_qPCR-F (5'-C A A G G C A C A G G C A G C T A A G G-3') (SEQ ID NO 7) and NtRPL25_qPCR-R (5'-A G G T C G G T G G A A T G T A A C T T T T G-3') (SEQ ID NO 8).

RNAseq service was performed by Sequentia Biotech SL (Barcelona, Spain). RNA concentration in each sample was assayed with a ND-1000 spectrophotometer (NanoDrop) and its quality assessed with the TapeStation 4200 (Agilent Technologies). Indexed libraries were prepared from 1 µg/ea purified RNA with TruSeq Stranded mRNA Sample Prep Kit (Illumina) according to the manufacturer's instructions. Libraries were quantified using the TapeStation 4200 and pooled such that each index-tagged sample was present in equimolar amounts, with final concentration of the pooled samples of 2 nM. The pooled samples were subject to cluster generation and sequencing using an Illumina NextSeq 500 System (Illumina) in a 2x75 paired end format at a final concentration of 1.8 pmol. The raw sequence files generated (.fastq files) underwent quality control analysis using FastQC (http://www.bioinformatics.babraham.ac.uk/projects/fastqc/). Data analysis was performed with the online platform AIR (www.transcriptomics.cloud) (Vara et al. 2019) using the SolGenomics Network (https://solgenomics.net/) *N. benthamiana* 1.01 (Niben v101) reference genome.

### Protein extraction and immunoblot analyses

Protein extraction, quantification, and immunoblot analyses were performed as described (Pulido et al. 2016) using anti-fibrillin (Simkin et al. 2007) or anti-PsbA serum (Agrisera). Thylakoid membrane isolation was performed as described (Russell et al. 1995).

### Photosynthetic measurements

Photosynthetic efficiencies were assessed by measuring chlorophyll a fluorescence with a MAXI-PAM fluorometer (Heinz Walz GmbH). Leaves previously agroinfiltrated were placed under the camera and effective quantum yield (ΔF/Fm') was measured as (Fm'-Fs)/ Fm', where Fm' and Fs are, respectively, the maximum and the minimum fluorescence of light exposed plants. The actinic light (AL) chosen was 21 PAR as the last value of light able to generate a response in the p(crtB) infiltrated part before having a null photosynthetic activity. Each value is the average result of three biological replicates and three different AOI for each replicate.

NPQ was also measured using the MAXI-PAM unit. All recordings were performed every day at the same time slot, but the order of the samples was randomized in order to reduce the bias related to the length of the NPQ protocol. Plants were dark-adapted for 30 min before measurement and then submitted to a continuous AL of 801 PAR for 10 min. After this period leaves were left recovering in darkness for 40 min. During the whole protocol, Fs and Fm' values were registered every 60 seconds. NPQ and its relative components qE, qZ and ql were calculated as described (Coate et al. 2013) with some modifications. Briefly: NPQ was calculated as (Fm-Fm0)/Fm, The relative contributions of qE, qZ and ql to NPQmax were estimated by monitoring NPQ relaxation kinetics in the dark: following the 10 min exposure to saturating light used to measure NPQ, leaves were left in darkness, and Fm0 was measured again after 10 and 40 min when the Ft value was appearing stable and not recovering anymore. The qE component of NPQ relaxes within 10 min of a leaf being placed in darkness such that NPQ persisting after 10 min in the dark consists of qZ + ql. The qZ component of NPQ relaxes within tens of minutes so that NPQ persisting after 40 min in the dark consists of ql, which is either irreversible in the dark or requires several hours to relax. Consequently, (ql+qZ) was calculated as (Fm-Fm1)/Fm1, where Fm1 is the value of Fm measured after 10 min in the dark following NPQmax measurement. qZ was calculated as (Fm-Fm2)/Fm2, where Fm2 is the value of Fm measured after 40 min in the dark following measurement of NPQmax. qE was calculated as NPQmax-(ql+qZ) and ql was calculated as (ql+qZ)-qZ.

For the calculation of the de-epoxidation state (DES), agroinfiltrated leaf areas were exposed for 10 min to a continuous AL of 801 PAR in the MAXI-PAM unit, sampled under the same light and immediately frozen before pigment extraction and quantification. The operation of the xanthophyll cycle, comprising the sequential de-epoxidation of the pigments violaxanthin (Vx) to antheraxanthin (Ax) and zeaxanthin (Zx) was followed by calculating DES as (Zx+0.5×Ax)/(Zx+Ax+Vx), where Zx, Ax and Vx are the concentrations of the corresponding xanthophylls.

### Respiration measurements

Before respiration measurements, *N. benthamiana* plants were placed in the dark for about 30 min to avoid light-enhanced dark respiration. Four leaf discs of 3.8 cm² each were harvested from leaf sections of two different plants infiltrated with the (p)crtB construct, weighted and placed into the respiration cuvette containing the respiration buffer (30 mM MES pH 6.2, 0.2 mM CaCl₂). Oxygen uptake rates were measured in darkness using a liquid-phase Clark-type oxygen electrode (Rank Brothers) at a constant temperature of 25°C. Dry weights from leaf discs were determined after drying for 2 days at 60°C.

### Statistical analyses

Differentially expressed genes (DEGs) were identified by comparing crtB and GFP RNAseq datasets with the DESeq2 statistical method in the AIR platform. The resulting crtB/GFP list was filtered using cut-offs of FDR <0.05 and log2-transformed fold change, log2(FC), >0.585 for upregulated genes and <-0.599 for downregulated genes. Gene ontology enrichments were identified by the Parametric Analysis of Gene Set Enrichment (PAGE) function of the AgriGO v2.0 web-based tool (http://bioinfo.cau.edu.cn/agriGO/) after transforming the gene IDs to the Niben v04.4 annotation. For the comparison of different biological systems, we selected the significantly enriched gene ontologies from our *N. benthamiana* RNAseq experiment (p and q values <0.05) and compared their Z-score values with those obtained from the analysis of published RNAseq data of tomato fruit ripening and Arabidopsis leaf senescence. In particular, we used the RPM values of the Total Pericarp MG, LR and RR ripening stages (Shinozaki et al. 2018) and the FPKM values of the 16D and 30D senescence stages (Woo et al. 2016). DEGs resulting from LR/MG, RR/MG and 30D/16D comparisons were filtered as described above for *N. benthamiana* crtB/GFP.

Student's t-tests were used for statistical analyses in metabolite profiling and respiration analyses. All other statistical analyses were performed using GraphPad Prism 5.0a (GraphPad Software).

### Example 1. The bacterial crtB enzyme induces the transformation of chloroplasts into chromoplasts when delivered to leaf cells (chlorophyll containing cells).

The first step specific of the carotenoid pathway is the conversion of geranylgeranyl diphosphate (GGPP) to phytoene, catalyzed by phytoene synthase (referred to as PSY in plants and crtB in bacteria). Desaturation and isomerization reactions then transform the non-colored phytoene molecule into lycopene, a red carotenoid that is later metabolized to orange-colored beta-carotene (pro-vitamin A) and derived yellowish xanthophylls such as violaxanthin and neoxanthin in one branch of the pathway. A second branch converts lycopene into lutein, a different xanthophyll. In plants, these reactions take place only in plastids (Rodriguez-Concepcion et al. 2018). We previously found that the virus-mediated expression of a bacterial *crtB* gene in tobacco (*Nicotiana tabacum*), tomato (*Solanum lycopersicum*), *Arabidopsis thaliana* and many other plants caused leaf yellowing (Majer et al. 2017). This phenotype was deduced to result from a combination of virus-induced chlorosis and increased accumulation of colored carotenoids downstream of phytoene. Besides typical chloroplast carotenoids such as lutein, beta-carotene, violaxanthin and neoxanthin, leaves expressing *crtB* accumulated lycopene (Majer et al. 2017). Lycopene is abundant in chromoplasts-containing organs such as the ripe fruits of tomato, but it is not normally found in leaf chloroplasts, where it is readily converted into downstream carotenoids. To test whether plastid ultrastructure was changing to accommodate a new carotenoid profile, we performed transmission electron microscopy (TEM) analysis of tobacco leaves infected with crtB-producing viruses. TEM revealed plastids with remodeled internal structures of distinctive chromoplast morphology (Fig. 1A). These plastids were devoid of organized photosynthetic thylakoid membranes and grana but contained tightly packed membrane stacks and a proliferation of small electron-dense (i.e. lipid-containing) plastoglobules typically observed in chromoplasts, where they contribute to carotenoid storage and stability (Fraser et al. 2007; Egea et al. 2010; Jeffery et al. 2012; Lado et al. 2015). Very similar structures were observed in the chromoplast-like plastids that developed when the *crtB* gene was expressed in virus-infected *Arabidopsis* leaves (Fig. 1B) but also when transiently expressed in agroinfiltrated *Nicotiana benthamiana* leaves (Fig. 1C).

To further substantiate the identity of the plastids that developed in crtB-producing leaves, we analyzed the level of chloroplast and chromoplast marker proteins by immunoblot analysis. Virus-infected or Agrobacterium-mediated expression of *crtB* in *N. tabacum* or *N. benthamiana* leaves, respectively, resulted in increased levels of fibrillin (Fig. 1D), a protein associated to carotenoid accumulation and chromoplast development (Deruere et al. 1994; Singh and McNellis 2011). In contrast, the levels of D1 (also known as PsbA), a core component of photosystem II (PSII) crucial for the photosynthetic electron transport chain that is highly downregulated during chloroplast to chromoplast differentiation (Barsan et al. 2012; Kahlau and Bock 2008), decreased in crtB-producing leaves (Fig. 1D). The main photosynthetic membrane lipid of chloroplasts, monogalactosyl diacylglycerol (MGDG), was also decreased in leaves containing chromoplast-like plastids (Fig. 9A).

Unlike that observed with viral vectors in *N. tabacum* (Majer et al. 2017), Agrobacterium-mediated expression of *crtB* in *N. benthamiana* did not reduce chlorophyll levels compared to control leaf tissues agroinfiltrated with GFP (Fig. 9B). This result indicates that the chlorosis associated with virus infection is not necessary for chloroplasts to be transformed into chromoplasts-like plastids and supports the conclusion that the crtB-triggered leaf yellowing phenotype is not a senescence process, which typically leads to chlorophyll loss. TEM examination of dark-induced senescent *N. benthamiana* leaves confirmed that the plastids found in crtB-producing cells were completely different from the gerontoplasts with very large and electron-lucent plastoglobules that develop in senescent leaf cells (Fig. 1E). Low expression of the senescence marker gene *SAG12* (Gan and Amasino 1995) in virus-infected *crtB*-expressing tobacco leaves further argued against the possibility of a senescence process (Fig. 9C). **These results together suggest that expressing the bacterial *crtB* gene in leaf cells is sufficient to differentiate chloroplasts into plastids of chromoplast phenotype.** Arabidopsis double mutants defective in OR chaperones (AtOR and AtOR-like) (Zhou et al. 2015) showed the characteristic yellow phenotype associated with carotenoid overaccumulation after infection with crtB-containing viral vectors (Fig. 9D). This result suggests that OR activity is not required for the crtB-mediated differentiation of leaf chloroplasts into chromoplasts.

### Example 2. The crtB enzyme only triggers chloroplast-to-chromoplast differentiation when localized in plastids

Consistent with the lack of natural or predicted plastid-targeting signals in crtB, a C-terminal fusion to the green fluorescent protein (GFP) resulted in the localization of the crtB-GFP protein to the cytosol in *N. benthamiana* leaf cells (Majer et al. 2017). It could not be excluded, however, that part of the crtB-GFP protein could localize to chloroplasts. By using *N*. *benthamiana* line RDR6i, in which gene silencing is impaired (Schwach et al. 2005), we could confirm that the crtB-GFP protein was present in both cytosol and chloroplasts of agroinfiltrated cells (Fig. 2). When GFP was fused to the N-terminus of crtB, the resulting protein (GFP-crtB) was completely excluded from chloroplasts (Fig. 2). It is therefore likely that the bacterial crtB enzyme harbors a cryptic plastid-targeting signal in its N-terminus that becomes blocked and hence inactivated in the GFP-crtB protein. To unambiguously target crtB to the chloroplast, we next added the plastid-targeting sequence of the Arabidopsis MEP pathway enzyme HDS (Gas et al. 2009) to the crtB-GFP reporter. As expected, the resulting (p)crtB-GFP protein was only found in chloroplasts (Fig. 2). Agroinfiltrated leaf tissues either expressing crtB-GFP or the plastidic (p)crtB-GFP version developed the characteristic yellow phenotype, whereas tissues expressing the cytosolic GFP-crtB version (renamed as (c)crtB) remained as green as the control expressing GFP (Fig. 3A) or a plastid-targeted version of GFP (Fig. 10). Analysis of carotenoid and chlorophyll contents showed identical profiles for leaf sections agroinfiltrated with GFP and (c)crtB and confirmed that, similary to crtB, (p)crtB was able to trigger carotenoid overaccumulation without changing chlorophyll levels (Fig. 3B). Despite unchanged chlorophyll contents, estimation of photosynthesis-related parameters such as effective quantum yield at PSII (φPSII) and non-photochemical quenching (NPQ) showed that both crtB and (p)crtB, but not (c)crtB, had a dramatic impact on chloroplast function (Fig. 3C). The fact that plastid targeting of GFP did not cause any φPSII defect (Fig. 10) confirms that disturbance of chloroplast photosynthesis is not caused by the accumulation of a foreign protein but specifically by crtB. TEM analyses confirmed that (p)crtB induced the differentiation of chromoplast-like plastids very similar to those found in leaf tissues expressing the untargeted crtB enzyme, whereas only chloroplasts were present in leaves producing either (c)crtB or GFP (Fig. 1D). **These results confirm that crtB activity inside chloroplasts elicits a non-natural (i.e. artificial) differentiation of chromoplasts.**

### Example 3. Artificial chromoplast biogenesis induces profound changes in nuclear gene expression and primary cell metabolism

The vast majority of plastidial proteins are encoded by chromosomal genes (Jarvis and Lopez-Juez 2013). We therefore reasoned that the dramatic remodeling of plastidial ultrastructure associated with crtB-triggered chromoplast differentiation would require changes in nuclear gene expression. Transcriptomic (i.e., RNA-seq) analyses of *N. benthamiana* leaf sections at 5 days post-infiltration (dpi) with Agrobacterium cultures to produce (p)crtB or GFP showed that about 5.000 genes were differentially expressed in the two samples.

Such a massive reprogramming of gene expression included the upregulation of 3.183 genes as well as the downregulation of 1,803 genes. Gene Ontology (GO) term enrichment analyses (Table 7) showed overrepresentation of genes involved in protein folding and binding to RNA and ribosomes among those induced by (p)crtB (Fig. 5).

**Table 5. Parametric Analysis of Gene Set Enrichment (PAGE) by AgriGO. p value ≤ 0,05 ; q value ≤ 0,05**

| **Cellular Component** | | | | | | | | |
|---|---|---|---|---|---|---|---|---|
| ***Nicotiana benthamiana*** | | | | | | **Tomato Fruit Ripening*** | | **Senesc ence* *** |
| **crtB vs GFP** | | | | | | **OR vs MG** | **RR vs MG** | **30D vs 16D** |
| **GO Accession** | **Description** | **number of genes** | **Z-score** | **p value** | **q value** | **Z-score** | **Z-score** | **Z-score** |
| GO:0005623 | cell | 349 | 3.5 | 0.00054 | 0.0013 | -1.2 | -0.25 | 0.22 |
| GO:0044464 | cell part | 349 | 3.5 | 0.00054 | 0.0013 | -1.2 | -0.25 | 0.22 |
| GO:0005737 | cytoplasm | 138 | 5.9 | 4.7E-09 | 0.00000015 | 1.3 | 2.2 | -1 |
| GO:0044444 | cytoplasmic part | 110 | 5.3 | 0.0000001 | 0.0000017 | 1.8 | 3.1 | -1.9 |
| GO:0019898 | extrinsic to membrane | 13 | 3.2 | 0.0012 | 0.0027 | #N/A | #N/A | -0.41 |
| GO:0005622 | intracellular | 222 | 4.4 | 0.00001 | 0.000048 | -0.18 | 0.66 | -0.19 |
| GO:0043232 | intracellular non-membrane-bounded organelle | 86 | 4.8 | 0.0000013 | 0.000007 | 0.72 | 1.1 | -1.5 |
| GO:0043229 | intracellular organelle | 150 | 3.6 | 0.00033 | 0.00095 | 0.93 | 2.2 | -0.7 |
| GO:0044424 | intracellular part | 191 | 4.1 | 0.000045 | 0.00016 | 0.13 | 1.3 | -0.19 |
| GO:0032991 | macromolecular complex | 106 | 4.2 | 0.000026 | 0.0001 | 1 | 1.6 | -0.74 |
| GO:0043228 | non-membrane-bounded organelle | 86 | 4.8 | 0.0000013 | 0.000007 | 0.72 | 1.1 | -1.5 |
| GO:0043226 | organelle | 150 | 3.6 | 0.00033 | 0.00095 | 0.93 | 2.2 | -0.7 |
| GO:0030529 | ribonucleoprote in complex | 81 | 5.2 | 0.00000026 | 0.0000028 | 2.1 | 2.3 | -2.2 |
| GO:0005840 | ribosome | 76 | 5.1 | 0.00000039 | 0.0000032 | 2.1 | 2.2 | -3.6 |

An enrichment of genes with roles in transmembrane transport, cell signaling (protein phosphorylation, calcium binding), and nuclear gene expression (transcription factors) was observed among those repressed when chromoplast biogenesis was artificially induced in *N*. *benthamiana* leaves (Fig. 4). Consistent with our previous conclusions, this profile was strikingly similar to that of ripening tomato fruits (where chromoplasts naturally differentiate from chloroplasts) but very different from that of senescent Arabidopsis leaves (Fig. 4). An important difference, however, is that crtB-triggered chromoplastogenesis hardly involved changes in the expression of genes with roles in carotenoid biosynthesis, degradation, or storage (Fig. 11 and Table 8).

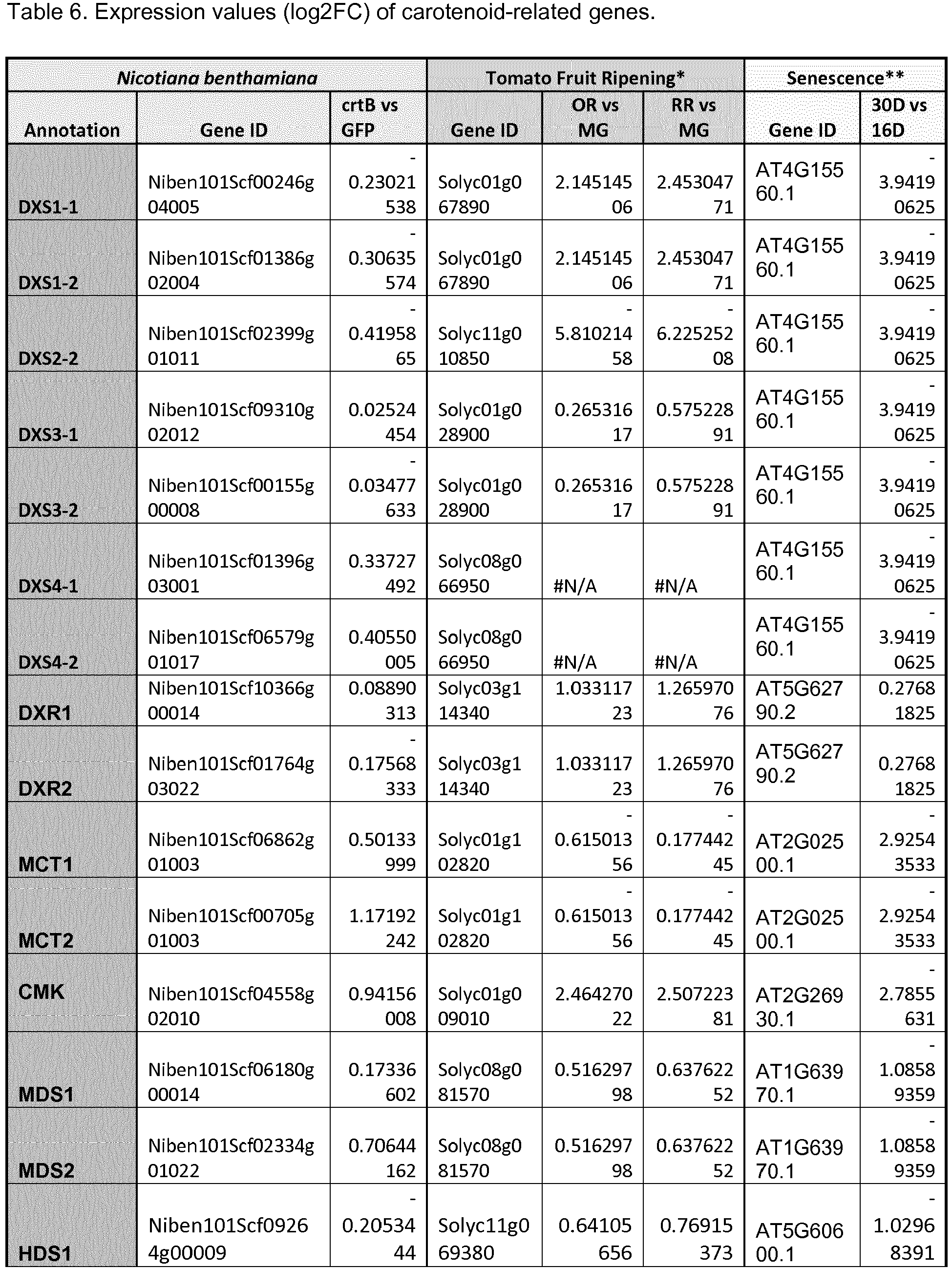

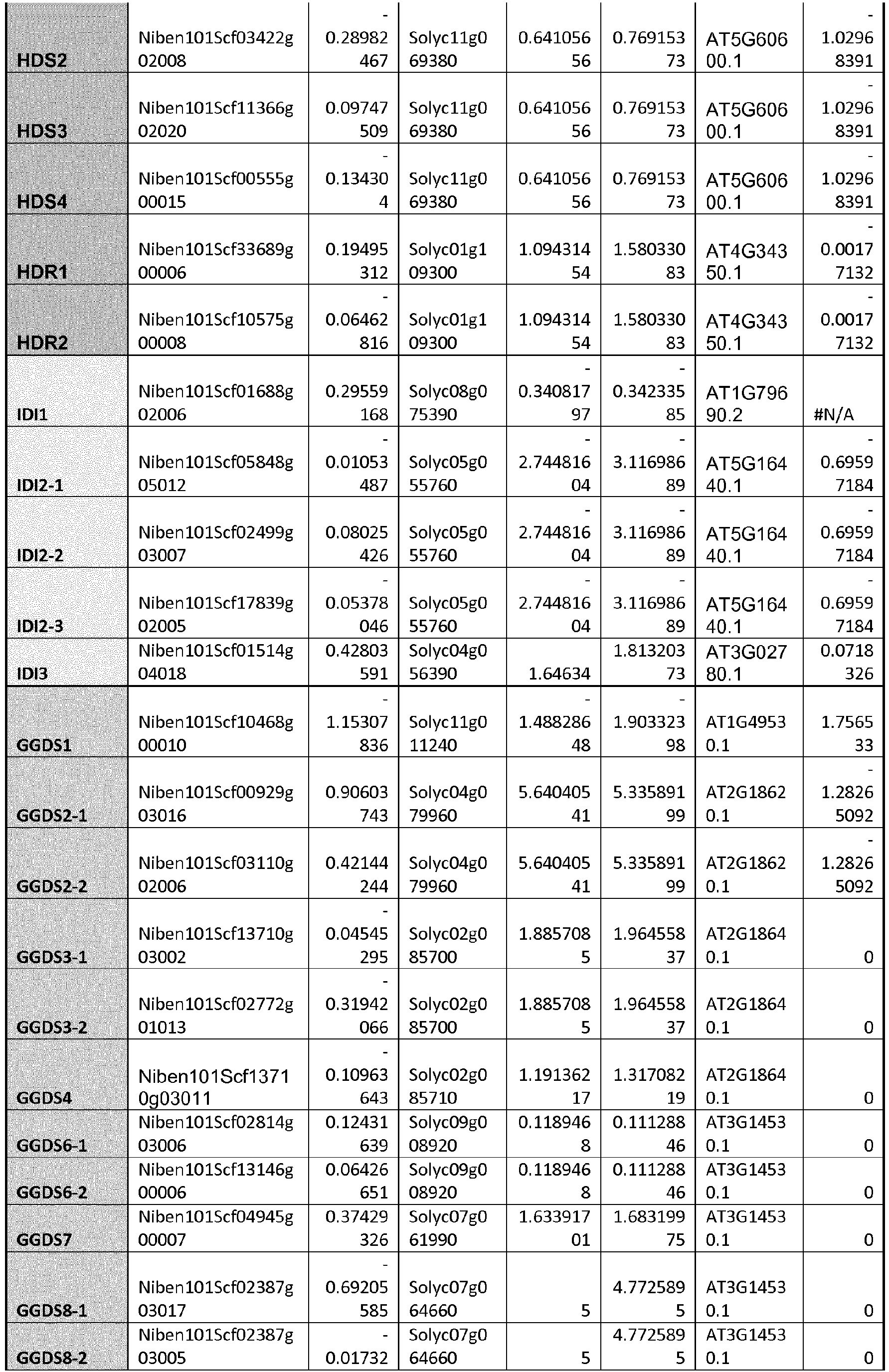

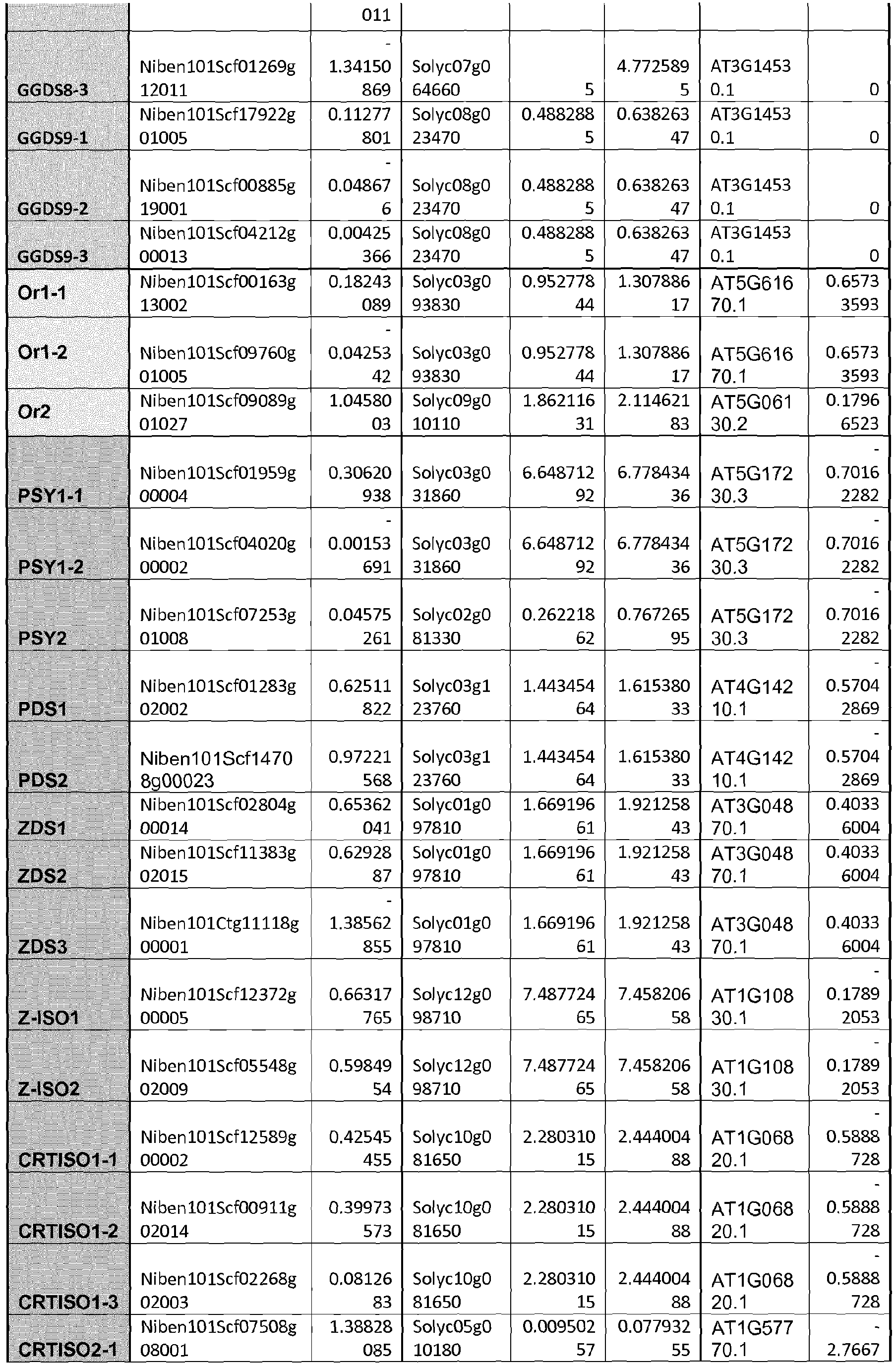

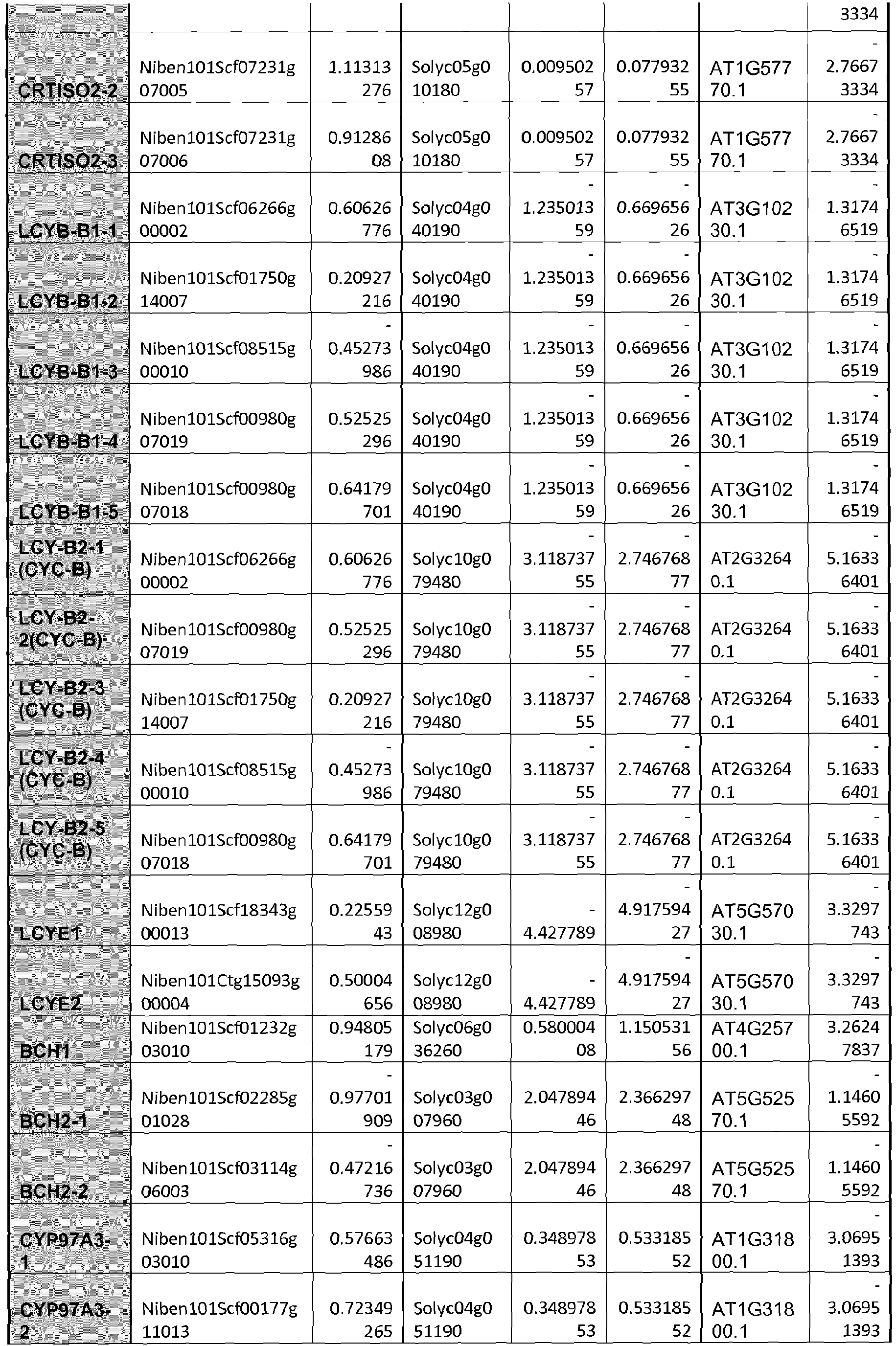

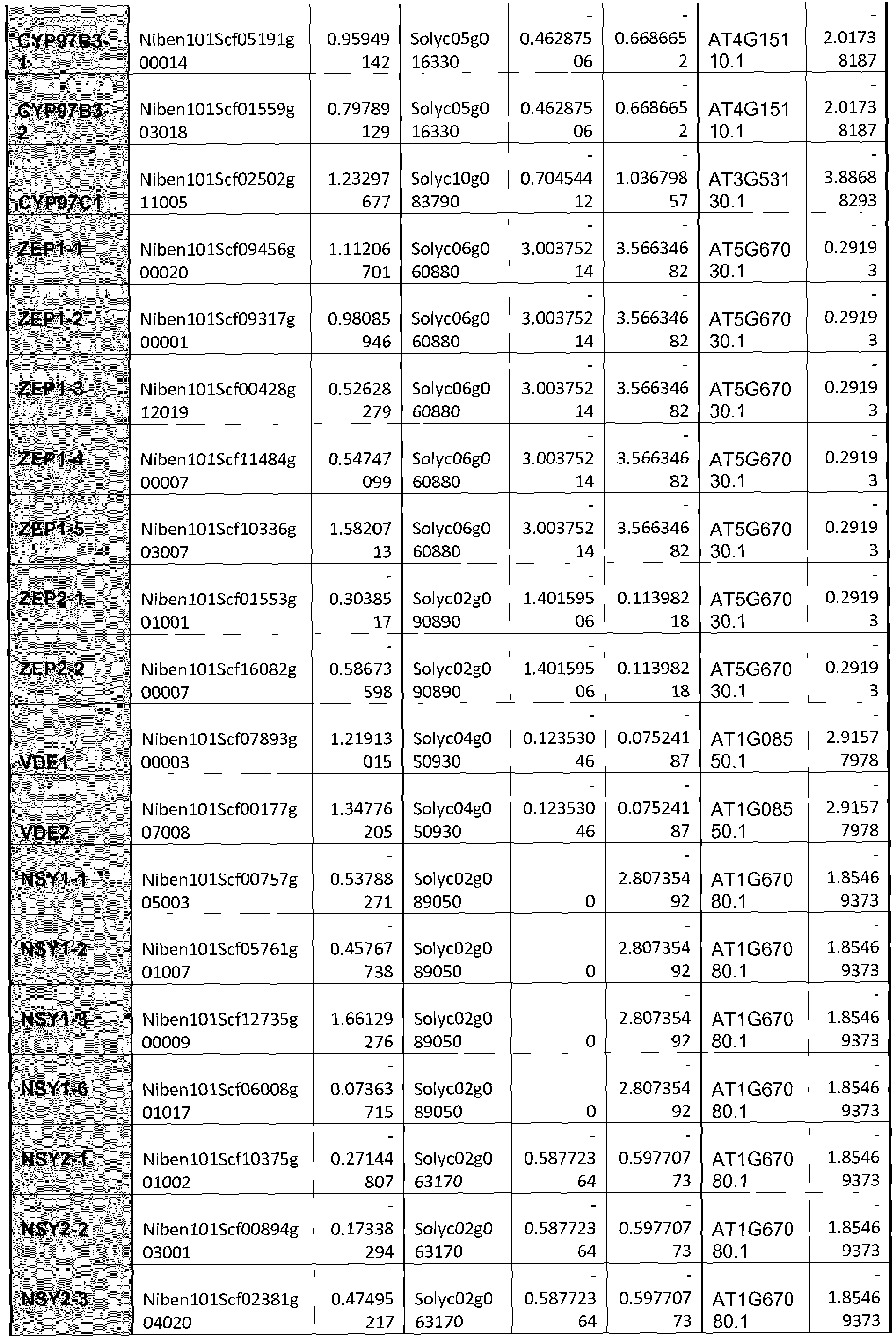

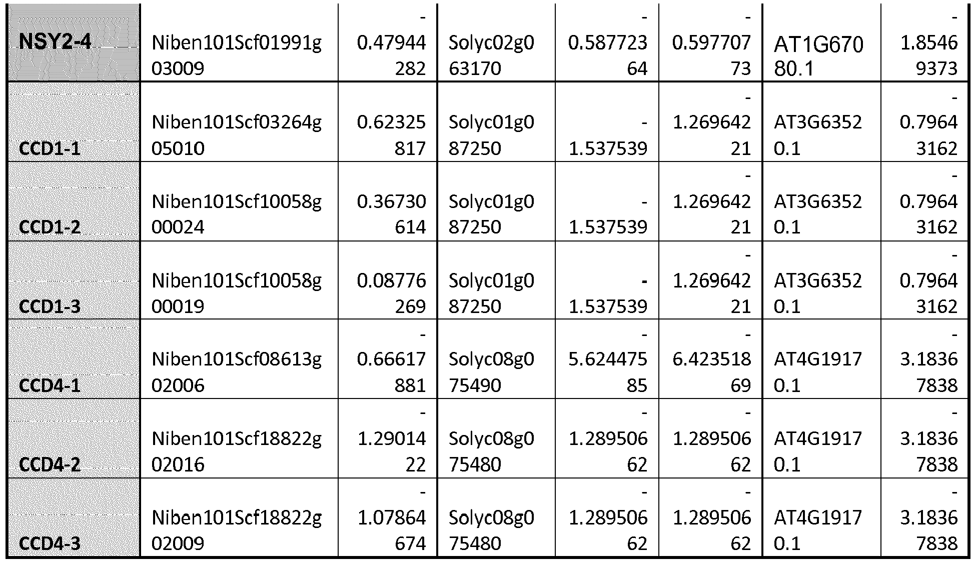

Energy and carbon required for carotenoid biosynthesis rely on photosynthesis (i.e. Calvin-Benson cycle) in chloroplasts. Because chromoplast differentiation in crtB-producing *N*. *benthamiana* leaves was associated with impairment of photosynthesis (Fig. 3C) and changes in the expression of genes potentially controlling the activity of many enzymes (Fig. 4), we asked whether primary cell metabolism might also be reprogrammed. To test this possibility, we conducted a GC-TOF-MS analyses of primary metabolites, including sugars, amino acids, and organic acids (Fig. 5). A total of 52 metabolites were annotated and their relative levels in (p)crtB leaf sections were normalized to the mean levels of GFP control sections. From these, 13 showed statistically significant changes: 3 were decreased and 10 were increased in (p)crtB samples (Fig. 5). We observed reductions in the levels of ascorbate but also of glucose and fructose, which are the main soluble carbohydrate stores and transport sugars as well as the main substrates for respiration. This, together with the increments in several intermediates of the tricarboxylic acid (TCA) cycle (citrate, 2-oxoglutarate and malate) and amino acids such as valine, isoleucine, aspartate and glutamate derived metabolites (Fig. 5), suggested that sugars were used to produce ATP through the TCA cycle to sustain amino acid synthesis and likely other cellular functions. Indeed, respiration rate determined as total oxygen consumption in the dark was found to be upregulated in chromoplast-containing leaf tissues (Fig. 5). We concluded that (p)crtB induced glycolytic and oxidative energy metabolism likely to compensate for photosynthesis shut down and subsequent reduction in the production of ATP and carotenoid substrates by the Calvin-Benson cycle.

### Example 4. Reaching a phytoene threshold is required to promote carotenoid overaccumulation in leaves.

In contrast with our results using a bacterial phytoene synthase (crtB), it has been previously shown that overproducing plant PSY enzymes does not increase carotenoid contents of leaves (Fraser et al. 2007; Maass et al. 2009; Busch et al. 2002). Consistent with these reports, when we transiently expressed PSY-encoding genes from Arabidopsis and tomato in *N. benthamiana* leaves, none of them induced the chromoplast-associated yellow phenotype (Fig. 12). Interestingly, the plant enzymes yielded significantly lower levels of phytoene than the plastid-targeted (p)crtB protein (Fig. 12).

The described results led us to hypothesize that a certain threshold level of phytoene or a downstream derivative might be needed to elicit chloroplast to chromoplast transformation. To test this hypothesis, we performed two experiments. In the first one, leaves of *N. benthamiana* plants were agroinfiltrated with serial dilutions of Agrobacterium cultures to express the (p)crtB construct at different levels. Samples infiltrated with low Agrobacterium densities showed no changes in total leaf carotenoid contents despite progressive increases in phytoene levels (Fig. 6A). When phytoene accumulated at levels higher than 20 ng/mg DW, however, a concomitant increase in total carotenoids was observed (Fig. 6A). The second experiment followed the time course of chromoplast differentiation after agroinfiltration of *N. benthamiana* leaves with the construct to express (p)crtB. Several leaves were agroinfiltrated with the construct and then pigment contents were measured at different time points. Phytoene became detectable at 36 hours post-infiltration (hpi), suddenly increased at 48 hpi, and then kept accumulating until 84 hpi (Fig. 5B). Downstream carotenoids started to increase at 48 dpi (Fig. 5B), again supporting the conclusion that carotenoid contents only increase once phytoene supply exceeds a certain threshold.

### Example 5. The supply of phytoene in plastids functions as a metabolic threshold switch that regulates chloroplast-to-chromoplast differentiation

Based on the described results, two mechanisms could be envisioned to explain how phytoene overproduction eventually led to chromoplast differentiation in leaves: (1) phytoene might readily feed the carotenoid pathway and promote carotenoid biosynthesis to reach levels so high that eventually changed plastid ultrastructure to accommodate these lipophilic compounds, or (2) phytoene per se might trigger the plastidial membrane remodeling process either directly (in the plastid) or indirectly (via signaling to the nucleus). Membrane reorganization would influence carotenoid biosynthesis, storage and/or degradation to achieve high carotenoid levels.

As a proxy of membrane remodeling dynamics, we monitored φPSII, NPQ and D1 protein levels during the crtB-mediated chloroplast to chromoplast transition (Fig. 7). These parameters are estimators of photosynthesis, photoprotection, and photodamage and hence of chloroplast membrane system (thylakoids and grana) functionality. In leaves agroinfiltrated with (p)crtB, both φPSII (Fig. 7A) and NPQ (Fig. 7B) remained unchanged up to 36 hpi, and then started to decrease as the levels of both phytoene and carotenoids increased (Fig. 6B). In the case of NPQ, the drop was mainly due to a decrease in qE, consistent with impairment of fast photoprotective responses such as the carotenoid-dependent xanthophyll cycle (Fig. S5). The levels of D1 started to decrease later, between 48 and 60 hpi (Fig. 7C), likely as a result of photodamage. These results together indicate that crtB-mediated upregulation of the carotenoid pathway rapidly disrupts the chloroplast photosynthetic membrane functionality.

To next test whether phytoene accumulation by itself could trigger the initial remodeling of plastidial membranes, we used norflurazon (NF) to block phytoene conversion into downstream carotenoids (Ortiz-Alcaide et al. 2019). *N. benthamiana* leaves were agroinfiltrated with different constructs and at 24 hpi some of them were also infiltrated with NF. The presence of NF led to the accumulation of some phytoene and slightly reduced carotenoid levels in GFP-producing leaves at 96 hpi (Fig. 8). NF-treated (p)crtB samples accumulated much more phytoene than GFP controls but showed similar levels of downstream carotenoids. Analysis of φPSII at different time points after agroinfiltration and NF treatment of these samples showed a negative correlation with phytoene rather than downstream or total carotenoid levels (Fig. 8). When the levels of total carotenoids (but not phytoene) were increased in leaves transiently expressing the Arabidopsis gene encoding PAR1, a transcription cofactor that promotes total carotenoid biosynthesis in photosynthetic tissues (Roig-Villanova et al. 2007; Bou-Torrent et al. 2015), no changes in φPSII were observed compared to GFP-producing controls (Fig. 8). We therefore conclude that phytoene accumulation by itself can disrupt the functionality (i.e. the identity) of the chloroplast. The observation that φPSII shows a much stronger and progressive reduction in untreated (p)crtB samples as carotenoids accumulate (Fig. 8) further suggests that exceeding a phytoene threshold might start the initial steps of the chromoplast differentiation program but production of downstream carotenoids is required to complete it.

In summary, our results are consistent with the existence of a two-step process responsible for the transformation of crtB-producing chloroplasts into chromoplasts. First, a metabolic switch mechanism elicits the initial changes of the process when the supply of phytoene exceeds a threshold, and second, increased accumulation of carotenoids due to enhanced phytoene availability but also to phytoene-triggered changes in plastid membranes completes the differentiation of chloroplasts into chromoplasts.

### References

Avendano-Vazquez, A. O., et al. (2014). "An Uncharacterized Apocarotenoid-Derived Signal Generated in zeta-Carotene Desaturase Mutants Regulates Leaf Development and the Expression of Chloroplast and Nuclear Genes in Arabidopsis." Plant Cell 26(6): 2524-2537.
Bai, C., et al. (2014). "An in vitro system for the rapid functional characterization of genes involved in carotenoid biosynthesis and accumulation." Plant J 77(3): 464-475.
Barsan, C., et al. (2010). "Characteristics of the tomato chromoplast revealed by proteomic analysis." J Exp Bot 61(9): 2413-2431.
Barsan, C., et al. (2012). "Proteomic analysis of chloroplast-to-chromoplast transition in tomato reveals metabolic shifts coupled with disrupted thylakoid biogenesis machinery and elevated energy-production components." Plant Physiol 160(2): 708-725.
Bedoya, L. C., et al. (2012). "Visual Tracking of Plant Virus Infection and Movement Using a Reporter MYB Transcription Factor That Activates Anthocyanin Biosynthesis." Plant Physiology 158(3): 1130-1138.
Besumbes, O., et al. (2004). "Metabolic engineering of isoprenoid biosynthesis in Arabidopsis for the production of taxadiene, the first committed precursor of Taxol." Biotechnol Bioeng 88(2): 168-175.
Brehelin, C., et al. (2007). "Plastoglobules: versatile lipoprotein particles in plastids." Trends Plant Sci 12(6): 260-266.
Cazzonelli, C. I., et al. (2019). "A cis-carotene derived apocarotenoid regulates etioplast and chloroplast development." bioRxiv.
Cunningham, F. X., Jr., et al. (1993). "Cloning and functional expression in Escherichia coli of a cyanobacterial gene for lycopene cyclase, the enzyme that catalyzes the biosynthesis of beta-carotene." FEBS Lett 328(1-2): 130-138.
D'Andrea, L., et al. (2018). "Interference with Clp protease impairs carotenoid accumulation during tomato fruit ripening." J Exp Bot 69(7): 1557-1568.
DellaPenna, D. and B. J. Pogson (2006). "Vitamin synthesis in plants: tocopherols and carotenoids." Annu Rev Plant Biol 57: 711-738.
Deruere, J., et al. (1994). "Fibril assembly and carotenoid overaccumulation in chromoplasts: a model for supramolecular lipoprotein structures." Plant Cell 6(1): 119-133.
Egea, I., et al. (2010). "Chromoplast differentiation: current status and perspectives." Plant Cell Physiol 51(10): 1601-1611.
Flores-Perez, U., et al. (2008). "A mutant impaired in the production of plastome-encoded proteins uncovers a mechanism for the homeostasis of isoprenoid biosynthetic enzymes in Arabidopsis plastids." Plant Cell 20(5): 1303-1315.
Fraser, P. D. and P. M. Bramley (2004). "The biosynthesis and nutritional uses of carotenoids." Prog Lipid Res 43(3): 228-265.
Gan, S. and R. M. Amasino (1995). "Inhibition of leaf senescence by autoregulated production of cytokinin." Science 270(5244): 1986-1988.
Giuliano, G. (2017). "Provitamin A biofortification of crop plants: a gold rush with many miners." Curr Opin Biotechnol 44: 169-180.
Gramegna, G., et al. (2018). "PHYTOCHROME-INTERACTING FACTOR 3 mediates light-dependent induction of tocopherol biosynthesis during tomato fruit ripening." Plant Cell Environ.
Hormaetxe, K., et al. (2004). "Role of red carotenoids in photoprotection during winter acclimation in Buxus sempervirens leaves." Plant Biol (Stuttg) 6(3): 325-332.
Hou, X., et al. (2016). "Synthesis and Function of Apocarotenoid Signals in Plants." Trends Plant Sci 21(9): 792-803.
Hughes, N. M. (2011). "Winter leaf reddening in 'evergreen' species." New Phytol 190(3): 573-581.
Jarvis, P. and E. Lopez-Juez (2013). "Biogenesis and homeostasis of chloroplasts and other plastids." Nat Rev Mol Cell Biol 14(12): 787-802.
Kahlau, S. and R. Bock (2008). "Plastid transcriptomics and translatomics of tomato fruit development and chloroplast-to-chromoplast differentiation: chromoplast gene expression largely serves the production of a single protein." Plant Cell 20(4): 856-874.
Kleffmann, T., et al. (2007). "Proteome dynamics during plastid differentiation in rice." Plant Physiol 143(2): 912-923.
Koiwa, H., et al. (1986). "Reversal of chromoplasts to chloroplasts in Buxus leaves." Botanical Magazine, Tokyo 99: 233-240.
Li, X. H. and J. C. Carrington (1995). "Complementation of tobacco etch potyvirus mutants by active RNA polymerase expressed in transgenic cells." Proc Natl Acad Sci U S A 92(2): 457-461.
Li-Beisson, Y., et al. (2013). "Acyl-lipid metabolism." Arabidopsis Book 11: e0161.
Liebers, M., et al. (2017). "Regulatory Shifts in Plastid Transcription Play a Key Role in Morphological Conversions of Plastids during Plant Development." Front Plant Sci 8: 23.
Llorente, B., et al. (2016). "Tomato fruit carotenoid biosynthesis is adjusted to actual ripening progression by a light-dependent mechanism." Plant J 85(1): 107-119.
Llorente, B., et al. (2017). "Illuminating colors: regulation of carotenoid biosynthesis and accumulation by light." Curr Opin Plant Biol 37: 49-55.
Lopez, A. B., et al. (2008). "Effect of the cauliflower Or transgene on carotenoid accumulation and chromoplast formation in transgenic potato tubers." J Exp Bot 59(2): 213-223.
Lu, Y., et al. (2017). "Horizontal Transfer of a Synthetic Metabolic Pathway between Plant Species." Curr Biol 27(19): 3034-3041 e3033.
Maass, D., et al. (2009). "Carotenoid crystal formation in Arabidopsis and carrot roots caused by increased phytoene synthase protein levels." PLoS One 4(7): e6373.
Majer, E., et al. (2017). "Rewiring carotenoid biosynthesis in plants using a viral vector." Sci Rep 7: 41645.
Nakagawa, T., et al. (2007). "Improved Gateway binary vectors: high-performance vectors for creation of fusion constructs in transgenic analysis of plants." Biosci Biotechnol Biochem 71(8): 2095-2100.
Ogbonna, J. C. (2009). "Microbiological production of tocopherols: current state and prospects." Appl Microbiol Biotechnol 84(2): 217-225.
Okada, R., et al. (2017). "Synthetic control of flowering in rice independent of the cultivation environment." Nat Plants 3: 17039.
Pulido, P., et al. (2016). "Specific Hsp100 Chaperones Determine the Fate of the First Enzyme of the Plastidial Isoprenoid Pathway for Either Refolding or Degradation by the Stromal Clp Protease in Arabidopsis." PLoS Genet 12(1): e1005824.
Rodriguez-Concepcion, M., et al. (2018). "A global perspective on carotenoids: Metabolism, biotechnology, and benefits for nutrition and health." Prog Lipid Res 70: 62-93.
Rodriguez-Concepcion, M. and A. Boronat (2002). "Elucidation of the methylerythritol phosphate pathway for isoprenoid biosynthesis in bacteria and plastids. A metabolic milestone achieved through genomics." Plant Physiol 130(3): 1079-1089.
Ruiz-Sola, M. A., et al. (2016). "A Single Arabidopsis Gene Encodes Two Differentially Targeted Geranylgeranyl Diphosphate Synthase Isoforms." Plant Physiol 172(3): 1393-1402.
Schaub, P., et al. (2018). "Establishment of an Arabidopsis callus system to study the interrelations of biosynthesis, degradation and accumulation of carotenoids." PLoS One 13(2): e0192158.
Schmidt, G. W. and S. K. Delaney (2010). "Stable internal reference genes for normalization of real-time RT-PCR in tobacco (Nicotiana tabacum) during development and abiotic stress." Mol Genet Genomics 283(3): 233-241.
Schwach, F., et al. (2005). "An RNA-dependent RNA polymerase prevents meristem invasion by potato virus X and is required for the activity but not the production of a systemic silencing signal." Plant Physiol 138(4): 1842-1852.
Simkin, A. J., et al. (2007). "Fibrillin influence on plastid ultrastructure and pigment content in tomato fruit." Phytochemistry 68(11): 1545-1556.
Singh, D. K. and T. W. McNellis (2011). "Fibrillin protein function: the tip of the iceberg?" Trends Plant Sci 16(8): 432-441.
Sparkes, I. A., et al. (2006). "Rapid, transient expression of fluorescent fusion proteins in tobacco plants and generation of stably transformed plants." Nat Protoc 1(4): 2019-2025.
Tholl, D. and S. Lee (2011). "Terpene Specialized Metabolism in Arabidopsis thaliana." Arabidopsis Book 9: e0143.
Van Norman, J. M., et al. (2014). "Periodic root branching in Arabidopsis requires synthesis of an uncharacterized carotenoid derivative." Proc Natl Acad Sci U S A 111(13): E1300-1309.
Wang, J. Y., et al. (2019). "The apocarotenoid metabolite zaxinone regulates growth and strigolactone biosynthesis in rice." Nat Commun 10(1): 810.
Wang, Z. and C. Benning (2011). "Arabidopsis thaliana polar glycerolipid profiling by thin layer chromatography (TLC) coupled with gas-liquid chromatography (GLC)." J Vis Exp(49).
Wurtzel, E. T. (2019). "Changing Form and Function through Carotenoids and Synthetic Biology." Plant Physiol 179(3): 830-843.
Yuan, H., et al. (2015). "A Single Amino Acid Substitution in an ORANGE Protein Promotes Carotenoid Overaccumulation in Arabidopsis." Plant Physiol 169(1): 421-431.
Yuan, H., et al. (2015). "Carotenoid metabolism and regulation in horticultural crops." Hortic Res 2: 15036.

## Claims

1. A method of inducing chloroplast-to-chromoplast differentiation biogenesis in leaf vegetables and/or forage crops that comprises the induction of a constitutive, inducible or transient expression of a plasmid construct or expression vector in the leaf vegetables and/or forage crops, wherein said plasmid construct is **characterized in that** the plasmid construct comprises a heterologous nucleic acid encoding a polypeptide exhibiting phytoene synthase activity, wherein said polypeptide exhibiting phytoene synthase activity further comprises a plastid-targeting signal in its N-terminus, and wherein said plasmid construct or expression vector is capable of accumulating in the chloroplasts of the plant tissue an amount of phtyoene sufficient to induce chloroplast to chromoplast differentiation in said plant tissue, preferably at least about **0.01 µg/mg DW** of phytoene in the leaf vegetables or forage crops between 36 and 48 hpi.

2. A method to increase carotenoid and tocopherol contents in leaf vegetables and forage crops that comprises the induction of a constitutive, inducible or transient expression of a plasmid construct or expression vector in the leaf vegetables and forage crops, wherein said plasmid construct is **characterized in that** the plasmid construct comprises a heterologous nucleic acid encoding a polypeptide exhibiting phytoene synthase activity, wherein said polypeptide exhibiting phytoene synthase activity further comprises a plastid-targeting signal in its N-terminus, and wherein said plasmid construct or expression vector is capable of accumulating in the chloroplasts of the plant tissue an amount of phtyoene sufficient to induce chloroplast to chromoplast differentiation in said plant tissue, preferably at least about **0.01 µg/mg DW** of phytoene in the leaf vegetables or forage crops between 36 and 48 hpi.

3. A method to enhance the production and storage capacity of lipophilic chemicals in plants, the method comprising the the induction of a constitutive, inducible or transient expression of a plasmid construct or expression vector in the leaf vegetables and forage crops, wherein said plasmid construct is **characterized in that** the plasmid construct comprises a heterologous nucleic acid encoding a polypeptide exhibiting phytoene synthase activity and/or may overexpress an endogenous nucleic acid encoding a polypeptide exhibiting phytoene synthase activity, wherein said polypeptide exhibiting phytoene synthase activity further comprises a plastid-targeting signal in its N-terminus, and wherein said plasmid construct or expression vector is capable of accumulating in the chloroplasts of the plant tissue an amount of phtyoene sufficient to induce chloroplast to chromoplast differentiation in said plant tissue, preferably at least about **0.01 µg/mg DW** of phytoene in the leaf vegetables or forage crops between 36 and 48 hpi.

4. A modified leaf vegetable or forage crop comprising the constitutive, inducible or transient expression of a plasmid construct or expression vector, wherein said plasmid construct is **characterized in that** the plasmid construct comprises a heterologous nucleic acid encoding a polypeptide exhibiting phytoene synthase activity, wherein said polypeptide exhibiting phytoene synthase activity further comprises a plastid-targeting signal, and wherein said plasmid construct or expression vector is capable of accumulating in the chloroplasts of the plant tissue an amount of phtyoene sufficient to induce chloroplast to chromoplast differentiation in said plant tissue, preferably at least about **0.01 µg/mg DW** of phytoene in the leaf vegetables or forage crops between 36 and 48 hpi.

5. The method of any of claims 1 to 3 or the modified leaf vegetable or forage crop of claim 4, wherein the polypeptide exhibiting phytoene synthase activity is CrtB.

6. The method of any of claims 1 to 3 or the modified leaf vegetable or forage crop of claim 4, wherein the polypeptide exhibiting phytoene synthase activity *Pantoea ananatis* crtB (GenBank accession number: P21683).

7. The method of claim 5 or 6 or the modified leaf vegetable or forage crop of claim 5 or 6, wherein plastid-targeting signal is the sequence of the hydroxymethylbutenyl 4-diphosphate synthase gene from *A. thaliana* cDNA.

8. The method or the modified leaf vegetable or forage crop of any of the precedent claims, wherein the expression cassette comprise at least a CrtB gene operably linked to a plastid-targeting signal, preferably in its N-terminus and optionally one or more control sequences, typically comprising a transcriptional promoter and a transcription terminator, that direct the expression of said phytoene synthase in the species and tissue/organ of interest.

9. The method or the modified leaf vegetable or forage crop of claim 8, wherein the control sequence includes a promoter that is recognized by the host cell.

10. The method or the modified leaf vegetable or forage crop of any of the precedent claims, wherein the production of phytoene is enhanced by genetically modified the leaf vegetables and forage crops to increase the carbon flux to isopentenyl pyrophosphate (IPP) and dimethylallyl pyrophosphate (DMAPP) and/or to increase the conversion of IPP and DMAPP to geranylgeranyl diphosphate (GGPP).

11. A cosmetic or pharmaceutical composition comprising the leaf vegetable or forage crop of any of the precedent claims, or an extract therefrom.

12. A nutraceutical or nutricosmetic composition, or a food or feed additive comprising the leaf vegetable or forage crop of any of the precedent claims.

13. The cosmetic or pharmaceutical composition of claim 11, wherein the cosmetic or pharmaceutical composition is a skin whitening, lightening or bleaching composition or a composition to prevent aging, oxidative or photo-oxidative damages.

14. The pharmaceutical composition of claim 11, for use in the treatment of cancer or in the treatment of inflammatory disorders. (cardiovascular disease)


SEQUENCE LISTING

<110>  Centre for Research in Agricultural Genomics

<120>  Artificial chromoplast biogenesis

<130>  904 166

<160>  19

<170>  PatentIn version 3.5

<210>  1
<211>  11680
<212>  DNA
<213>  Artificial Sequence

<220>
<223>  (p)crtB_stop_pGWB605


<220>
<221>  misc_feature
<222>  (1)..(11680)

<400>  1
cttttcacgc ccttttaaat atccgattat tctaataaac gctcttttct cttaggttta      60

cccgccaata tatcctgtca aacactgata gtttaaactg aaggcgggaa acgacaatct     120

gatccaagct caagctgctc tagcattcgc cattcaggct gcgcaactgt tgggaagggc     180

gatcggtgcg ggcctcttcg ctattacgcc agctggcgaa agggggatgt gctgcaaggc     240

gattaagttg ggtaacgcca gggttttccc agtcacgacg ttgtaaaacg acggccagtg     300

ccaagcttgc atgcctgcag gtccccagat tagccttttc aatttcagaa agaatgctaa     360

cccacagatg gttagagagg cttacgcagc aggtctcatc aagacgatct acccgagcaa     420

taatctccag gaaatcaaat accttcccaa gaaggttaaa gatgcagtca aaagattcag     480

gactaactgc atcaagaaca cagagaaaga tatatttctc aagatcagaa gtactattcc     540

agtatggacg attcaaggct tgcttcacaa accaaggcaa gtaatagaga ttggagtctc     600

taaaaaggta gttcccactg aatcaaaggc catggagtca aagattcaaa tagaggacct     660

aacagaactc gccgtaaaga ctggcgaaca gttcatacag agtctcttac gactcaatga     720

caagaagaaa atcttcgtca acatggtgga gcacgacaca cttgtctact ccaaaaatat     780

caaagataca gtctcagaag accaaagggc aattgagact tttcaacaaa gggtaatatc     840

cggaaacctc ctcggattcc attgcccagc tatctgtcac tttattgtga agatagtgga     900

aaaggaaggt ggctcctaca aatgccatca ttgcgataaa ggaaaggcca tcgttgaaga     960

tgcctctgcc gacagtggtc ccaaagatgg acccccaccc acgaggagca tcgtggaaaa    1020

agaagacgtt ccaaccacgt cttcaaagca agtggattga tgtgatatct ccactgacgt    1080

aagggatgac gcacaatccc actatccttc gcaagaccct tcctctatat aaggaagttc    1140

atttcatttg gagagaacac gggggactct agagttatca acaagtttgt acaaaaaagc 1200

aggctatggc gactggagta ttgccagctc cggtttctgg gatcaagata ccggattcga 1260

aagtcgggtt tggtaaaagc atgaatcttg tgagaatttg tgatgttagg agtctaagat 1320

ctgctaggag aagagtttcg gttatccgga atatgaataa tccgtcgtta ctcaatcatg 1380

cggtcgaaac gatggcagtt ggctcgaaaa gttttgcgac agcctcaaag ttatttgatg 1440

caaaaacccg gcgcagcgta ctgatgctct acgcctggtg ccgccattgt gacgatgtta 1500

ttgacgatca gacgctgggc tttcaggccc ggcagcctgc cttacaaacg cccgaacaac 1560

gtctgatgca acttgagatg aaaacgcgcc aggcctatgc aggatcgcag atgcacgaac 1620

cggcgtttgc ggcttttcag gaagtggcta tggctcatga tatcgccccg gcttacgcgt 1680

ttgatcatct ggaaggcttc gccatggatg tacgcgaagc gcaatacagc caactggatg 1740

atacgctgcg ctattgctat cacgttgcag gcgttgtcgg cttgatgatg gcgcaaatca 1800

tgggcgtgcg ggataacgcc acgctggacc gcgcctgtga ccttgggctg gcatttcagt 1860

tgaccaatat tgctcgcgat attgtggacg atgcgcatgc gggccgctgt tatctgccgg 1920

caagctggct ggagcatgaa ggtctgaaca aagagaatta tgcggcacct gaaaaccgtc 1980

aggcgctgag ccgtatcgcc cgtcgtttgg tgcaggaagc agaaccttac tatttgtctg 2040

ccacagccgg cctggcaggg ttgcccctgc gttccgcctg ggcaatcgct acggcgaagc 2100

aggtttaccg gaaaataggt gtcaaagttg aacaggccgg tcagcaagcc tgggatcagc 2160

ggcagtcaac gaccacgccc gaaaaattaa cgctgctgct ggccgcctct ggtcaggccc 2220

ttacttcccg gatgcgggct catcctcccc gccctgcgca tctctggcag cgcccgctct 2280

agacccagct ttcttgtaca aagtggttcg atctagagga tccatggtga gcaagggcga 2340

ggagctgttc accggggtgg tgcccatcct ggtcgagctg gacggcgacg tgaacggcca 2400

caagttcagc gtgtccggcg agggcgaggg cgatgccacc tacggcaagc tgaccctgaa 2460

gttcatctgc accaccggca agctgcccgt gccctggccc accctcgtga ccaccttcac 2520

ctacggcgtg cagtgcttca gccgctaccc cgaccacatg aagcagcacg acttcttcaa 2580

gtccgccatg cccgaaggct acgtccagga gcgcaccatc ttcttcaagg acgacggcaa 2640

ctacaagacc cgcgccgagg tgaagttcga gggcgacacc ctggtgaacc gcatcgagct 2700

gaagggcatc gacttcaagg aggacggcaa catcctgggg cacaagctgg agtacaacta 2760

caacagccac aacgtctata tcatggccga caagcagaag aacggcatca aggtgaactt 2820

caagatccgc cacaacatcg aggacggcag cgtgcagctc gccgaccact accagcagaa 2880

cacccccatc ggcgacggcc ccgtgctgct gcccgacaac cactacctga gcacccagtc 2940

cgccctgagc aaagacccca acgagaagcg cgatcacatg gtcctgctgg agttcgtgac 3000

cgccgccggg atcactcacg gcatggacga gctgtacaag taaagcggcc cgagctcgaa 3060
tttccccgat cgttcaaaca tttggcaata aagtttctta agattgaatc ctgttgccgg 3120
tcttgcgatg attatcatat aatttctgtt gaattacgtt aagcatgtaa taattaacat 3180
gtaatgcatg acgttattta tgagatgggt ttttatgatt agagtcccgc aattatacat 3240
ttaatacgcg atagaaaaca aaatatagcg cgcaaactag gataaattat cgcgcgcggt 3300
gtcatctatg ttactagatc gggaattggt tccggaacca attcgtaatc atggtcatag 3360
ctgtttcctg tgtgaaattg ttatccgctc acaattccac acaacatacg agccggaagc 3420
ataaagtgta aagcctgggg tgcctaatga gtgagctaac tcacattagg ctgaattagg 3480
cgcgcctatt tctattaatt cccgatctag taacatagat gacaccgcgc gcgataattt 3540
atcctagttt gcgcgctata ttttgttttc tatcgcgtat taaatgtata attgcgggac 3600
tctaatcata aaaacccatc tcataaataa cgtcatgcat tacatgttaa ttattacatg 3660
cttaacgtaa ttcaacagaa attatatgat aatcatcgca agaccggcaa caggattcaa 3720
tcttaagaaa ctttattgcc aaatgtttga acgatcgggg aaattcgagc tggggacgtc 3780
cccagctcgg taccccctcc tgggcacggt cgtacgcgat ccgccgcggt tccacctcac 3840
cattgcgggt atgccagttg agttcgatga gttcccgtac cagctccaac tcgggcgatg 3900
ccacgcgcac tccgttcaga tctcggtgac gggcaggacc ggacggggcg gtaccggcag 3960
gctgaagtcc agctgccaga aacccacgtc atgccagttc ccgtgcttga agccggccgc 4020
ccgcagcatg ccgcgggggg catatccgag cgcctcgtgc atgcgcacgc tcgggtcgtt 4080
gggcagcccg atgacagcga ccacgctctt gaagccctgt gcctccaggg acttcagcag 4140
gtgggtgtag agcgtggagc ccagtcccgt ccgctggtgg cggggggaga cgtacacggt 4200
cgactcggcc gtccagtcgt aggcgttgcg tgccttccag gggcccgcgt aggcgatgcc 4260
ggcgacctcg ccgtccacct cggcgacgag ccagggatag cgctcccgca gacggacgag 4320
gtcgtccgtc cactcctgcg gttcctgcgg ctcggtacgg aagttgaccg tgcttgtctc 4380
gatgtagtgg ttgacgatgg tgcagaccgc cggcatgtcc gcctcggtgg cacggcggat 4440
gtcggccggg cgtcgttctg ggctcatatc ttattgcccc ccgggatcag atctggattg 4500
agagtgaata tgagactcta attggatacc gaggggaatt tatggaacgt cagtggagca 4560
tttttgacaa gaaatatttg ctagctgata gtgaccttag gcgacttttg aacgcgcaat 4620
aatggtttct gacgtatgtg cttagctcat taaactccag aaacccgcgg ctgagtggct 4680
ccttcaacgt tgcggttctg tcagttccaa acgtaaaacg gcttgtcccg cgtcatcggc 4740
gggggtcata acgtgactcc cttaattctc cgctcatgat cttgatcccc tgcgccatca 4800
gatccttggc ggcaagaaag ccatccagtt tactttgcag ggcttcccaa ccttaccaga 4860
gggcgcccca gctggcaatt ccggttcgct tgctgtccat aaaaccgccc agtctagcta 4920

tcgccatgta agcccactgc aagctacctg ctttctcttt gcgcttgcgt tttcccttgt 4980

ccagatagcc cagtagctga cattcatccg gggtcagcac cgtttctgcg gactggcttt 5040

ctacgtgttc cgcttccttt agcagccctt gcgccctgag tgcttgcggc agcgtgaagc 5100

tgatccgtcg agattaatag aaataaattc agcctaattc ggcgttaatt cagtacatta 5160

aaaacgtccg caatgtgtta ttaagttgtc taagcgtcaa tttgtttaca ccacaatata 5220

tcctgccacc agccagccaa cagctccccg accggcagct cggcacaaaa tcaccactcg 5280

atacaggcag cccatcagtc cgggacggcg tcagcgggag agccgttgta aggcggcaga 5340

ctttgctcat gttaccgatg ctattcggaa gaacggcaac taagctgccg ggtttgaaac 5400

acggatgatc tcgcggaggg tagcatgttg attgtaacga tgacagagcg ttgctgcctg 5460

tgatcaattc gggcacgaac ccagtggaca taagcctgtt cggttcgtaa gctgtaatgc 5520

aagtagcgta tgcgctcacg caactggtcc agaaccttga ccgaacgcag cggtggtaac 5580

ggcgcagtgg cggttttcat ggcttgttat gactgttttt ttggggtaca gtctatgcct 5640

cgggcatcca agcagcaagc gcgttacgcc gtgggtcgat gtttgatgtt atggagcagc 5700

aacgatgtta cgcagcaggg cagtcgccct aaaacaaagt taaacatcat gggggaagcg 5760

gtgatcgccg aagtatcgac tcaactatca gaggtagttg gcgtcatcga gcgccatctc 5820

gaaccgacgt tgctggccgt acatttgtac ggctccgcag tggatggcgg cctgaagcca 5880

cacagtgata ttgatttgct ggttacggtg accgtaaggc ttgatgaaac aacgcggcga 5940

gctttgatca acgacctttt ggaaacttcg gcttcccctg gagagagcga gattctccgc 6000

gctgtagaag tcaccattgt tgtgcacgac gacatcattc cgtggcgtta tccagctaag 6060

cgcgaactgc aatttggaga atggcagcgc aatgacattc ttgcaggtat cttcgagcca 6120

gccacgatcg acattgatct ggctatcttg ctgacaaaag caagagaaca tagcgttgcc 6180

ttggtaggtc cagcggcgga ggaactcttt gatccggttc ctgaacagga tctatttgag 6240

gcgctaaatg aaaccttaac gctatggaac tcgccgcccg actgggctgg cgatgagcga 6300

aatgtagtgc ttacgttgtc ccgcatttgg tacagcgcag taaccggcaa aatcgcgccg 6360

aaggatgtcg ctgccgactg ggcaatggag cgcctgccgg cccagtatca gcccgtcata 6420

cttgaagcta gacaggctta tcttggacaa gaagaagatc gcttggcctc gcgcgcagat 6480

cagttggaag aatttgtcca ctacgtgaaa ggcgagatca ccaaggtagt cggcaaataa 6540

tgtctagcta gaaattcgtt caagccgacg ccgcttcgcg gcgcggctta actcaagcgt 6600

tagatgcact aagcacataa ttgctcacag ccaaactatc aggtcaagtc tgcttttatt 6660

atttttaagc gtgcataata agccctacac aaattgggag atatatcatg catgaccaaa 6720

atcccttaac gtgagttttc gttccactga gcgtcagacc ccgtagaaaa gatcaaagga 6780

tcttcttgag atcctttttt tctgcgcgta atctgctgct tgcaaacaaa aaaaccaccg 6840

ctaccagcgg tggtttgttt gccggatcaa gagctaccaa ctctttttcc gaaggtaact 6900

ggcttcagca gagcgcagat accaaatact gtccttctag tgtagccgta gttaggccac 6960

cacttcaaga actctgtagc accgcctaca tacctcgctc tgctaatcct gttaccagtg 7020

gctgctgcca gtggcgataa gtcgtgtctt accgggttgg actcaagacg atagttaccg 7080

gataaggcgc agcggtcggg ctgaacgggg ggttcgtgca cacagcccag cttggagcga 7140

acgacctaca ccgaactgag atacctacag cgtgagctat gagaaagcgc cacgcttccc 7200

gaagggagaa aggcggacag gtatccggta agcggcaggg tcggaacagg agagcgcacg 7260

agggagcttc cagggggaaa cgcctggtat ctttatagtc ctgtcgggtt tcgccacctc 7320

tgacttgagc gtcgattttt gtgatgctcg tcaggggggc ggagcctatg gaaaaacgcc 7380

agcaacgcgg cctttttacg gttcctggcc ttttgctggc cttttgctca catgttcttt 7440

cctgcgttat cccctgattc tgtggataac cgtattaccg cctttgagtg agctgatacc 7500

gctcgccgca gccgaacgac cgagcgcagc gagtcagtga gcgaggaagc ggaagagcgc 7560

ctgatgcggt attttctcct tacgcatctg tgcggtattt cacaccgcat atggtgcact 7620

ctcagtacaa tctgctctga tgccgcatag ttaagccagt atacactccg ctatcgctac 7680

gtgactgggt catggctgcg ccccgacacc cgccaacacc cgctgacgcg ccctgacggg 7740

cttgtctgct cccggcatcc gcttacagac aagctgtgac cgtctccggg agctgcatgt 7800

gtcagaggtt ttcaccgtca tcaccgaaac gcgcgaggca gggtgccttg atgtgggcgc 7860

cggcggtcga gtggcgacgg cgcggcttgt ccgcgccctg gtagattgcc tggccgtagg 7920

ccagccattt ttgagcggcc agcggccgcg ataggccgac gcgaagcggc ggggcgtagg 7980

gagcgcagcg accgaagggt aggcgctttt tgcagctctt cggctgtgcg ctggccagac 8040

agttatgcac aggccaggcg ggttttaaga gttttaataa gttttaaaga gttttaggcg 8100

gaaaaatcgc cttttttctc ttttatatca gtcacttaca tgtgtgaccg gttcccaatg 8160

tacggctttg ggttcccaat gtacgggttc cggttcccaa tgtacggctt tgggttccca 8220

atgtacgtgc tatccacagg aaagagacct tttcgacctt tttcccctgc tagggcaatt 8280

tgccctagca tctgctccgt acattaggaa ccggcggatg cttcgccctc gatcaggttg 8340

cggtagcgca tgactaggat cgggccagcc tgccccgcct cctccttcaa atcgtactcc 8400

ggcaggtcat ttgacccgat cagcttgcgc acggtgaaac agaacttctt gaactctccg 8460

gcgctgccac tgcgttcgta gatcgtcttg aacaaccatc tggcttctgc cttgcctgcg 8520

gcgcggcgtg ccaggcggta gagaaaacgg ccgatgccgg gatcgatcaa aaagtaatcg 8580

gggtgaaccg tcagcacgtc cgggttcttg ccttctgtga tctcgcggta catccaatca 8640

gctagctcga tctcgatgta ctccggccgc ccggtttcgc tctttacgat cttgtagcgg 8700

ctaatcaagg cttcaccctc ggataccgtc accaggcggc cgttcttggc cttcttcgta 8760

cgctgcatgg caacgtgcgt ggtgtttaac cgaatgcagg tttctaccag gtcgtctttc 8820

tgctttccgc catcggctcg ccggcagaac ttgagtacgt ccgcaacgtg tggacggaac 8880

acgcggccgg gcttgtctcc cttcccttcc cggtatcggt tcatggattc ggttagatgg 8940

gaaaccgcca tcagtaccag gtcgtaatcc cacacactgg ccatgccggc cggccctgcg 9000

gaaacctcta cgtgcccgtc tggaagctcg tagcggatca cctcgccagc tcgtcggtca 9060

cgcttcgaca gacggaaaac ggccacgtcc atgatgctgc gactatcgcg ggtgcccacg 9120

tcatagagca tcggaacgaa aaaatctggt tgctcgtcgc ccttgggcgg cttcctaatc 9180

gacggcgcac cggctgccgg cggttgccgg gattctttgc ggattcgatc agcggccgct 9240

tgccacgatt caccggggcg tgcttctgcc tcgatgcgtt gccgctgggc ggcctgcgcg 9300

gccttcaact tctccaccag gtcatcaccc agcgccgcgc cgatttgtac cgggccggat 9360

ggtttgcgac cgctcacgcc gattcctcgg gcttgggggt tccagtgcca ttgcagggcc 9420

ggcagacaac ccagccgctt acgcctggcc aaccgcccgt tcctccacac atggggcatt 9480

ccacggcgtc ggtgcctggt tgttcttgat tttccatgcc gcctccttta gccgctaaaa 9540

ttcatctact catttattca tttgctcatt tactctggta gctgcgcgat gtattcagat 9600

agcagctcgg taatggtctt gccttggcgt accgcgtaca tcttcagctt ggtgtgatcc 9660

tccgccggca actgaaagtt gacccgcttc atggctggcg tgtctgccag gctggccaac 9720

gttgcagcct tgctgctgcg tgcgctcgga cggccggcac ttagcgtgtt tgtgcttttg 9780

ctcattttct ctttacctca ttaactcaaa tgagttttga tttaatttca gcggccagcg 9840

cctggacctc gcgggcagcg tcgccctcgg gttctgattc aagaacggtt gtgccggcgg 9900

cggcagtgcc tgggtagctc acgcgctgcg tgatacggga ctcaagaatg ggcagctcgt 9960

acccggccag cgcctcggca acctcaccgc cgatgcgcgt gcctttgatc gcccgcgaca 10020

cgacaaaggc cgcttgtagc cttccatccg tgacctcaat gcgctgctta accagctcca 10080

ccaggtcggc ggtggcccat atgtcgtaag ggcttggctg caccggaatc agcacgaagt 10140

cggctgcctt gatcgcggac acagccaagt ccgccgcctg gggcgctccg tcgatcacta 10200

cgaagtcgcg ccggccgatg gccttcacgt cgcggtcaat cgtcgggcgg tcgatgccga 10260

caacggttag cggttgatct tcccgcacgg ccgcccaatc gcgggcactg ccctggggat 10320

cggaatcgac taacagaaca tcggccccgg cgagttgcag ggcgcgggct agatgggttg 10380

cgatggtcgt cttgcctgac ccgcctttct ggttaagtac agcgataacc ttcatgcgtt 10440

ccccttgcgt atttgtttat ttactcatcg catcatatac gcagcgaccg catgacgcaa 10500

gctgttttac tcaaatacac atcacctttt tagacggcgg cgctcggttt cttcagcggc 10560

caagctggcc ggccaggccg ccagcttggc atcagacaaa ccggccagga tttcatgcag 10620

ccgcacggtt gagacgtgcg cgggcggctc gaacacgtac ccggccgcga tcatctccgc 10680

ctcgatctct tcggtaatga aaaacggttc gtcctggccg tcctggtgcg gtttcatgct 10740

tgttcctctt ggcgttcatt ctcggcggcc gccagggcgt cggcctcggt caatgcgtcc 10800

tcacggaagg caccgcgccg cctggcctcg gtgggcgtca cttcctcgct gcgctcaagt 10860

gcgcggtaca gggtcgagcg atgcacgcca agcagtgcag ccgcctcttt cacggtgcgg 10920

ccttcctggt cgatcagctc gcgggcgtgc gcgatctgtg ccggggtgag ggtagggcgg 10980

gggccaaact tcacgcctcg ggccttggcg gcctcgcgcc cgctccgggt gcggtcgatg 11040

attagggaac gctcgaactc ggcaatgccg gcgaacacgg tcaacaccat gcggccggcc 11100

ggcgtggtgg tgtcggccca cggctctgcc aggctacgca ggcccgcgcc ggcctcctgg 11160

atgcgctcgg caatgtccag taggtcgcgg gtgctgcggg ccaggcggtc tagcctggtc 11220

actgtcacaa cgtcgccagg gcgtaggtgg tcaagcatcc tggccagctc cgggcggtcg 11280

cgcctggtgc cggtgatctt ctcggaaaac agcttggtgc agccggccgc gtgcagttcg 11340

gcccgttggt tggtcaagtc ctggtcgtcg gtgctgacgc gggcatagcc cagcaggcca 11400

gcggcggcgc tcttgttcat ggcgtaatgt ctccggttct agtcgcaagt attctacttt 11460

atgcgactaa aacacgcgac aagaaaacgc caggaaaagg gcagggcggc agcctgtcgc 11520

gtaacttagg acttgtgcga catgtcgttt tcagaagacg gctgcactga acgtcagaag 11580

ccgactgcac tatagcagcg gaggggttgg atcaaagtac tttgatcccg aggggaaccc 11640

tgtggttggc atgcacatac aaatggacga acggataaac 11680

<210> 2
<211> 11824
<212> DNA
<213> Artificial Sequence

<220>
<223> (p)crtB_stop_pGWB405

<220>
<221> misc_feature
<222> (1)..(11824)

<400> 2
cttttcacgc ccttttaaat atccgattat tctaataaac gctcttttct cttaggttta 60

cccgccaata tatcctgtca aacactgata gtttaaactg aaggcgggaa acgacaatct 120

gatccaagct caagctgctc tagcattcgc cattcaggct gcgcaactgt tgggaagggc 180

gatcggtgcg ggcctcttcg ctattacgcc agctggcgaa agggggatgt gctgcaaggc 240

gattaagttg ggtaacgcca gggttttccc agtcacgacg ttgtaaaacg acggccagtg 300

ccaagcttgc atgcctgcag gtccccagat tagccttttc aatttcagaa agaatgctaa 360

cccacagatg gttagagagg cttacgcagc aggtctcatc aagacgatct acccgagcaa 420

taatctccag gaaatcaaat accttcccaa gaaggttaaa gatgcagtca aaagattcag 480

gactaactgc atcaagaaca cagagaaaga tatatttctc aagatcagaa gtactattcc 540

agtatggacg attcaaggct tgcttcacaa accaaggcaa gtaatagaga ttggagtctc 600

taaaaaggta gttcccactg aatcaaaggc catggagtca aagattcaaa tagaggacct 660

aacagaactc gccgtaaaga ctggcgaaca gttcatacag agtctcttac gactcaatga 720

caagaagaaa atcttcgtca acatggtgga gcacgacaca cttgtctact ccaaaaatat 780

caaagataca gtctcagaag accaaagggc aattgagact tttcaacaaa gggtaatatc 840

cggaaacctc ctcggattcc attgcccagc tatctgtcac tttattgtga agatagtgga 900

aaaggaaggt ggctcctaca aatgccatca ttgcgataaa ggaaaggcca tcgttgaaga 960

tgcctctgcc gacagtggtc ccaaagatgg acccccaccc acgaggagca tcgtggaaaa 1020

agaagacgtt ccaaccacgt cttcaaagca agtggattga tgtgatatct ccactgacgt 1080

aagggatgac gcacaatccc actatccttc gcaagaccct tcctctatat aaggaagttc 1140

atttcatttg gagagaacac gggggactct agagttatca acaagtttgt acaaaaaagc 1200

aggctatggc gactggagta ttgccagctc cggtttctgg gatcaagata ccggattcga 1260

aagtcgggtt tggtaaaagc atgaatcttg tgagaatttg tgatgttagg agtctaagat 1320

ctgctaggag aagagtttcg gttatccgga atatgaataa tccgtcgtta ctcaatcatg 1380

cggtcgaaac gatggcagtt ggctcgaaaa gttttgcgac agcctcaaag ttatttgatg 1440

caaaaacccg gcgcagcgta ctgatgctct acgcctggtg ccgccattgt gacgatgtta 1500

ttgacgatca gacgctgggc tttcaggccc ggcagcctgc cttacaaacg cccgaacaac 1560

gtctgatgca acttgagatg aaaacgcgcc aggcctatgc aggatcgcag atgcacgaac 1620

cggcgtttgc ggcttttcag gaagtggcta tggctcatga tatcgccccg gcttacgcgt 1680

ttgatcatct ggaaggcttc gccatggatg tacgcgaagc gcaatacagc caactggatg 1740

atacgctgcg ctattgctat cacgttgcag gcgttgtcgg cttgatgatg gcgcaaatca 1800

tgggcgtgcg ggataacgcc acgctggacc gcgcctgtga ccttgggctg gcatttcagt 1860

tgaccaatat tgctcgcgat attgtggacg atgcgcatgc gggccgctgt tatctgccgg 1920

caagctggct ggagcatgaa ggtctgaaca aagagaatta tgcggcacct gaaaaccgtc 1980

aggcgctgag ccgtatcgcc cgtcgtttgg tgcaggaagc agaaccttac tatttgtctg 2040

ccacagccgg cctggcaggg ttgcccctgc gttccgcctg ggcaatcgct acggcgaagc 2100

aggtttaccg gaaaataggt gtcaaagttg aacaggccgg tcagcaagcc tgggatcagc 2160

ggcagtcaac gaccacgccc gaaaaattaa cgctgctgct ggccgcctct ggtcaggccc 2220

ttacttcccg gatgcgggct catcctcccc gccctgcgca tctctggcag cgcccgctct 2280

agacccagct ttcttgtaca aagtggttcg atctagagga tccatggtga gcaagggcga 2340

ggagctgttc accggggtgg tgcccatcct ggtcgagctg gacggcgacg tgaacggcca 2400

caagttcagc gtgtccggcg agggcgaggg cgatgccacc tacggcaagc tgaccctgaa 2460

gttcatctgc accaccggca agctgcccgt gccctggccc accctcgtga ccaccttcac 2520

ctacggcgtg cagtgcttca gccgctaccc cgaccacatg aagcagcacg acttcttcaa 2580

gtccgccatg cccgaaggct acgtccagga gcgcaccatc ttcttcaagg acgacggcaa 2640

ctacaagacc cgcgccgagg tgaagttcga gggcgacacc ctggtgaacc gcatcgagct 2700

gaagggcatc gacttcaagg aggacggcaa catcctgggg cacaagctgg agtacaacta 2760

caacagccac aacgtctata tcatggccga caagcagaag aacggcatca aggtgaactt 2820

caagatccgc cacaacatcg aggacggcag cgtgcagctc gccgaccact accagcagaa 2880

cacccccatc ggcgacggcc ccgtgctgct gcccgacaac cactacctga gcacccagtc 2940

cgccctgagc aaagacccca acgagaagcg cgatcacatg gtcctgctgg agttcgtgac 3000

cgccgccggg atcactcacg gcatggacga gctgtacaag taaagcggcc cgagctcgaa 3060

tttccccgat cgttcaaaca tttggcaata aagtttctta agattgaatc ctgttgccgg 3120

tcttgcgatg attatcatat aatttctgtt gaattacgtt aagcatgtaa taattaacat 3180

gtaatgcatg acgttattta tgagatgggt ttttatgatt agagtcccgc aattatacat 3240

ttaatacgcg atagaaaaca aaatatagcg cgcaaactag gataaattat cgcgcgcggt 3300

gtcatctatg ttactagatc gggaattggt tccggaacca attcgtaatc atggtcatag 3360

ctgtttcctg tgtgaaattg ttatccgctc acaattccac acaacatacg agccggaagc 3420

ataaagtgta aagcctgggg tgcctaatga gtgagctaac tcacattagg ctgaattagg 3480

cgcgcctatt tctgaattcc cgatctagta acatagatga caccgcgcgc gataatttat 3540

cctagtttgc gcgctatatt ttgttttcta tcgcgtatta aatgtataat tgcgggactc 3600

taatcataaa aacccatctc ataaataacg tcatgcatta catgttaatt attacatgct 3660

taacgtaatt caacagaaat tatatgataa tcatcgcaag accggcaaca ggattcaatc 3720

ttaagaaact ttattgccaa atgtttgaac gatcggggat catccgggtc tgtggcggga 3780

actccacgaa aatatccgaa cgcagcaaga tatcgcggtg catctcggtc ttgcctgggc 3840

agtcgccgcc gacgccgttg atgtggacgc cgggcccgat catattgtcg ctcaggatcg 3900

tggcgttgtg cttgtcggcc gttgctgtcg taatgatatc ggcaccttcg accgcctgtt 3960

ccgcagagat cccgtgggcg aagaactcca gcatgagatc cccgcgctgg aggatcatcc 4020

agccggcgtc ccggaaaacg attccgaagc ccaacctttc atagaaggcg gcggtggaat 4080

cgaaatctcg tgatggcagg ttgggcgtcg cttggtcggt catttcgaac cccagagtcc 4140

cgctcagaag aactcgtcaa gaaggcgata gaaggcgatg cgctgcgaat cgggagcggc 4200

gataccgtaa agcacgagga agcggtcagc ccattcgccg ccaagctctt cagcaatatc 4260

acgggtagcc aacgctatgt cctgatagcg gtccgccaca cccagccggc cacagtcgat 4320

gaatccagaa aagcggccat tttccaccat gatattcggc aagcaggcat cgccatgggt 4380

cacgacgaga tcatcgccgt cgggcatgcg cgccttgagc ctggcgaaca gttcggctgg 4440

cgcgagcccc tgatgctctt cgtccagatc atcctgatcg acaagaccgg cttccatccg 4500

agtacgtgct cgctcgatgc gatgtttcgc ttggtggtcg aatgggcagg tagccggatc 4560

aagcgtatgc agccgccgca ttgcatcagc catgatggat actttctcgg caggagcaag 4620

gtgagatgac aggagatcct gccccggcac ttcgcccaat agcagccagt cccttcccgc 4680

ttcagtgaca acgtcgagca cagctgcgca aggaacgccc gtcgtggcca gccacgatag 4740

ccgcgctgcc tcgtcctgca gttcattcag ggcaccggac aggtcggtct tgacaaaaag 4800

aaccgggcgc ccctgcgctg acagccggaa cacggcggca tcagagcagc cgattgtctg 4860

ttgtgcccag tcatagccga atagcctctc cacccaagcg gccggagaac ctgcgtgcaa 4920

tccatcttgt tcaatcatgc gaaacgatcc agatccggtg cagattattt ggattgagag 4980

tgaatatgag actctaattg gataccgagg ggaatttatg gaacgtcagt ggagcatttt 5040

tgacaagaaa tatttgctag ctgatagtga ccttaggcga cttttgaacg cgcaataatg 5100

gtttctgacg tatgtgctta gctcattaaa ctccagaaac ccgcggctga gtggctcctt 5160

caacgttgcg gttctgtcag ttccaaacgt aaaacggctt gtcccgcgtc atcggcgggg 5220

gtcataacgt gactccctta attctccgct catgatgaat tcagaaataa attcagccta 5280

attcggcgtt aattcagtac attaaaaacg tccgcaatgt gttattaagt tgtctaagcg 5340

tcaatttgtt tacaccacaa tatatcctgc caccagccag ccaacagctc cccgaccggc 5400

agctcggcac aaaatcacca ctcgatacag gcagcccatc agtccgggac ggcgtcagcg 5460

ggagagccgt tgtaaggcgg cagactttgc tcatgttacc gatgctattc ggaagaacgg 5520

caactaagct gccgggtttg aaacacggat gatctcgcgg agggtagcat gttgattgta 5580

acgatgacag agcgttgctg cctgtgatca attcgggcac gaacccagtg gacataagcc 5640

tgttcggttc gtaagctgta atgcaagtag cgtatgcgct cacgcaactg gtccagaacc 5700

ttgaccgaac gcagcggtgg taacggcgca gtggcggttt tcatggcttg ttatgactgt 5760

tttttttggg tacagtctat gcctcgggca tccaagcagc aagcgcgtta cgccgtgggt 5820

cgatgtttga tgttatggag cagcaacgat gttacgcagc agggcagtcg ccctaaaaca 5880

aagttaaaca tcatggggga agcggtgatc gccgaagtat cgactcaact atcagaggta 5940

gttggcgtca tcgagcgcca tctcgaaccg acgttgctgg ccgtacattt gtacggctcc 6000

gcagtggatg gcggcctgaa gccacacagt gatattgatt tgctggttac ggtgaccgta 6060

aggcttgatg aaacaacgcg gcgagctttg atcaacgacc ttttggaaac ttcggcttcc 6120

cctggagaga gcgagattct ccgcgctgta gaagtcacca ttgttgtgca cgacgacatc 6180

attccgtggc gttatccagc taagcgcgaa ctgcaatttg gagaatggca gcgcaatgac 6240

attcttgcag gtatcttcga gccagccacg atcgacattg atctggctat cttgctgaca 6300

aaagcaagag aacatagcgt tgccttggta ggtccagcgg cggaggaact ctttgatccg 6360

gttcctgaac aggatctatt tgaggcgcta aatgaaacct taacgctatg gaactcgccg 6420

cccgactggg ctggcgatga gcgaaatgta gtgcttacgt tgtcccgcat ttggtacagc 6480

gcagtaaccg gcaaaatcgc gccgaaggat gtcgctgccg actgggcaat ggagcgcctg 6540

ccggcccagt atcagcccgt catacttgaa gctagacagg cttatcttgg acaagaagaa 6600

gatcgcttgg cctcgcgcgc agatcagttg gaagaatttg tccactacgt gaaaggcgag 6660

atcaccaagg tagtcggcaa ataatgtcta gctagaaatt cgttcaagcc gacgccgctt 6720

cgcggcgcgg cttaactcaa gcgttagatg cactaagcac ataattgctc acagccaaac 6780

tatcaggtca agtctgcttt tattattttt aagcgtgcat aataagccct acacaaattg 6840

ggagatatat catgcatgac caaaatccct taacgtgagt tttcgttcca ctgagcgtca 6900

gaccccgtag aaaagatcaa aggatcttct tgagatcctt tttttctgcg cgtaatctgc 6960

tgcttgcaaa caaaaaaacc accgctacca gcggtggttt gtttgccgga tcaagagcta 7020

ccaactcttt ttccgaaggt aactggcttc agcagagcgc agataccaaa tactgtcctt 7080

ctagtgtagc cgtagttagg ccaccacttc aagaactctg tagcaccgcc tacatacctc 7140

gctctgctaa tcctgttacc agtggctgct gccagtggcg ataagtcgtg tcttaccggg 7200

ttggactcaa gacgatagtt accggataag gcgcagcggt cgggctgaac ggggggttcg 7260

tgcacacagc ccagcttgga gcgaacgacc tacaccgaac tgagatacct acagcgtgag 7320

ctatgagaaa gcgccacgct tcccgaaggg agaaaggcgg acaggtatcc ggtaagcggc 7380

agggtcggaa caggagagcg cacgagggag cttccagggg gaaacgcctg gtatctttat 7440

agtcctgtcg ggtttcgcca cctctgactt gagcgtcgat ttttgtgatg ctcgtcaggg 7500

gggcggagcc tatggaaaaa cgccagcaac gcggcctttt tacggttcct ggccttttgc 7560

tggccttttg ctcacatgtt ctttcctgcg ttatcccctg attctgtgga taaccgtatt 7620

accgcctttg agtgagctga taccgctcgc cgcagccgaa cgaccgagcg cagcgagtca 7680

gtgagcgagg aagcggaaga gcgcctgatg cggtattttc tccttacgca tctgtgcggt 7740

atttcacacc gcatatggtg cactctcagt acaatctgct ctgatgccgc atagttaagc 7800

cagtatacac tccgctatcg ctacgtgact gggtcatggc tgcgccccga cacccgccaa 7860

cacccgctga cgcgccctga cgggcttgtc tgctcccggc atccgcttac agacaagctg 7920

tgaccgtctc cgggagctgc atgtgtcaga ggttttcacc gtcatcaccg aaacgcgcga 7980

ggcagggtgc cttgatgtgg gcgccggcgg tcgagtggcg acggcgcggc ttgtccgcgc 8040

cctggtagat tgcctggccg taggccagcc atttttgagc ggccagcggc cgcgataggc 8100

cgacgcgaag cggcggggcg tagggagcgc agcgaccgaa gggtaggcgc tttttgcagc 8160

tcttcggctg tgcgctggcc agacagttat gcacaggcca ggcgggtttt aagagtttta 8220

ataagtttta aagagtttta ggcggaaaaa tcgccttttt tctcttttat atcagtcact 8280

tacatgtgtg accggttccc aatgtacggc tttgggttcc caatgtacgg gttccggttc 8340

ccaatgtacg gctttgggtt cccaatgtac gtgctatcca caggaaagag accttttcga 8400

cctttttccc ctgctagggc aatttgccct agcatctgct ccgtacatta ggaaccggcg 8460

gatgcttcgc cctcgatcag gttgcggtag cgcatgacta ggatcgggcc agcctgcccc 8520

gcctcctcct tcaaatcgta ctccggcagg tcatttgacc cgatcagctt gcgcacggtg 8580

aaacagaact tcttgaactc tccggcgctg ccactgcgtt cgtagatcgt cttgaacaac 8640

catctggctt ctgccttgcc tgcggcgcgg cgtgccaggc ggtagagaaa acggccgatg 8700

ccgggatcga tcaaaaagta atcggggtga accgtcagca cgtccgggtt cttgccttct 8760

gtgatctcgc ggtacatcca atcagctagc tcgatctcga tgtactccgg ccgcccggtt 8820

tcgctcttta cgatcttgta gcggctaatc aaggcttcac cctcggatac cgtcaccagg 8880

cggccgttct tggccttctt cgtacgctgc atggcaacgt gcgtggtgtt taaccgaatg 8940

caggtttcta ccaggtcgtc tttctgcttt ccgccatcgg ctcgccggca gaacttgagt 9000

acgtccgcaa cgtgtggacg gaacacgcgg ccgggcttgt ctcccttccc ttcccggtat 9060

cggttcatgg attcggttag atgggaaacc gccatcagta ccaggtcgta atcccacaca 9120

ctggccatgc cggccggccc tgcggaaacc tctacgtgcc cgtctggaag ctcgtagcgg 9180

atcacctcgc cagctcgtcg gtcacgcttc gacagacgga aaacggccac gtccatgatg 9240

ctgcgactat cgcgggtgcc cacgtcatag agcatcggaa cgaaaaaatc tggttgctcg 9300

tcgcccttgg gcggcttcct aatcgacggc gcaccggctg ccggcggttg ccgggattct 9360

ttgcggattc gatcagcggc cgcttgccac gattcaccgg ggcgtgcttc tgcctcgatg 9420

cgttgccgct gggcggcctg cgcggccttc aacttctcca ccaggtcatc acccagcgcc 9480

gcgccgattt gtaccgggcc ggatggtttg cgaccgctca cgccgattcc tcgggcttgg 9540

gggttccagt gccattgcag ggccggcaga caacccagcc gcttacgcct ggccaaccgc 9600

ccgttcctcc acacatgggg cattccacgg cgtcggtgcc tggttgttct tgattttcca 9660

tgccgcctcc tttagccgct aaaattcatc tactcattta ttcatttgct catttactct 9720

ggtagctgcg cgatgtattc agatagcagc tcggtaatgg tcttgccttg gcgtaccgcg 9780

tacatcttca gcttggtgtg atcctccgcc ggcaactgaa agttgacccg cttcatggct 9840

ggcgtgtctg ccaggctggc caacgttgca gccttgctgc tgcgtgcgct cggacggccg 9900

gcacttagcg tgtttgtgct tttgctcatt ttctctttac ctcattaact caaatgagtt 9960

ttgatttaat ttcagcggcc agcgcctgga cctcgcgggc agcgtcgccc tcgggttctg 10020

attcaagaac ggttgtgccg gcggcggcag tgcctgggta gctcacgcgc tgcgtgatac 10080

gggactcaag aatgggcagc tcgtacccgg ccagcgcctc ggcaacctca ccgccgatgc 10140

gcgtgccttt gatcgcccgc gacacgacaa aggccgcttg tagccttcca tccgtgacct 10200

caatgcgctg cttaaccagc tccaccaggt cggcggtggc ccatatgtcg taagggcttg 10260

gctgcaccgg aatcagcacg aagtcggctg ccttgatcgc ggacacagcc aagtccgccg 10320

cctggggcgc tccgtcgatc actacgaagt cgcgccggcc gatggccttc acgtcgcggt 10380

caatcgtcgg gcggtcgatg ccgacaacgg ttagcggttg atcttcccgc acggccgccc 10440

aatcgcgggc actgccctgg ggatcggaat cgactaacag aacatcggcc ccggcgagtt 10500

gcagggcgcg ggctagatgg gttgcgatgg tcgtcttgcc tgacccgcct ttctggttaa 10560

gtacagcgat aaccttcatg cgttcccctt gcgtatttgt ttatttactc atcgcatcat 10620

atacgcagcg accgcatgac gcaagctgtt ttactcaaat acacatcacc tttttagacg 10680

gcggcgctcg gtttcttcag cggccaagct ggccggccag gccgccagct tggcatcaga 10740

caaaccggcc aggatttcat gcagccgcac ggttgagacg tgcgcgggcg gctcgaacac 10800

gtacccggcc gcgatcatct ccgcctcgat ctcttcggta atgaaaaacg gttcgtcctg 10860

gccgtcctgg tgcggtttca tgcttgttcc tcttggcgtt cattctcggc ggccgccagg 10920

gcgtcggcct cggtcaatgc gtcctcacgg aaggcaccgc gccgcctggc ctcggtgggc 10980

gtcacttcct cgctgcgctc aagtgcgcgg tacagggtcg agcgatgcac gccaagcagt 11040

gcagccgcct ctttcacggt gcggccttcc tggtcgatca gctcgcgggc gtgcgcgatc 11100

tgtgccgggg tgagggtagg gcgggggcca aacttcacgc ctcgggcctt ggcggcctcg 11160

cgcccgctcc gggtgcggtc gatgattagg gaacgctcga actcggcaat gccggcgaac 11220

acggtcaaca ccatgcggcc ggccggcgtg gtggtgtcgg cccacggctc tgccaggcta 11280

cgcaggcccg cgccggcctc ctggatgcgc tcggcaatgt ccagtaggtc gcgggtgctg 11340

cgggccaggc ggtctagcct ggtcactgtc acaacgtcgc cagggcgtag gtggtcaagc 11400

atcctggcca gctccgggcg gtcgcgcctg gtgccggtga tcttctcgga aaacagcttg 11460

gtgcagccgg ccgcgtgcag ttcggcccgt tggttggtca agtcctggtc gtcggtgctg 11520

acgcgggcat agcccagcag gccagcggcg gcgctcttgt tcatggcgta atgtctccgg 11580

ttctagtcgc aagtattcta ctttatgcga ctaaaacacg cgacaagaaa acgccaggaa 11640

aagggcaggg cggcagcctg tcgcgtaact taggacttgt gcgacatgtc gttttcagaa 11700

gacggctgca ctgaacgtca gaagccgact gcactatagc agcggagggg ttggatcaaa 11760

gtactttgat cccgagggga accctgtggt tggcatgcac atacaaatgg acgaacggat 11820

aaac 11824

<210> 3
<211> 11679
<212> DNA
<213> Artificial Sequence

<220>
<223> crtB_stop_pGWB405

<220>
<221> misc_feature
<222> (1)..(11679)

<400> 3
cttttcacgc ccttttaaat atccgattat tctaataaac gctcttttct cttaggttta 60

cccgccaata tatcctgtca aacactgata gtttaaactg aaggcgggaa acgacaatct 120

gatccaagct caagctgctc tagcattcgc cattcaggct gcgcaactgt tgggaagggc 180

gatcggtgcg ggcctcttcg ctattacgcc agctggcgaa agggggatgt gctgcaaggc 240

gattaagttg ggtaacgcca gggttttccc agtcacgacg ttgtaaaacg acggccagtg 300

ccaagcttgc atgcctgcag gtccccagat tagccttttc aatttcagaa agaatgctaa 360

cccacagatg gttagagagg cttacgcagc aggtctcatc aagacgatct acccgagcaa 420

taatctccag gaaatcaaat accttcccaa gaaggttaaa gatgcagtca aaagattcag 480

gactaactgc atcaagaaca cagagaaaga tatatttctc aagatcagaa gtactattcc 540

agtatggacg attcaaggct tgcttcacaa accaaggcaa gtaatagaga ttggagtctc 600

taaaaaggta gttcccactg aatcaaaggc catggagtca aagattcaaa tagaggacct 660

aacagaactc gccgtaaaga ctggcgaaca gttcatacag agtctcttac gactcaatga 720

caagaagaaa atcttcgtca acatggtgga gcacgacaca cttgtctact ccaaaaatat 780

caaagataca gtctcagaag accaaagggc aattgagact tttcaacaaa gggtaatatc 840

cggaaacctc ctcggattcc attgcccagc tatctgtcac tttattgtga agatagtgga 900

aaaggaaggt ggctcctaca aatgccatca ttgcgataaa ggaaaggcca tcgttgaaga 960

tgcctctgcc gacagtggtc ccaaagatgg acccccaccc acgaggagca tcgtggaaaa 1020

agaagacgtt ccaaccacgt cttcaaagca agtggattga tgtgatatct ccactgacgt 1080

aagggatgac gcacaatccc actatccttc gcaagaccct tcctctatat aaggaagttc 1140

atttcatttg gagagaacac gggggactct agagttatca acaagtttgt acaaaaaagc 1200

aggcttcatg aataatccgt cgttactcaa tcatgcggtc gaaacgatgg cagttggctc 1260

gaaaagtttt gcgacagcct caaagttatt tgatgcaaaa acccggcgca gcgtactgat 1320

gctctacgcc tggtgccgcc attgtgacga tgttattgac gatcagacgc tgggctttca 1380

ggcccggcag cctgccttac aaacgcccga acaacgtctg atgcaacttg agatgaaaac 1440

gcgccaggcc tatgcaggat cgcagatgca cgaaccggcg tttgcggctt ttcaggaagt 1500

ggctatggct catgatatcg ccccggctta cgcgtttgat catctggaag gcttcgccat 1560

ggatgtacgc gaagcgcaat acagccaact ggatgatacg ctgcgctatt gctatcacgt 1620

tgcaggcgtt gtcggcttga tgatggcgca aatcatgggc gtgcgggata acgccacgct 1680

ggaccgcgcc tgtgaccttg ggctggcatt tcagttgacc aatattgctc gcgatattgt 1740

ggacgatgcg catgcgggcc gctgttatct gccggcaagc tggctggagc atgaaggtct 1800

gaacaaagag aattatgcgg cacctgaaaa ccgtcaggcg ctgagccgta tcgcccgtcg 1860

tttggtgcag gaagcagaac cttactattt gtctgccaca gccggcctgg cagggttgcc 1920

cctgcgttcc gcctgggcaa tcgctacggc gaagcaggtt taccggaaaa taggtgtcaa 1980

agttgaacag gccggtcagc aagcctggga tcagcggcag tcaacgacca cgcccgaaaa 2040

attaacgctg ctgctggccg cctctggtca ggcccttact tcccggatgc gggctcatcc 2100

tccccgccct gcgcatctct ggcagcgccc gctctagacc cagctttctt gtacaaagtg 2160

gttcgatcta gaggatccat ggtgagcaag ggcgaggagc tgttcaccgg ggtggtgccc 2220

atcctggtcg agctggacgg cgacgtgaac ggccacaagt tcagcgtgtc cggcgagggc 2280

gagggcgatg ccacctacgg caagctgacc ctgaagttca tctgcaccac cggcaagctg 2340

cccgtgccct ggcccaccct cgtgaccacc ttcacctacg gcgtgcagtg cttcagccgc 2400

taccccgacc acatgaagca gcacgacttc ttcaagtccg ccatgcccga aggctacgtc 2460

caggagcgca ccatcttctt caaggacgac ggcaactaca agacccgcgc cgaggtgaag 2520

ttcgagggcg acaccctggt gaaccgcatc gagctgaagg gcatcgactt caaggaggac 2580

ggcaacatcc tggggcacaa gctggagtac aactacaaca gccacaacgt ctatatcatg 2640

gccgacaagc agaagaacgg catcaaggtg aacttcaaga tccgccacaa catcgaggac 2700

ggcagcgtgc agctcgccga ccactaccag cagaacaccc ccatcggcga cggccccgtg 2760

ctgctgcccg acaaccacta cctgagcacc cagtccgccc tgagcaaaga ccccaacgag 2820

aagcgcgatc acatggtcct gctggagttc gtgaccgccg ccgggatcac tcacggcatg 2880

gacgagctgt acaagtaaag cggcccgagc tcgaatttcc ccgatcgttc aaacatttgg 2940

caataaagtt tcttaagatt gaatcctgtt gccggtcttg cgatgattat catataattt 3000

ctgttgaatt acgttaagca tgtaataatt aacatgtaat gcatgacgtt atttatgaga 3060

tgggttttta tgattagagt cccgcaatta tacatttaat acgcgataga aaacaaaata 3120

tagcgcgcaa actaggataa attatcgcgc gcggtgtcat ctatgttact agatcgggaa 3180

ttggttccgg aaccaattcg taatcatggt catagctgtt tcctgtgtga aattgttatc 3240

cgctcacaat tccacacaac atacgagccg gaagcataaa gtgtaaagcc tggggtgcct 3300

aatgagtgag ctaactcaca ttaggctgaa ttaggcgcgc ctatttctga attcccgatc 3360

tagtaacata gatgacaccg cgcgcgataa tttatcctag tttgcgcgct atattttgtt 3420

ttctatcgcg tattaaatgt ataattgcgg gactctaatc ataaaaaccc atctcataaa 3480

taacgtcatg cattacatgt taattattac atgcttaacg taattcaaca gaaattatat 3540

gataatcatc gcaagaccgg caacaggatt caatcttaag aaactttatt gccaaatgtt 3600

tgaacgatcg gggatcatcc gggtctgtgg cgggaactcc acgaaaatat ccgaacgcag 3660

caagatatcg cggtgcatct cggtcttgcc tgggcagtcg ccgccgacgc cgttgatgtg 3720

gacgccgggc ccgatcatat tgtcgctcag gatcgtggcg ttgtgcttgt cggccgttgc 3780

tgtcgtaatg atatcggcac cttcgaccgc ctgttccgca gagatcccgt gggcgaagaa 3840

ctccagcatg agatccccgc gctggaggat catccagccg gcgtcccgga aaacgattcc 3900

gaagcccaac ctttcataga aggcggcggt ggaatcgaaa tctcgtgatg gcaggttggg 3960

cgtcgcttgg tcggtcattt cgaaccccag agtcccgctc agaagaactc gtcaagaagg 4020

cgatagaagg cgatgcgctg cgaatcggga gcggcgatac cgtaaagcac gaggaagcgg 4080

tcagcccatt cgccgccaag ctcttcagca atatcacggg tagccaacgc tatgtcctga 4140

tagcggtccg ccacacccag ccggccacag tcgatgaatc cagaaaagcg gccattttcc 4200

accatgatat tcggcaagca ggcatcgcca tgggtcacga cgagatcatc gccgtcgggc 4260

atgcgcgcct tgagcctggc gaacagttcg gctggcgcga gcccctgatg ctcttcgtcc 4320

agatcatcct gatcgacaag accggcttcc atccgagtac gtgctcgctc gatgcgatgt 4380

ttcgcttggt ggtcgaatgg gcaggtagcc ggatcaagcg tatgcagccg ccgcattgca 4440

tcagccatga tggatacttt ctcggcagga gcaaggtgag atgacaggag atcctgcccc 4500

ggcacttcgc ccaatagcag ccagtccctt cccgcttcag tgacaacgtc gagcacagct 4560

gcgcaaggaa cgcccgtcgt ggccagccac gatagccgcg ctgcctcgtc ctgcagttca 4620

ttcagggcac cggacaggtc ggtcttgaca aaaagaaccg ggcgcccctg cgctgacagc 4680

cggaacacgg cggcatcaga gcagccgatt gtctgttgtg cccagtcata gccgaatagc 4740

ctctccaccc aagcggccgg agaacctgcg tgcaatccat cttgttcaat catgcgaaac 4800

gatccagatc cggtgcagat tatttggatt gagagtgaat atgagactct aattggatac 4860

cgaggggaat ttatggaacg tcagtggagc atttttgaca agaaatattt gctagctgat 4920

agtgacctta ggcgactttt gaacgcgcaa taatggtttc tgacgtatgt gcttagctca 4980

ttaaactcca gaaacccgcg gctgagtggc tccttcaacg ttgcggttct gtcagttcca 5040

aacgtaaaac ggcttgtccc gcgtcatcgg cgggggtcat aacgtgactc ccttaattct 5100

ccgctcatga tgaattcaga aataaattca gcctaattcg gcgttaattc agtacattaa 5160

aaacgtccgc aatgtgttat taagttgtct aagcgtcaat ttgtttacac cacaatatat 5220

cctgccacca gccagccaac agctccccga ccggcagctc ggcacaaaat caccactcga 5280

tacaggcagc ccatcagtcc gggacggcgt cagcgggaga gccgttgtaa ggcggcagac 5340

tttgctcatg ttaccgatgc tattcggaag aacggcaact aagctgccgg gtttgaaaca 5400

cggatgatct cgcggagggt agcatgttga ttgtaacgat gacagagcgt tgctgcctgt 5460

gatcaattcg ggcacgaacc cagtggacat aagcctgttc ggttcgtaag ctgtaatgca 5520

agtagcgtat gcgctcacgc aactggtcca gaaccttgac cgaacgcagc ggtggtaacg 5580

gcgcagtggc ggttttcatg gcttgttatg actgtttttt tggggtacag tctatgcctc 5640

gggcatccaa gcagcaagcg cgttacgccg tgggtcgatg tttgatgtta tggagcagca 5700

acgatgttac gcagcagggc agtcgcccta aaacaaagtt aaacatcatg ggggaagcgg 5760

tgatcgccga agtatcgact caactatcag aggtagttgg cgtcatcgag cgccatctcg 5820

aaccgacgtt gctggccgta catttgtacg gctccgcagt ggatggcggc ctgaagccac 5880

acagtgatat tgatttgctg gttacggtga ccgtaaggct tgatgaaaca acgcggcgag 5940

ctttgatcaa cgaccttttg gaaacttcgg cttcccctgg agagagcgag attctccgcg 6000

ctgtagaagt caccattgtt gtgcacgacg acatcattcc gtggcgttat ccagctaagc 6060

gcgaactgca atttggagaa tggcagcgca atgacattct tgcaggtatc ttcgagccag 6120

ccacgatcga cattgatctg gctatcttgc tgacaaaagc aagagaacat agcgttgcct 6180

tggtaggtcc agcggcggag gaactctttg atccggttcc tgaacaggat ctatttgagg 6240

cgctaaatga aaccttaacg ctatggaact cgccgcccga ctgggctggc gatgagcgaa 6300

atgtagtgct tacgttgtcc cgcatttggt acagcgcagt aaccggcaaa atcgcgccga 6360

aggatgtcgc tgccgactgg gcaatggagc gcctgccggc ccagtatcag cccgtcatac 6420

ttgaagctag acaggcttat cttggacaag aagaagatcg cttggcctcg cgcgcagatc 6480

agttggaaga atttgtccac tacgtgaaag gcgagatcac caaggtagtc ggcaaataat 6540

gtctagctag aaattcgttc aagccgacgc cgcttcgcgg cgcggcttaa ctcaagcgtt 6600

agatgcacta agcacataat tgctcacagc caaactatca ggtcaagtct gcttttatta 6660

tttttaagcg tgcataataa gccctacaca aattgggaga tatatcatgc atgaccaaaa 6720

tcccttaacg tgagttttcg ttccactgag cgtcagaccc cgtagaaaag atcaaaggat 6780

cttcttgaga tccttttttt ctgcgcgtaa tctgctgctt gcaaacaaaa aaaccaccgc 6840

taccagcggt ggtttgtttg ccggatcaag agctaccaac tctttttccg aaggtaactg 6900

gcttcagcag agcgcagata ccaaatactg tccttctagt gtagccgtag ttaggccacc 6960

acttcaagaa ctctgtagca ccgcctacat acctcgctct gctaatcctg ttaccagtgg 7020

ctgctgccag tggcgataag tcgtgtctta ccgggttgga ctcaagacga tagttaccgg 7080

ataaggcgca gcggtcgggc tgaacggggg gttcgtgcac acagcccagc ttggagcgaa 7140

cgacctacac cgaactgaga tacctacagc gtgagctatg agaaagcgcc acgcttcccg 7200

aagggagaaa ggcggacagg tatccggtaa gcggcagggt cggaacagga gagcgcacga 7260

gggagcttcc agggggaaac gcctggtatc tttatagtcc tgtcgggttt cgccacctct 7320

gacttgagcg tcgatttttg tgatgctcgt caggggggcg gagcctatgg aaaaacgcca 7380

gcaacgcggc ctttttacgg ttcctggcct tttgctggcc ttttgctcac atgttctttc 7440

ctgcgttatc ccctgattct gtggataacc gtattaccgc ctttgagtga gctgataccg 7500

ctcgccgcag ccgaacgacc gagcgcagcg agtcagtgag cgaggaagcg gaagagcgcc 7560

tgatgcggta ttttctcctt acgcatctgt gcggtatttc acaccgcata tggtgcactc 7620

tcagtacaat ctgctctgat gccgcatagt taagccagta tacactccgc tatcgctacg 7680

tgactgggtc atggctgcgc cccgacaccc gccaacaccc gctgacgcgc cctgacgggc 7740

ttgtctgctc ccggcatccg cttacagaca agctgtgacc gtctccggga gctgcatgtg 7800

tcagaggttt tcaccgtcat caccgaaacg cgcgaggcag ggtgccttga tgtgggcgcc 7860

ggcggtcgag tggcgacggc gcggcttgtc cgcgccctgg tagattgcct ggccgtaggc 7920

cagccatttt tgagcggcca gcggccgcga taggccgacg cgaagcggcg gggcgtaggg 7980

agcgcagcga ccgaagggta ggcgcttttt gcagctcttc ggctgtgcgc tggccagaca 8040

gttatgcaca ggccaggcgg gttttaagag ttttaataag ttttaaagag ttttaggcgg 8100

aaaaatcgcc ttttttctct tttatatcag tcacttacat gtgtgaccgg ttcccaatgt 8160

acggctttgg gttcccaatg tacgggttcc ggttcccaat gtacggcttt gggttcccaa 8220

tgtacgtgct atccacagga aagagacctt ttcgaccttt ttcccctgct agggcaattt 8280

gccctagcat ctgctccgta cattaggaac cggcggatgc ttcgccctcg atcaggttgc 8340

ggtagcgcat gactaggatc gggccagcct gccccgcctc ctccttcaaa tcgtactccg 8400

gcaggtcatt tgacccgatc agcttgcgca cggtgaaaca gaacttcttg aactctccgg 8460

cgctgccact gcgttcgtag atcgtcttga acaaccatct ggcttctgcc ttgcctgcgg 8520

cgcggcgtgc caggcggtag agaaaacggc cgatgccggg atcgatcaaa aagtaatcgg 8580

ggtgaaccgt cagcacgtcc gggttcttgc cttctgtgat ctcgcggtac atccaatcag 8640

ctagctcgat ctcgatgtac tccggccgcc cggtttcgct ctttacgatc ttgtagcggc 8700

taatcaaggc ttcaccctcg gataccgtca ccaggcggcc gttcttggcc ttcttcgtac 8760

gctgcatggc aacgtgcgtg gtgtttaacc gaatgcaggt ttctaccagg tcgtctttct 8820

gctttccgcc atcggctcgc cggcagaact tgagtacgtc cgcaacgtgt ggacggaaca 8880

cgcggccggg cttgtctccc ttcccttccc ggtatcggtt catggattcg gttagatggg 8940

aaaccgccat cagtaccagg tcgtaatccc acacactggc catgccggcc ggccctgcgg 9000

aaacctctac gtgcccgtct ggaagctcgt agcggatcac ctcgccagct cgtcggtcac 9060

gcttcgacag acggaaaacg gccacgtcca tgatgctgcg actatcgcgg gtgcccacgt 9120

catagagcat cggaacgaaa aaatctggtt gctcgtcgcc cttgggcggc ttcctaatcg 9180

acggcgcacc ggctgccggc ggttgccggg attctttgcg gattcgatca gcggccgctt 9240

gccacgattc accggggcgt gcttctgcct cgatgcgttg ccgctgggcg gcctgcgcgg 9300

ccttcaactt ctccaccagg tcatcaccca gcgccgcgcc gatttgtacc gggccggatg 9360

gtttgcgacc gctcacgccg attcctcggg cttgggggtt ccagtgccat tgcagggccg 9420

gcagacaacc cagccgctta cgcctggcca accgcccgtt cctccacaca tggggcattc 9480

cacggcgtcg gtgcctggtt gttcttgatt ttccatgccg cctcctttag ccgctaaaat 9540

tcatctactc atttattcat ttgctcattt actctggtag ctgcgcgatg tattcagata 9600

gcagctcggt aatggtcttg ccttggcgta ccgcgtacat cttcagcttg gtgtgatcct 9660

ccgccggcaa ctgaaagttg acccgcttca tggctggcgt gtctgccagg ctggccaacg 9720

ttgcagcctt gctgctgcgt gcgctcggac ggccggcact tagcgtgttt gtgcttttgc 9780

tcattttctc tttacctcat taactcaaat gagttttgat ttaatttcag cggccagcgc 9840

ctggacctcg cgggcagcgt cgccctcggg ttctgattca agaacggttg tgccggcggc 9900

ggcagtgcct gggtagctca cgcgctgcgt gatacgggac tcaagaatgg gcagctcgta 9960

cccggccagc gcctcggcaa cctcaccgcc gatgcgcgtg cctttgatcg cccgcgacac 10020

gacaaaggcc gcttgtagcc ttccatccgt gacctcaatg cgctgcttaa ccagctccac 10080

caggtcggcg gtggcccata tgtcgtaagg gcttggctgc accggaatca gcacgaagtc 10140

ggctgccttg atcgcggaca cagccaagtc cgccgcctgg ggcgctccgt cgatcactac 10200

gaagtcgcgc cggccgatgg ccttcacgtc gcggtcaatc gtcgggcggt cgatgccgac 10260

aacggttagc ggttgatctt cccgcacggc cgcccaatcg cgggcactgc cctggggatc 10320

ggaatcgact aacagaacat cggccccggc gagttgcagg gcgcgggcta gatgggttgc 10380

gatggtcgtc ttgcctgacc cgcctttctg gttaagtaca gcgataacct tcatgcgttc 10440

cccttgcgta tttgtttatt tactcatcgc atcatatacg cagcgaccgc atgacgcaag 10500

ctgttttact caaatacaca tcaccttttt agacggcggc gctcggtttc ttcagcggcc 10560

aagctggccg gccaggccgc cagcttggca tcagacaaac cggccaggat ttcatgcagc 10620

cgcacggttg agacgtgcgc gggcggctcg aacacgtacc cggccgcgat catctccgcc 10680

tcgatctctt cggtaatgaa aaacggttcg tcctggccgt cctggtgcgg tttcatgctt 10740

gttcctcttg gcgttcattc tcggcggccg ccagggcgtc ggcctcggtc aatgcgtcct 10800

cacggaaggc accgcgccgc ctggcctcgg tgggcgtcac ttcctcgctg cgctcaagtg 10860

cgcggtacag ggtcgagcga tgcacgccaa gcagtgcagc cgcctctttc acggtgcggc 10920

cttcctggtc gatcagctcg cgggcgtgcg cgatctgtgc cggggtgagg gtagggcggg 10980

ggccaaactt cacgcctcgg gccttggcgg cctcgcgccc gctccgggtg cggtcgatga 11040

ttagggaacg ctcgaactcg gcaatgccgg cgaacacggt caacaccatg cggccggccg 11100

gcgtggtggt gtcggcccac ggctctgcca ggctacgcag gcccgcgccg gcctcctgga 11160

tgcgctcggc aatgtccagt aggtcgcggg tgctgcgggc caggcggtct agcctggtca 11220

ctgtcacaac gtcgccaggg cgtaggtggt caagcatcct ggccagctcc gggcggtcgc 11280

gcctggtgcc ggtgatcttc tcggaaaaca gcttggtgca gccggccgcg tgcagttcgg 11340

cccgttggtt ggtcaagtcc tggtcgtcgg tgctgacgcg ggcatagccc agcaggccag 11400

cggcggcgct cttgttcatg gcgtaatgtc tccggttcta gtcgcaagta ttctacttta 11460

tgcgactaaa acacgcgaca agaaaacgcc aggaaaaggg cagggcggca gcctgtcgcg 11520

taacttagga cttgtgcgac atgtcgtttt cagaagacgg ctgcactgaa cgtcagaagc 11580

cgactgcact atagcagcgg aggggttgga tcaaagtact ttgatcccga ggggaaccct 11640

gtggttggca tgcacataca aatggacgaa cggataaac 11679

<210> 4
<211> 11535
<212> DNA
<213> Artificial Sequence

<220>
<223> crtB_stop_pGWB605


<220>
<221> misc_feature
<222> (1)..(11535)

<400> 4
cttttcacgc ccttttaaat atccgattat tctaataaac gctcttttct cttaggttta 60

cccgccaata tatcctgtca aacactgata gtttaaactg aaggcgggaa acgacaatct 120

gatccaagct caagctgctc tagcattcgc cattcaggct gcgcaactgt tgggaagggc 180

gatcggtgcg ggcctcttcg ctattacgcc agctggcgaa agggggatgt gctgcaaggc 240

gattaagttg ggtaacgcca gggttttccc agtcacgacg ttgtaaaacg acggccagtg 300

ccaagcttgc atgcctgcag gtccccagat tagccttttc aatttcagaa agaatgctaa 360

cccacagatg gttagagagg cttacgcagc aggtctcatc aagacgatct acccgagcaa 420

taatctccag gaaatcaaat accttcccaa gaaggttaaa gatgcagtca aaagattcag 480

gactaactgc atcaagaaca cagagaaaga tatatttctc aagatcagaa gtactattcc 540

agtatggacg attcaaggct tgcttcacaa accaaggcaa gtaatagaga ttggagtctc 600

taaaaaggta gttcccactg aatcaaaggc catggagtca aagattcaaa tagaggacct 660

aacagaactc gccgtaaaga ctggcgaaca gttcatacag agtctcttac gactcaatga 720

caagaagaaa atcttcgtca acatggtgga gcacgacaca cttgtctact ccaaaaatat 780

caaagataca gtctcagaag accaaagggc aattgagact tttcaacaaa gggtaatatc 840

cggaaacctc ctcggattcc attgcccagc tatctgtcac tttattgtga agatagtgga 900

aaaggaaggt ggctcctaca aatgccatca ttgcgataaa ggaaaggcca tcgttgaaga 960

tgcctctgcc gacagtggtc ccaaagatgg acccccaccc acgaggagca tcgtggaaaa 1020

agaagacgtt ccaaccacgt cttcaaagca agtggattga tgtgatatct ccactgacgt 1080

aagggatgac gcacaatccc actatccttc gcaagaccct tcctctatat aaggaagttc 1140

atttcatttg gagagaacac gggggactct agagttatca acaagtttgt acaaaaaagc 1200

aggcttcatg aataatccgt cgttactcaa tcatgcggtc gaaacgatgg cagttggctc 1260

gaaaagtttt gcgacagcct caaagttatt tgatgcaaaa acccggcgca gcgtactgat 1320

gctctacgcc tggtgccgcc attgtgacga tgttattgac gatcagacgc tgggctttca 1380

ggcccggcag cctgccttac aaacgcccga acaacgtctg atgcaacttg agatgaaaac 1440

gcgccaggcc tatgcaggat cgcagatgca cgaaccggcg tttgcggctt ttcaggaagt 1500

ggctatggct catgatatcg ccccggctta cgcgtttgat catctggaag gcttcgccat 1560

ggatgtacgc gaagcgcaat acagccaact ggatgatacg ctgcgctatt gctatcacgt 1620

tgcaggcgtt gtcggcttga tgatggcgca aatcatgggc gtgcgggata acgccacgct 1680

ggaccgcgcc tgtgaccttg ggctggcatt tcagttgacc aatattgctc gcgatattgt 1740

ggacgatgcg catgcgggcc gctgttatct gccggcaagc tggctggagc atgaaggtct 1800

gaacaaagag aattatgcgg cacctgaaaa ccgtcaggcg ctgagccgta tcgcccgtcg 1860

tttggtgcag gaagcagaac cttactattt gtctgccaca gccggcctgg cagggttgcc 1920

cctgcgttcc gcctgggcaa tcgctacggc gaagcaggtt taccggaaaa taggtgtcaa 1980

agttgaacag gccggtcagc aagcctggga tcagcggcag tcaacgacca cgcccgaaaa 2040

attaacgctg ctgctggccg cctctggtca ggcccttact tcccggatgc gggctcatcc 2100

tccccgccct gcgcatctct ggcagcgccc gctctagacc cagctttctt gtacaaagtg 2160

gttcgatcta gaggatccat ggtgagcaag ggcgaggagc tgttcaccgg ggtggtgccc 2220

atcctggtcg agctggacgg cgacgtgaac ggccacaagt tcagcgtgtc cggcgagggc 2280

gagggcgatg ccacctacgg caagctgacc ctgaagttca tctgcaccac cggcaagctg 2340

cccgtgccct ggcccaccct cgtgaccacc ttcacctacg gcgtgcagtg cttcagccgc 2400

taccccgacc acatgaagca gcacgacttc ttcaagtccg ccatgcccga aggctacgtc 2460

caggagcgca ccatcttctt caaggacgac ggcaactaca agacccgcgc cgaggtgaag 2520

ttcgagggcg acaccctggt gaaccgcatc gagctgaagg gcatcgactt caaggaggac 2580

ggcaacatcc tggggcacaa gctggagtac aactacaaca gccacaacgt ctatatcatg 2640

gccgacaagc agaagaacgg catcaaggtg aacttcaaga tccgccacaa catcgaggac 2700

ggcagcgtgc agctcgccga ccactaccag cagaacaccc ccatcggcga cggccccgtg 2760

ctgctgcccg acaaccacta cctgagcacc cagtccgccc tgagcaaaga ccccaacgag 2820

aagcgcgatc acatggtcct gctggagttc gtgaccgccg ccgggatcac tcacggcatg 2880

gacgagctgt acaagtaaag cggcccgagc tcgaatttcc ccgatcgttc aaacatttgg 2940

caataaagtt tcttaagatt gaatcctgtt gccggtcttg cgatgattat catataattt 3000

ctgttgaatt acgttaagca tgtaataatt aacatgtaat gcatgacgtt atttatgaga 3060

tgggttttta tgattagagt cccgcaatta tacatttaat acgcgataga aaacaaaata 3120

tagcgcgcaa actaggataa attatcgcgc gcggtgtcat ctatgttact agatcgggaa 3180

ttggttccgg aaccaattcg taatcatggt catagctgtt tcctgtgtga aattgttatc 3240

cgctcacaat tccacacaac atacgagccg gaagcataaa gtgtaaagcc tggggtgcct 3300

aatgagtgag ctaactcaca ttaggctgaa ttaggcgcgc ctatttctat taattcccga 3360

tctagtaaca tagatgacac cgcgcgcgat aatttatcct agtttgcgcg ctatattttg 3420

ttttctatcg cgtattaaat gtataattgc gggactctaa tcataaaaac ccatctcata 3480

aataacgtca tgcattacat gttaattatt acatgcttaa cgtaattcaa cagaaattat 3540

atgataatca tcgcaagacc ggcaacagga ttcaatctta agaaacttta ttgccaaatg 3600

tttgaacgat cggggaaatt cgagctgggg acgtccccag ctcggtaccc cctcctgggc 3660

acggtcgtac gcgatccgcc gcggttccac ctcaccattg cgggtatgcc agttgagttc 3720

gatgagttcc cgtaccagct ccaactcggg cgatgccacg cgcactccgt tcagatctcg 3780

gtgacgggca ggaccggacg gggcggtacc ggcaggctga agtccagctg ccagaaaccc 3840

acgtcatgcc agttcccgtg cttgaagccg gccgcccgca gcatgccgcg gggggcatat 3900

ccgagcgcct cgtgcatgcg cacgctcggg tcgttgggca gcccgatgac agcgaccacg 3960

ctcttgaagc cctgtgcctc cagggacttc agcaggtggg tgtagagcgt ggagcccagt 4020

cccgtccgct ggtggcgggg ggagacgtac acggtcgact cggccgtcca gtcgtaggcg 4080

ttgcgtgcct tccaggggcc cgcgtaggcg atgccggcga cctcgccgtc cacctcggcg 4140

acgagccagg gatagcgctc ccgcagacgg acgaggtcgt ccgtccactc ctgcggttcc 4200

tgcggctcgg tacggaagtt gaccgtgctt gtctcgatgt agtggttgac gatggtgcag 4260

accgccggca tgtccgcctc ggtggcacgg cggatgtcgg ccgggcgtcg ttctgggctc 4320

atatcttatt gccccccggg atcagatctg gattgagagt gaatatgaga ctctaattgg 4380

ataccgaggg gaatttatgg aacgtcagtg gagcattttt gacaagaaat atttgctagc 4440

tgatagtgac cttaggcgac ttttgaacgc gcaataatgg tttctgacgt atgtgcttag 4500

ctcattaaac tccagaaacc cgcggctgag tggctccttc aacgttgcgg ttctgtcagt 4560

tccaaacgta aaacggcttg tcccgcgtca tcggcggggg tcataacgtg actcccttaa 4620

ttctccgctc atgatcttga tcccctgcgc catcagatcc ttggcggcaa gaaagccatc 4680

cagtttactt tgcagggctt cccaacctta ccagagggcg ccccagctgg caattccggt 4740

tcgcttgctg tccataaaac cgcccagtct agctatcgcc atgtaagccc actgcaagct 4800

acctgctttc tctttgcgct tgcgttttcc cttgtccaga tagcccagta gctgacattc 4860

atccggggtc agcaccgttt ctgcggactg gctttctacg tgttccgctt cctttagcag 4920

cccttgcgcc ctgagtgctt gcggcagcgt gaagctgatc cgtcgagatt aatagaaata 4980

aattcagcct aattcggcgt taattcagta cattaaaaac gtccgcaatg tgttattaag 5040

ttgtctaagc gtcaatttgt ttacaccaca atatatcctg ccaccagcca gccaacagct 5100

ccccgaccgg cagctcggca caaaatcacc actcgataca ggcagcccat cagtccggga 5160

cggcgtcagc gggagagccg ttgtaaggcg gcagactttg ctcatgttac cgatgctatt 5220

cggaagaacg gcaactaagc tgccgggttt gaaacacgga tgatctcgcg gagggtagca 5280

tgttgattgt aacgatgaca gagcgttgct gcctgtgatc aattcgggca cgaacccagt 5340

ggacataagc ctgttcggtt cgtaagctgt aatgcaagta gcgtatgcgc tcacgcaact 5400

ggtccagaac cttgaccgaa cgcagcggtg gtaacggcgc agtggcggtt ttcatggctt 5460

gttatgactg tttttttggg gtacagtcta tgcctcgggc atccaagcag caagcgcgtt 5520

acgccgtggg tcgatgtttg atgttatgga gcagcaacga tgttacgcag cagggcagtc 5580

gccctaaaac aaagttaaac atcatggggg aagcggtgat cgccgaagta tcgactcaac 5640

tatcagaggt agttggcgtc atcgagcgcc atctcgaacc gacgttgctg gccgtacatt 5700

tgtacggctc cgcagtggat ggcggcctga agccacacag tgatattgat ttgctggtta 5760

cggtgaccgt aaggcttgat gaaacaacgc ggcgagcttt gatcaacgac cttttggaaa 5820

cttcggcttc ccctggagag agcgagattc tccgcgctgt agaagtcacc attgttgtgc 5880

acgacgacat cattccgtgg cgttatccag ctaagcgcga actgcaattt ggagaatggc 5940

agcgcaatga cattcttgca ggtatcttcg agccagccac gatcgacatt gatctggcta 6000

tcttgctgac aaaagcaaga gaacatagcg ttgccttggt aggtccagcg gcggaggaac 6060

tctttgatcc ggttcctgaa caggatctat ttgaggcgct aaatgaaacc ttaacgctat 6120
ggaactcgcc gcccgactgg gctggcgatg agcgaaatgt agtgcttacg ttgtcccgca 6180
tttggtacag cgcagtaacc ggcaaaatcg cgccgaagga tgtcgctgcc gactgggcaa 6240
tggagcgcct gccggcccag tatcagcccg tcatacttga agctagacag gcttatcttg 6300
gacaagaaga agatcgcttg gcctcgcgcg cagatcagtt ggaagaattt gtccactacg 6360
tgaaaggcga gatcaccaag gtagtcggca aataatgtct agctagaaat tcgttcaagc 6420
cgacgccgct tcgcggcgcg gcttaactca agcgttagat gcactaagca cataattgct 6480
cacagccaaa ctatcaggtc aagtctgctt ttattatttt taagcgtgca taataagccc 6540
tacacaaatt gggagatata tcatgcatga ccaaaatccc ttaacgtgag ttttcgttcc 6600
actgagcgtc agaccccgta gaaaagatca aaggatcttc ttgagatcct ttttttctgc 6660
gcgtaatctg ctgcttgcaa acaaaaaaac caccgctacc agcggtggtt tgtttgccgg 6720
atcaagagct accaactctt tttccgaagg taactggctt cagcagagcg cagataccaa 6780
atactgtcct tctagtgtag ccgtagttag gccaccactt caagaactct gtagcaccgc 6840
ctacatacct cgctctgcta atcctgttac cagtggctgc tgccagtggc gataagtcgt 6900
gtcttaccgg gttggactca agacgatagt taccggataa ggcgcagcgg tcgggctgaa 6960
cggggggttc gtgcacacag cccagcttgg agcgaacgac ctacaccgaa ctgagatacc 7020
tacagcgtga gctatgagaa agcgccacgc ttcccgaagg gagaaaggcg gacaggtatc 7080
cggtaagcgg cagggtcgga acaggagagc gcacgaggga gcttccaggg ggaaacgcct 7140
ggtatcttta tagtcctgtc gggtttcgcc acctctgact tgagcgtcga tttttgtgat 7200
gctcgtcagg ggggcggagc ctatggaaaa acgccagcaa cgcggccttt ttacggttcc 7260
tggccttttg ctggcctttt gctcacatgt tctttcctgc gttatcccct gattctgtgg 7320
ataaccgtat taccgccttt gagtgagctg ataccgctcg ccgcagccga acgaccgagc 7380
gcagcgagtc agtgagcgag gaagcggaag agcgcctgat gcggtatttt ctccttacgc 7440
atctgtgcgg tatttcacac cgcatatggt gcactctcag tacaatctgc tctgatgccg 7500
catagttaag ccagtataca ctccgctatc gctacgtgac tgggtcatgg ctgcgccccg 7560
acacccgcca acacccgctg acgcgccctg acgggcttgt ctgctcccgg catccgctta 7620
cagacaagct gtgaccgtct ccgggagctg catgtgtcag aggttttcac cgtcatcacc 7680
gaaacgcgcg aggcagggtg ccttgatgtg ggcgccggcg gtcgagtggc gacggcgcgg 7740
cttgtccgcg ccctggtaga ttgcctggcc gtaggccagc catttttgag cggccagcgg 7800
ccgcgatagg ccgacgcgaa gcggcggggc gtagggagcg cagcgaccga agggtaggcg 7860
ctttttgcag ctcttcggct gtgcgctggc cagacagtta tgcacaggcc aggcgggttt 7920
taagagtttt aataagtttt aaagagtttt aggcggaaaa atcgcctttt ttctctttta 7980

tatcagtcac ttacatgtgt gaccggttcc caatgtacgg ctttgggttc ccaatgtacg 8040

ggttccggtt cccaatgtac ggctttgggt tcccaatgta cgtgctatcc acaggaaaga 8100

gaccttttcg acctttttcc cctgctaggg caatttgccc tagcatctgc tccgtacatt 8160

aggaaccggc ggatgcttcg ccctcgatca ggttgcggta gcgcatgact aggatcgggc 8220

cagcctgccc cgcctcctcc ttcaaatcgt actccggcag gtcatttgac ccgatcagct 8280

tgcgcacggt gaaacagaac ttcttgaact ctccggcgct gccactgcgt tcgtagatcg 8340

tcttgaacaa ccatctggct tctgccttgc ctgcggcgcg gcgtgccagg cggtagagaa 8400

aacggccgat gccgggatcg atcaaaaagt aatcggggtg aaccgtcagc acgtccgggt 8460

tcttgccttc tgtgatctcg cggtacatcc aatcagctag ctcgatctcg atgtactccg 8520

gccgcccggt ttcgctcttt acgatcttgt agcggctaat caaggcttca ccctcggata 8580

ccgtcaccag gcggccgttc ttggccttct tcgtacgctg catggcaacg tgcgtggtgt 8640

ttaaccgaat gcaggtttct accaggtcgt ctttctgctt tccgccatcg gctcgccggc 8700

agaacttgag tacgtccgca acgtgtggac ggaacacgcg gccgggcttg tctcccttcc 8760

cttcccggta tcggttcatg gattcggtta gatgggaaac cgccatcagt accaggtcgt 8820

aatcccacac actggccatg ccggccggcc ctgcggaaac ctctacgtgc ccgtctggaa 8880

gctcgtagcg gatcacctcg ccagctcgtc ggtcacgctt cgacagacgg aaaacggcca 8940

cgtccatgat gctgcgacta tcgcgggtgc ccacgtcata gagcatcgga acgaaaaaat 9000

ctggttgctc gtcgcccttg ggcggcttcc taatcgacgg cgcaccggct gccggcggtt 9060

gccgggattc tttgcggatt cgatcagcgg ccgcttgcca cgattcaccg gggcgtgctt 9120

ctgcctcgat gcgttgccgc tgggcggcct gcgcggcctt caacttctcc accaggtcat 9180

cacccagcgc cgcgccgatt tgtaccgggc cggatggttt gcgaccgctc acgccgattc 9240

ctcgggcttg ggggttccag tgccattgca gggccggcag acaacccagc cgcttacgcc 9300

tggccaaccg cccgttcctc cacacatggg gcattccacg gcgtcggtgc ctggttgttc 9360

ttgattttcc atgccgcctc ctttagccgc taaaattcat ctactcattt attcatttgc 9420

tcatttactc tggtagctgc gcgatgtatt cagatagcag ctcggtaatg gtcttgcctt 9480

ggcgtaccgc gtacatcttc agcttggtgt gatcctccgc cggcaactga aagttgaccc 9540

gcttcatggc tggcgtgtct gccaggctgg ccaacgttgc agccttgctg ctgcgtgcgc 9600

tcggacggcc ggcacttagc gtgtttgtgc ttttgctcat tttctcttta cctcattaac 9660

tcaaatgagt tttgatttaa tttcagcggc cagcgcctgg acctcgcggg cagcgtcgcc 9720

ctcgggttct gattcaagaa cggttgtgcc ggcggcggca gtgcctgggt agctcacgcg 9780

ctgcgtgata cgggactcaa gaatgggcag ctcgtacccg gccagcgcct cggcaacctc 9840

accgccgatg cgcgtgcctt tgatcgcccg cgacacgaca aaggccgctt gtagccttcc 9900

atccgtgacc tcaatgcgct gcttaaccag ctccaccagg tcggcggtgg cccatatgtc 9960

gtaagggctt ggctgcaccg gaatcagcac gaagtcggct gccttgatcg cggacacagc 10020

caagtccgcc gcctggggcg ctccgtcgat cactacgaag tcgcgccggc cgatggcctt 10080

cacgtcgcgg tcaatcgtcg ggcggtcgat gccgacaacg gttagcggtt gatcttcccg 10140

cacggccgcc caatcgcggg cactgccctg gggatcggaa tcgactaaca gaacatcggc 10200

cccggcgagt tgcagggcgc gggctagatg ggttgcgatg gtcgtcttgc ctgacccgcc 10260

tttctggtta agtacagcga taaccttcat gcgttcccct tgcgtatttg tttatttact 10320

catcgcatca tatacgcagc gaccgcatga cgcaagctgt tttactcaaa tacacatcac 10380

ctttttagac ggcggcgctc ggtttcttca gcggccaagc tggccggcca ggccgccagc 10440

ttggcatcag acaaaccggc caggatttca tgcagccgca cggttgagac gtgcgcgggc 10500

ggctcgaaca cgtacccggc cgcgatcatc tccgcctcga tctcttcggt aatgaaaaac 10560

ggttcgtcct ggccgtcctg gtgcggtttc atgcttgttc ctcttggcgt tcattctcgg 10620

cggccgccag ggcgtcggcc tcggtcaatg cgtcctcacg gaaggcaccg cgccgcctgg 10680

cctcggtggg cgtcacttcc tcgctgcgct caagtgcgcg gtacagggtc gagcgatgca 10740

cgccaagcag tgcagccgcc tctttcacgg tgcggccttc ctggtcgatc agctcgcggg 10800

cgtgcgcgat ctgtgccggg gtgagggtag ggcgggggcc aaacttcacg cctcgggcct 10860

tggcggcctc gcgcccgctc cgggtgcggt cgatgattag ggaacgctcg aactcggcaa 10920

tgccggcgaa cacggtcaac accatgcggc cggccggcgt ggtggtgtcg gcccacggct 10980

ctgccaggct acgcaggccc gcgccggcct cctggatgcg ctcggcaatg tccagtaggt 11040

cgcgggtgct gcgggccagg cggtctagcc tggtcactgt cacaacgtcg ccagggcgta 11100

ggtggtcaag catcctggcc agctccgggc ggtcgcgcct ggtgccggtg atcttctcgg 11160

aaaacagctt ggtgcagccg gccgcgtgca gttcggcccg ttggttggtc aagtcctggt 11220

cgtcggtgct gacgcgggca tagcccagca ggccagcggc ggcgctcttg ttcatggcgt 11280

aatgtctccg gttctagtcg caagtattct actttatgcg actaaaacac gcgacaagaa 11340

aacgccagga aaagggcagg gcggcagcct gtcgcgtaac ttaggacttg tgcgacatgt 11400

cgttttcaga agacggctgc actgaacgtc agaagccgac tgcactatag cagcggaggg 11460

gttggatcaa agtactttga tcccgagggg aaccctgtgg ttggcatgca catacaaatg 11520

gacgaacgga taaac 11535

<210> 5
<211> 20
<212> DNA
<213> Artificial Sequence

<220>
<223>  primers NtSAG12_qPCR-F


<220>
<221>  misc_feature
<222>  (1)..(20)

<400>  5
attcatgggg cagtaaatgg                                                   20


<210>  6
<211>  20
<212>  DNA
<213>  Artificial Sequence

<220>
<223>  NtSAG12_qPCR-R


<220>
<221>  misc_feature
<222>  (1)..(20)

<400>  6
gaagcgtcca tagcaagtcc                                                   20


<210>  7
<211>  19
<212>  DNA
<213>  Artificial Sequence

<220>
<223>  NtRPL25_qPCR-F


<220>
<221>  misc_feature
<222>  (1)..(19)

<400>  7
aaggcacagg cagctaagg                                                    19


<210>  8
<211>  22
<212>  DNA
<213>  Artificial Sequence

<220>
<223>  NtRPL25_qPCR-R


<220>
<221>  misc_feature
<222>  (1)..(22)

<400>  8
aggtcggtgg aatgtaactt tt                                                22

<210>  9
<211>  56
<212>  DNA
<213>  Artificial Sequence

<220>
<223>  PacrtB_N-tag_attB1-F


<220>
<221>  misc_feature
<222>  (1)..(56)

<400>  9
ggggacaagt ttgtacaaaa aagcaggctg gatgaataat ccgtcgttac tcaatc          56


<210>  10
<211>  53
<212>  DNA
<213>  Artificial Sequence

<220>
<223>  PacrtB_C-tag_attB2-R


<220>
<221>  misc_feature
<222>  (1)..(53)

<400>  10
ggggaccact ttgtacaaga aagctgggtc tagagcgggc gctgccagag atg          53


<210>  11
<211>  53
<212>  DNA
<213>  Artificial Sequence

<220>
<223>  PacrtB_STOP_attB2-R


<220>
<221>  misc_feature
<222>  (1)..(53)

<400>  11
ggggaccact ttgtacaaga aagctgggtc tagagcgggc gctgccagag atg          53


<210>  12
<211>  56
<212>  DNA
<213>  Artificial Sequence

<220>
<223>  AtHDS_N-tag_attB1-F


<220>
<221>  misc_feature

<222>  (1)..(56)

<400>  12
ggggacaagt ttgtacaaaa aagcaggctt catggcgact ggagtattgc cagctc          56


<210>  13
<211>  50
<212>  DNA
<213>  Artificial Sequence

<220>
<223>  crtB-5\222-AtHDS-3\222-R


<220>
<221>  misc_feature
<222>  (1)..(50)

<400>  13
gattgagtaa cgacggatta ttcatattcc ggataaccga aactcttctc                50


<210>  14
<211>  57
<212>  DNA
<213>  Artificial Sequence

<220>
<223>  AtPSY_N-tag_attB1-F


<220>
<221>  misc_feature
<222>  (1)..(57)

<400>  14
ggggacaagt ttgtacaaaa aagcaggctt catgtcaagc ttagtagcaa gtccttc         57


<210>  15
<211>  55
<212>  DNA
<213>  Artificial Sequence

<220>
<223>  AtPSY_STOP_attB2-R


<220>
<221>  misc_feature
<222>  (1)..(55)

<400>  15
ggggaccact ttgtacaaga aagctgggtt catatcgata gtcttgaact tgaag           55


<210>  16
<211>  54
<212>  DNA
<213>  Artificial Sequence

<220>

<223>  SlPSY2_N-tag_attB1-F

<220>
<221>  misc_feature
<222>  (1)..(54)

<400>  16
ggggacaagt ttgtacaaaa aagcaggcta tgtctgttgc tttgttgtgg gttg          54

<210>  17
<211>  55
<212>  DNA
<213>  Artificial Sequence

<220>
<223>  SlPSY2_STOP_attB2-R

<220>
<221>  misc_feature
<222>  (1)..(55)

<400>  17
ggggaccact ttgtacaaga aagctgggtt catgtctttg ctagtgggga agaag          55

<210>  18
<211>  55
<212>  DNA
<213>  Artificial Sequence

<220>
<223>  PacrtE_N-tag_attB1-F

<220>
<221>  misc_feature
<222>  (1)..(55)

<400>  18
ggggacaagt ttgtacaaaa aagcaggctg gatgacggtc tgcgcaaaaa aacac          55

<210>  19
<211>  57
<212>  DNA
<213>  Artificial Sequence

<220>
<223>  PacrtE_STOP_attB2-R

<220>
<221>  misc_feature
<222>  (1)..(57)

<400>  19
ggggaccact ttgtacaaga aagctgggtt tatactgacg gcagcgagtt ttttgtc          57